(19) Europäisches Patentamt
European Patent Office
Office européen des brevets

(11) Numéro de publication: 0 004 863
B1

(12) # FASCICULE DE BREVET EUROPEEN

(45) Date de publication du fascicule du brevet: 16.06.82

(21) Numéro de dépôt: 79100759.4

(22) Date de dépôt: 14.03.79

(51) Int. Cl.³: C 07 D 323/00, A 61 K 7/06, A 61 K 7/13, A 61 K 31/335 //C07D407/12

(54) Compositions cosmétiques et pharmaceutiques, polyéthers cycliques tensio-actifs et procédés pour les préparer.

(30) Priorité: 17.03.78 FR 7807914

(43) Date de publication de la demande: 31.10.79 Bulletin 79/22

(45) Mention de la délivrance du brevet: 16.06.82 Bulletin 82/24

(84) Etats contractants désignés: BE CH DE FR GB IT NL SE

(56) Documents cités:
FR - A - 2 359 165

A. M. Schwartz, J. W. Perry, Chimie et Technologie des Agents Tensio-Actifs (édition Dunod, Paris 1955) p. 209 et suivantes.

(73) Titulaire: L'OREAL Société anonyme dite:
14, Rue Royale
F-75008 Paris (FR)

(72) Inventeur: Vanlerberghe, Guy
Rue du Général de Gaulle Villevaudé
F-77410 Claye-Souilly (FR)
Inventeur: Sebag, Henri
26, rue Erlanger
F-75016 Paris (FR)

(74) Mandataire: Casalonga, Axel et al,
BUREAU D.A. CASALONGA OFFICE JOSSE & PETIT Baaderstrasse 12-14
D-8000 München 5 (DE)

EP 0 004 863 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Compositions cosmétiques et pharmaceutiques, polyéthers cycliques tensio-actifs et procédés pour les préparer

L'invention a pour objet de nouvelles compositions cosmétiques et pharmaceutiques contenant de nouveaux polyéthers cycliques tensio-actifs, ces nouveaux composés et les procédés pour les préparer.

Le brevet français n° 2.359.165 décrit des oligomères tensio-actifs séquencés linéaires, constitués d'une séquence amphiphile et d'une ou de deux séquences hydrophiles.

La préparation de ces composés est assez complexe, nécessite trois matières de départ et des opérations multiples.

Les composés tensio-actifs selon l'invention ont une structure plus simple et très différente de ces oligomères séquencés. Ils comportent uniquement des groupements amphiphiles, ils sont cycliques et sont obtenus à partir d'une matière de départ unique.

Les nouveaux polyéthers cycliques tensio-actifs présentent des propriétés remarquables qui les différencient des composés analogues décrits jusqu'à présent. Par leur structure chimique ils sont apparentés aux éthers-couronnes (Crown ethers) qui ont été très étudiés au cours des dernières années comme complexants des cations de métaux alcalins ou alcalino-terreux.

Ils s'en différencient par le caractère amphiphile c'est-à-dire une affinité à la fois vis-à-vis de l'eau et des milieux organiques, ce qui leur confère une forte activité interfaciale.

Ils se distinguent également des agents de surface conventionnels qui comportent une seule chaîne lipophile par molécule.

Comme cela est bien connu, ces derniers, lorsqu'ils sont dissous dans l'eau, manifestent au-delà d'un seuil de concentration appelé concentration micellaire critique (CMC), un ensemble de propriétés très avantageuses en vue d'un grand nombre d'applications. En particulier, à des concentrations au moins égales à CMC, ils solubilisent dans l'eau des substances organiques telles que des colorants liposolubles et des hydrocarbures.

Les composés selon l'invention possèdent des propriétés solubilisantes à des concentrations infiniment petites, bien inférieures à CMC des agents de surface qui comportent une chaîne lipophile de longueur comparable.

Ceci constitue un avantage important pour certaines utilisations de tensio-actifs telles que par exemple dans des compositions pharmaceutiques ou cosmétiques où il y a intérêt à réduire le plus possible la quantité de composé tensio-actif utilisée afin de ne pas interférer avec le principe actif de ces compositions.

En outre, les composés de l'invention sont moins agressifs vis-à-vis de la peau ou des muqueuses, en particulier les muqueuses oculaires, que les agents de surface comportant une seule chaîne lipophile par molécule et des groupements fonctionnels comparables.

Les compositions cosmétiques et pharmaceutiques selon l'invention sont caractérisées par le fait qu'elles renferment au moins un nouveau polyéther cyclique tensio-actif de formule générale.

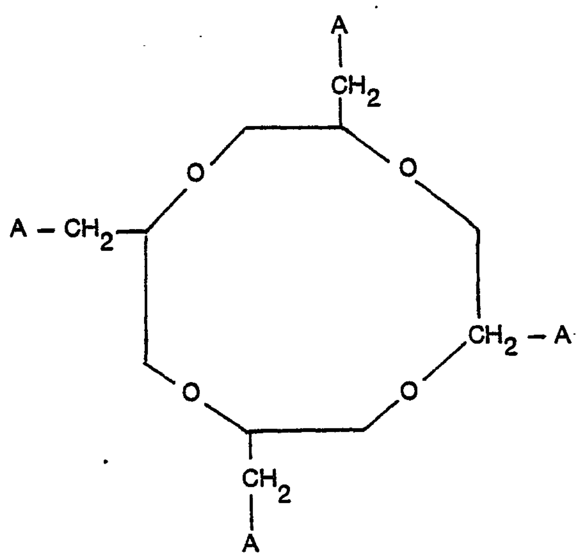

(I)

dans laquelle A désigne un ensemble amphiphile constitué d'une partie hydrophile et d'une partie lipophile, cet ensemble amphiphile étant relié par la partie hydrophile, au cycle, par liaison covalente; la partie hydrophile comporte un ou plusieurs groupements amine, oxyde d'amine, ammonium, ammonio alcanoate, ammonio alcoyl sulfonate, ammonio alcoyl sulfinate, thioéther, sulfoxyde, sulfonium, sulfoxonium, éther, hydroxyle, ester, amide, acide; la partie lipophile est constituée d'un ou plusieurs groupements choisis parmi (i) les groupements aliphatiques ayant de 1 à 20 atomes de carbone, (ii) les

groupements aliphatiques substitués ayant de 7 à 20 atomes de carbone, (iii) les groupements alcoylaryle ayant de 7 à 20 atomes de carbone, la partie alcoyle du groupement alcoylaryl ayant jusqu'à 14 atomes de carbone.

Les fonctions amine et leurs dérivés, les fonctions éther, thioéther, alcool, ester carboxylique, amide ont des propriétés nettement hydrophiles. Voir à ce sujet le livre de A.M. SCHWARTZ et J.W. PERRRY "Chimie et Technologie des Agents Tensio-Actifs" traduit de l'américain par J. COLONGE et al, édition Dunod, Paris (1955) et en particulier page 209 et suivantes.

Parmi les groupements aliphatiques non substitués, aliphatiques substitués et alcoylaryle présents dans l'ensemble amphiphile A, on peut citer comme exemple les groupements alcoyle, hydroxyalcoyle, alcényle, alcoylphényle, benzyle et alcoylbenzyle.

La formule générale (I) peut également s'écrire sous la forme simplifiée:

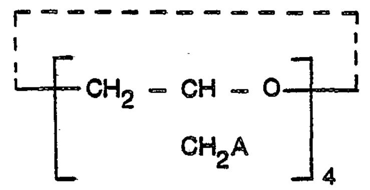

(Ia)

Comme groupement amphiphile A on peut citer à titre d'exemple les suivants:

a) le groupement

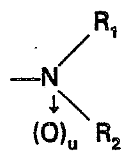

où $R_1$ et $R_2$, identiques ou différents, désignent des radicaux aliphatiques et de préférence alcoyle, hydroxyalcoyle ou alcényle ayant de 1 à 20 atomes de carbone, l'un des radicaux comportant au moins 8 atomes de carbone; ou des radicaux aliphatiques substitués ayant de 6 à 20 atomes de carbone ou des radicaux alcoylaryl ayant de 6 à 20 atomes de carbone; la somme des atomes de carbone de $R_1$ et $R_2$ étant, de préférence, inférieure ou égale à 28; l'un des radicaux peut, en outre, désigner un radical diméthylaminoéthyle ou propyle, diéthylaminoéthyle ou propyle, pipéridino éthyle ou propyle, morpholino éthyle ou propyle, alcoyl polyhydroxy propylène;

*u* désigne zéro ou 1;

$a_1$) le groupement

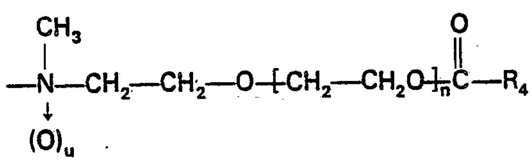

où $R_4$ désigne un radical aliphatique non substitué ou substitué ou un radical alcoylaryle ayant de 6 à 20, en particulier de 8 à 16 et plus particulièrement de 12 à 16 atomes de carbone et de préférence un radical alcoyle, hydroxyalcoyle, alcényle et alcoylphényle, la partie alcoyle du radical alcoylphényle ayant jusqu'à 14 atomes de carbone;

*n* désigne un nombre quelconque de 1 à 20, avantageusement de 1 à 10, en particulier de 2 à 8 et plus particulièrement de 4 à 6;

*u* désigne le nombre 0 ou 1;

$a_2$) le groupement

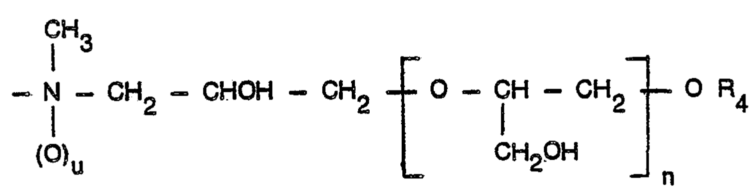

*u*, $R_4$ et *n* ayant la signification ci-dessus indiquée.

b) le groupement

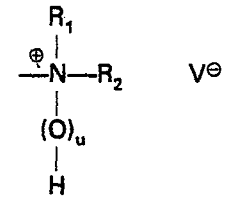

$b_1$) le groupement

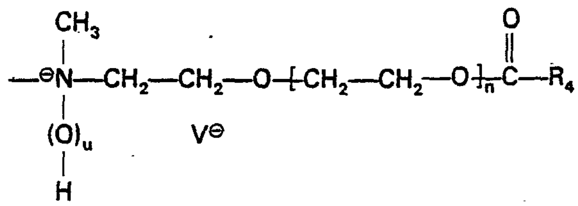

$b_2$) le groupement

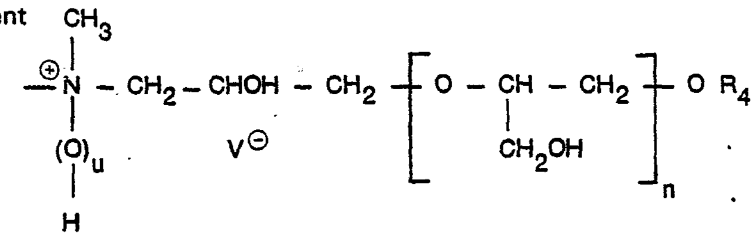

où *u* désigne le nombre 0 ou 1;
*n* désigne un nombre quelconque de 1 à 20, avantageusement de 1 à 10, en particulier de 2 à 8 et plus particulièrement de 4 à 6;
$V^{\ominus}$ désigne un anion et de préférence un anion formiate, acétate, citrate ou lactate, $R_1$, $R_2$, $R_4$ ont les significations indiquées précédemment.

c) le groupement

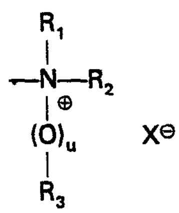

$R_1$, $R_2$ et *u* ayant la même signification que ci-dessus,
$R_3$ désigne un radical alcoyle ou hydroxyalcoyle ayant de 1 à 3 atomes de carbone et de préférence un radical méthyle, éthyle, hydroxyéthyle, dihydroxypropyle; ou un radical benzyle;
$X^{\ominus}$ désigne un anion et de préférence un chlorure, bromure, iodure, monométhyl sulfate, méthyl sulfonate, p.toluène sulfonate;

$c_1$) le groupement

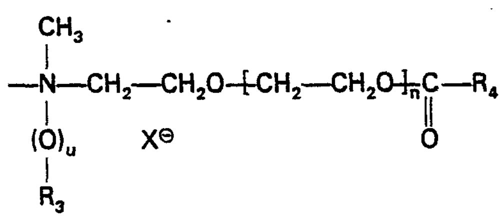

$c_2$) le groupement

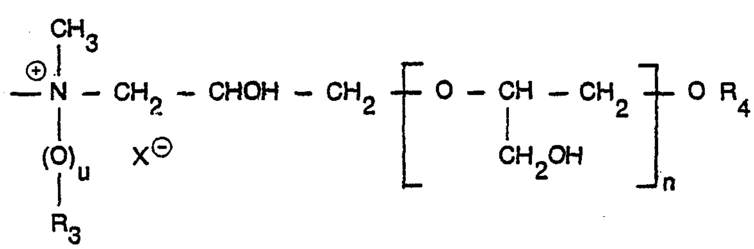

dans ces groupements ($c_1$) et ($c_2$)
*u* désigne le nombre 0 ou 1

*n* désigne un nombre quelconque de 1 à 20, avantageusement de 1 à 10, en particulier de 2 à 8 et plus particulièrement de 4 à 6;
$R_4$ a les significations ci-dessus indiquées

d) le groupement

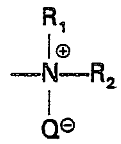

où $Q^{\ominus}$ désigne l'un des groupements suivants:

$$-(CH_2)_m-COO^{\ominus}$$ où *m* désigne 1, 2 ou 3

$$-CH_2-CH_2-CH_2-SO_3^{\ominus} \quad -CH_2-CH_2\ O\ SO_2^{\ominus}$$

$d_1$) le groupement

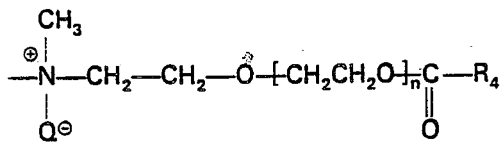

$d_2$) le groupement

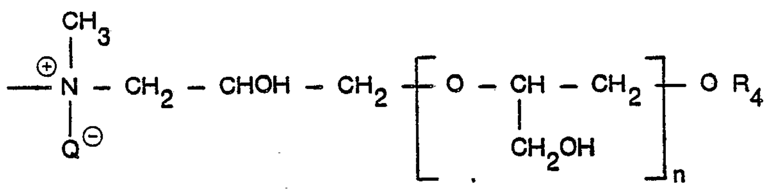

dans les groupements ($d_1$) et ($d_2$), $R_4$ a la signification indiquée pour le groupement ($a_1$), *n* désigne un nombre quelconque de 1 à 20, avantageusement de 1 à 10, en particulier de 2 à 8 et plus particulièrement de 4 à 6;
$Q^{\ominus}$ a la signification indiquée pour le groupement (d).

e) le groupement

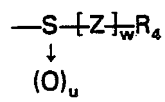

où $R_4$ a la signification indiquée pour le groupement ($a_1$);
*u* désigne zéro ou 1;
*w* désigne zéro ou 1;
Z désigne des groupements d'atomes à caractères hydrophile qui peuvent être éther, ester, amide, amine, ammonium et/ou hydroxyle.

Comme exemple de groupements Z on peut citer les suivants:

$-CH_2-CHOH-$ $\quad -(CH_2-CH_2O)_n-$ 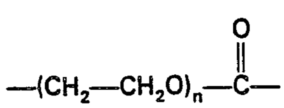 $-(CH_2)_x-COO-$

$-(CH_2)_x-COOCH_2-CHOH-$ $\quad -(CH_2)_x-CONH-$ 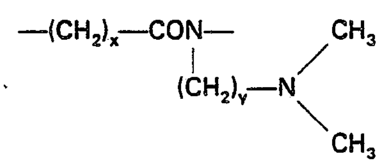

où x représente 1 ou 2; y représente 2 ou 3; *n* représente un nombre quelconque de 1 à 20.

$e_1$) le groupement

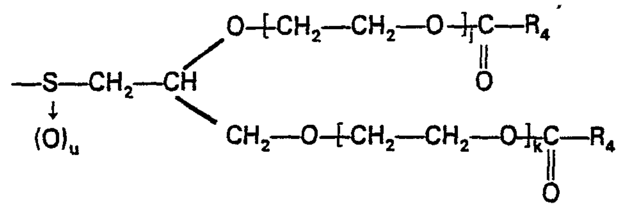

j et k, identiques ou différents, désignent chacun un nombre quelconque inférieur à 20, la somme j + k étant comprise entre 1 et 20, $R_4$ ayant la signification indiquée pour le groupement ($a_1$).

f) le groupement

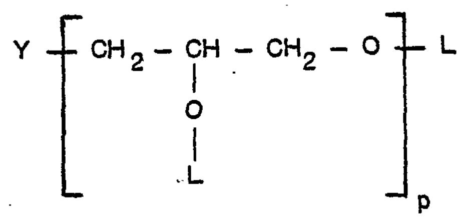

où L désigne en même temps un atome d'hydrogène et l'un des groupements:

$$-CH_2-CHOH-CH_2-(O)_q-R_5$$

où q désigne 0 ou 1

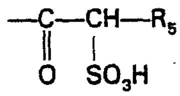
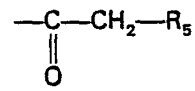

l'atome d'hydrogène et l'autre groupement étant répartis de façon statistique;
$R_5$ désigne un radical alcoyle linéaire ayant de 6 à 18 et en particulier de 8 à 16 atomes de carbone; p désigne un nombre quelconque de 1 à 10, en particulier de 2 à 8, plus particulièrement de 3 à 6, et représente une valeur statistique moyenne;
Y désigne:

$-O-$; $-S-$ ou 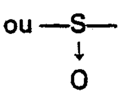

g) le groupement

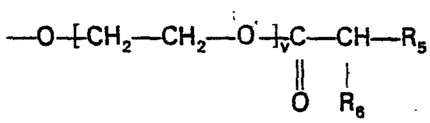

où v désigne un nombre quelconque de 0 à 20, en particulier de 0 à 10, plus particulièrement de 0 à 5; $R_6$ désigne un atome d'hydrogène ou le radical $-SO_3H$; $R_5$ ayant la signification ci-dessus indiquée;

h) le groupement (e) ou ($e_1$) alcoylé par un agent d'alcoylation choisi dans le groupe formé par le bromure de méthyle, l'iodure de méthyle et le sulfate de diméthyle et de préférence par le sulfate de diméthyle;

i) le groupement (f) alcoylé par un agent d'alcoylation choisi dans le groupe formé par le bromure de méthyle, l'iodure de méthyle et le sulfate de diméthyle et de préférence par le sulfate de diméthyle;

Les polyéthers cycliques tensio-actifs de type la répondant à la formule (I) dans laquelle A désigne le groupement (a)

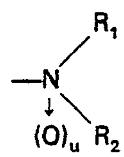

ne sont pas solubles dans l'eau sous cette forme, mais ils peuvent conduire à des produits solubles par salification ou alcoylation.

Les polyéthers cycliques tensio-actifs de formule (I) dans laquelle A désigne l'un des groupements ($a_1$), ($a_2$), (b), ($b_1$), ($b_2$), (c), ($c_1$), ($c_2$), (d), ($d_1$), ($d_2$), (e), ($e_1$), (f), (g), (h), (i) sont généralement solubles dans l'eau.

Les polyéthers cycliques tensio-actifs selon l'invention sont tous obtenus à partir du polyéther cyclique polychoré, tétramère de l'épichlorhydrine, de formule:

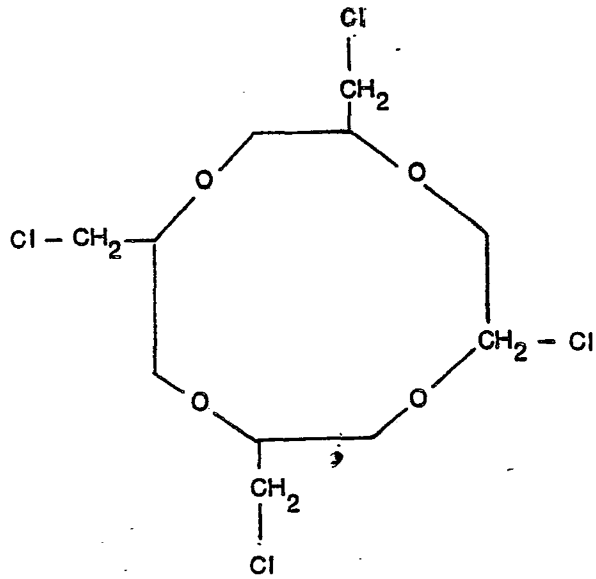

(III)

De façon simplifiée cette formule s'écrit:

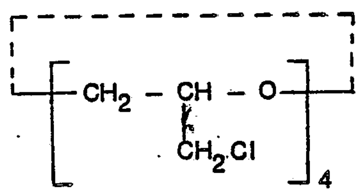

(IIIa)

Le tétramère de l'épichlorhydrine est préparé d'une manière connue par polymérisation de l'épichlorhydrine en présence d'un catalyseur acide de Lewis, tels que $BF_3$, $SnCl_4$ ou $SbCl_5$ et purifié par fractionnement sous pression réduite. La préparation de ce composé est décrite dans l'exemple I.

Le tétramère de l'épichlorhydrine de formule (III) réagit avec les amines secondaires entre 80 et 150°C et avantageusement entre 130 et 150°C, à la pression normale ou en autoclave, en présence éventuellement d'un solvant choisi parmi les alcools inférieurs en $C_1$—$C_4$, les alkoxyéthanols, les diéthers de glycol, le diméthylformamide ou le méthyl caprolactame, en donnant les polyéthers cycliques tensio-actifs du type I(a) (composés de formule (I) dans laquelle A désigne le groupement (a)

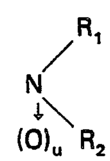

(a)

où $R_1$, $R_2$ et *u* ont les significations ci-dessus indiquées.

Les composés du type $I(a_1)$, répondant à la formule (I) dans laquelle A désigne le groupement $(a_1)$

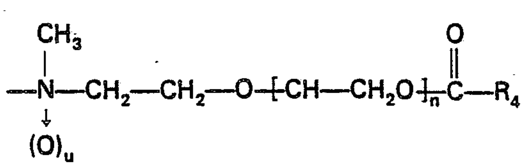

$(a_1)$

où *u, n,* $R_4$ ont les significations ci-dessus indiquées peuvent être préparés en faisant réagir la méthyléthanolamine avec le tétramère de l'épichlorhydrine de formule (III); on fixe ensuite *n* moles d'oxyde d'éthylène sur le produit obtenu précédemment et on estérifie enfin les fonctions OH avec un acide de formule $R_4$—COOH où $R_4$ a la signification ci-dessus indiquée.

Les composés du type $I(a_2)$ répondant à la formule (I) dans laquelle A désigne le groupement $(a_2)$

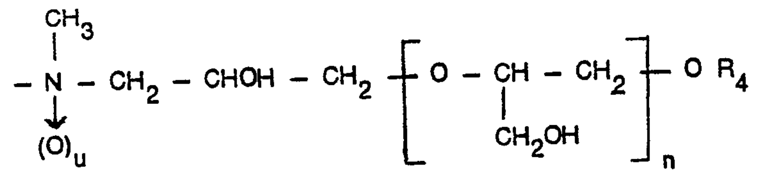

$(a_2)$

où *u*, $R_4$ et *n* ont la signification ci-dessus indiquée, peuvent être préparés en faisant réagir le tétramère de l'épichlorhydrine de formule (III) avec un important excès de méthylamine. Sur le produit ainsi obtenu on condense un composé de formule:

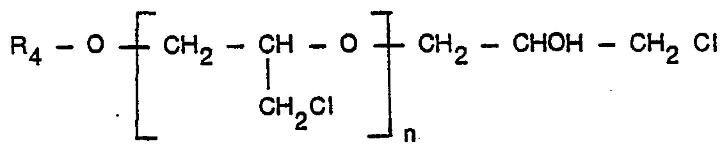

(que l'on peut préparer par polyaddition de n + 1 moles d'épichlorhydrine sur une mole de composé de formule $R_4OH$, $R_4$ ayant la signification ci-dessus indiquée).

On remplace enfin dans le composé intermédiaire ainsi obtenu les atomes de chlore par des groupes hydroxyle à l'aide d'acétate de sodium ou de potassium suivi d'hydrolyse ou l'alcoolyse de l'ester acétique formé.

Ce procédé de préparation est décrit plus en détail dans le brevet français n° 1.538.525.

Les composés du type I(b), $I(b_1)$ et $I(b_2)$ répondant à la formule (I) dans laquelle A désigne le groupement (b), $(b_1)$ ou $(b_2)$

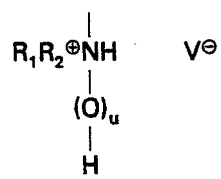

(b)

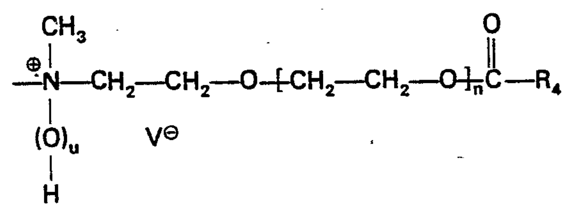

$(b_1)$

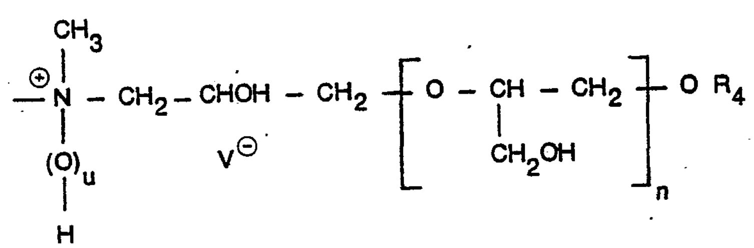

$(b_2)$

où $R_4$, $V^-$, *u, n,* ont les significations ci-dessus indiquées, peuvent être préparés respectivement par salification des groupements (a), $(a_1)$ ou $(a_2)$

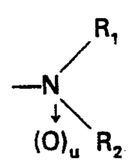

(a)

ou

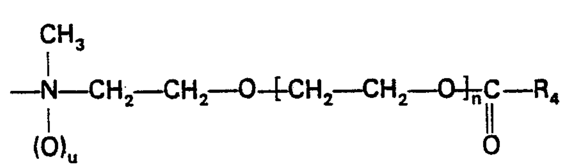

$(a_1)$

ou 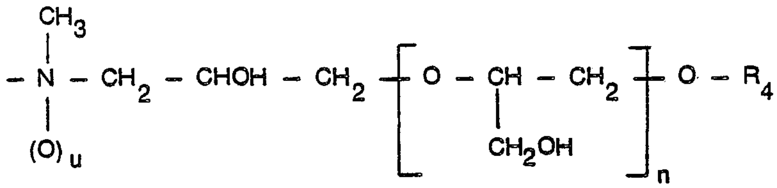 (a₂)

d'un composé du type I(a), I($a_1$), I($a_2$) avec un acide approprié, avantageusement avec un acide organique, choisi de préférence parmi les acides formique, acétique, citrique, lactique, tartrique.

Les composés du type I(c), I($c_1$) et I($c_2$) répondant à la formule (I) dans laquelle A désigne respectivement le groupement (c), ($c_1$) ou ($c_2$)

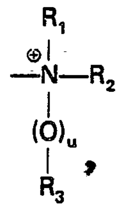 (c)

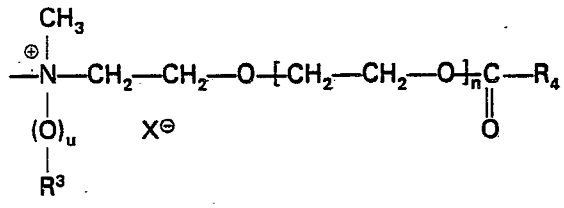 ($c_1$)

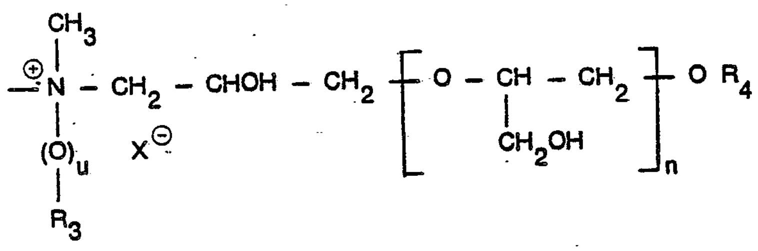 ($c_2$)

peuvent être préparés respectivement par alcoylation du groupement (a), ($a_1$) ou ($a_2$) d'un composé du type I(a), I($a_1$) ou I($a_2$).

On utilise avantageusement un agent alcoylant de formule X $R_3$, où X et $R_3$ ont les significations ci-dessus indiquées.

Parmi ces agents alcoylants on peut citer le chlorure, bromure, iodure de méthyle, le sulfate de diméthyle ou diéthyle, le méthane sulfonate de méthyle, le p.toluène sulfonate de méthyle, la monochlorhydrine de glycol et de glycérol. On préfère plus particulièrement le sulfate de diméthyle.

Quelques composés du type I(c) peuvent encore être obtenus par réaction du tétramère de l'épichlorhydrine de formule (III) avec des amines secondaires à courte chaîne de formule:

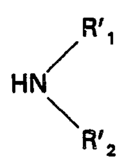

où $R'_1$ et $R'_2$ désignent un alkyle ou hydroxyalkyle ayant de 1 à 3 atomes de carbone, suivie d'une alcoylation avec un mésylate ou tosylate d'un alcool ou d'un mélange d'alcools ayant de 8 à 20 atomes de carbone.

Les composés du type I($c_2$) peuvent encore être préparés par réaction du tétramère de l'épichlorhydrine de formule (III) avec des composés de formule:

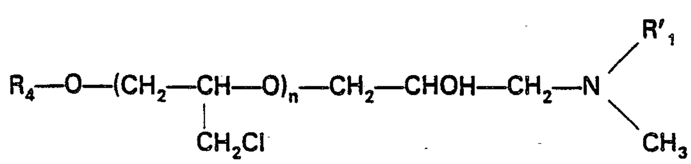

où $R_4$, $R'_1$ et *n* ont les significations ci-dessus indiquées, suivie d'une hydrolyse des groupements chloro-méthylés.

Parmi les amines secondaires à courte chaîne on peut citer, à titre d'exemple, la diméthylamine, la méthyléthanolamine.

Les composés du type I(d), I($d_1$), I($d_2$) répondant à la formule (I) dans laquelle A désigne respectivement le groupement (d), ($d_1$) ou ($d_2$)

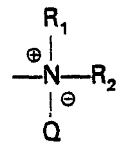
(d)

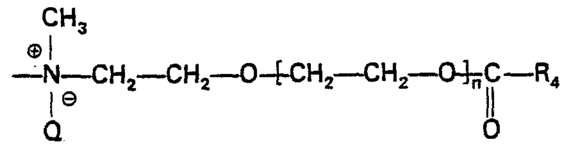
($d_1$)

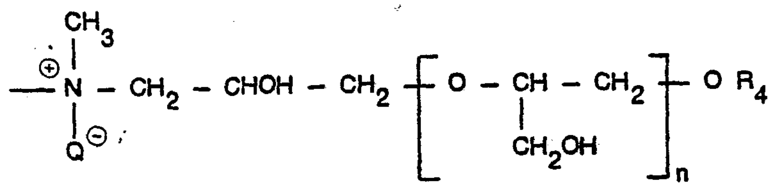
($d_2$)

où $R_1$, $R_2$, $Q^{\ominus}$, n, ont les significations ci-dessus indiquées, peuvent être obtenus par alcoylation respectivement d'un composé du type I(a), I($a_1$), I($a_2$), dans laquelle u signifie zero, avec l'un des reactifs ci-après:

les acides de formules:

$Cl\ (CH_2)_m—COOH$ $Br\ (CH_2)_m—COOH$

où m désigne 1, 2 ou 3

$Cl\ CH_2—CH_2—CH_2—SO_3H$ $Br\ CH_2—CH_2—CH_2—SO_3H$,

le sel de sodium ou de potassium d'un tel acide, la propane sultone de formule:

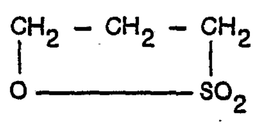

ou le sulfite de glycol de formule:

$CH_2OH—CH_2—OSO_2H$.

Les composés du type I(e) répondant à la formule (I) dans laquelle A désigne le groupement

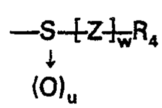
(e)

$R_4$, *u*, *w*, ayant la signification ci-dessus indiquée et Z désigne $—CH_2—CHOH—$ ou $—(CH_2—CH_2O)_n—$ où *n* désigne un nombre quelconque de 1 à 20 peuvent être obtenus par réaction du tétramère de l'épichlorhydrine de formule (III) avec des alcoyl mercaptans, des hydroxyalcoyl mercaptans, des alcoyl mono- ou poly-éthoxy mercaptans, les groupements alcoyle et hydroxyalcoyle ayant de 6 à 20 atomes de carbone.

Les composés du type I(e) dans la formule desquels Z désigne

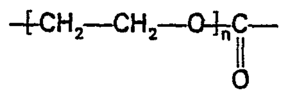

peuvent être préparés par un procédé en trois stades:

1) Condensation du thioéthanol avec le tétramère de l'épichlorhydrine de formule (III).
2) Addition de 1 à 20 moles d'oxyde d'éthylène sur le composé obtenu au premier stade.
3) Estérification du composé obtenu au deuxième stade par un acide de formule $R_4COOH$.

Pour préparer les composés du type (e) dans lesquels Z désigne

$—(CH_2)_x—COO—$ $—(CH_2)_x—COOCH_2—CHOH—$

$—(CH_2)_x—CONH—$ 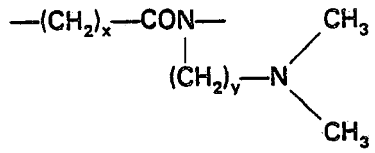

où x désigne 1 ou 2 et y désigne 2 ou 3, on procède en deux stades.

Dans un premier stade on fait réagir le tétramère de l'épichlorhydrine de formule (III) avec un ester méthylique ou éthylique d'un acide mercaptoacétique ou mercapto propionique de formule:

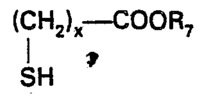

où x désigne 1 ou 2 et $R_7$ désigne $CH_3$ ou $C_2H_5$.

Les réactions de condensation des mercaptans et des esters d'un acide mercapto acétique ou propionique avec le tétramère de l'épichlorhydrine de formule (III) sont réalisées en présence d'un composé basique, par exemple un alcoolate alcalin tel que le méthylate ou l'éthylate de sodium ou de potassium, de préférence dans des solvants, a'une température comprise entre 80 et 150°C.

Les solvants utilisables sont les alcanols ayant de 1 à 4 atomes de carbone ou les alkoxyéthanols tels que le méthoxy-, éthoxy-, butoxy-, éthanol, en présence éventuellement d'une faible quantité d'eau, lorsqu'on utilise les alcoyl, hydroxyalcoyl ou alcoyl mono- ou poly-éthoxy mercaptans.

Dans un second stade on transforme le composé intermédiaire de formule (IV) ci-après, obtenu dans le premier stade.

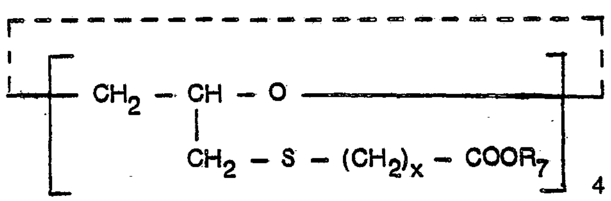
(IV)

où x et $R_7$ ont les significations ci-dessus indiquées, en composés du type I(e) ci-dessus définis.

En faisant réagir le composé intermédiaire de formule (IV) avec un alcool de formule $R_{4a}OH$ où $R_{4a}$ désigne un radical alcoyle, hydroxyalcoyle ou alcényle ou alcoylphenyle ayant de 6 à 20 atomes de carbone, la partie alcoyle du radical alcoylphenyle ayant jusqu'à 14 atomes de carbone on obtient le composé de formule (V):

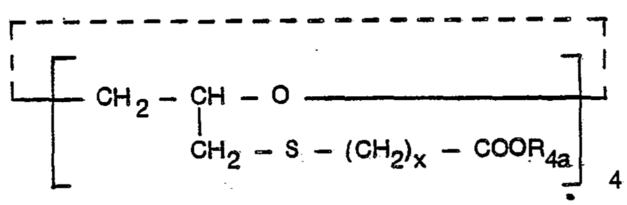
(V)

$R_{4a}$ ayant la signification ci-dessus indiquée.

Par réaction du composé intermédiaire de formule (IV A)

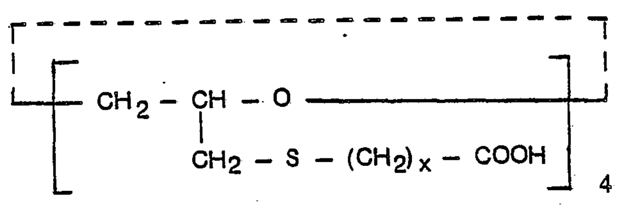
(IV A)

obtenu par saponification ou hydrolyse du composé de formule (IV) avec les époxy-1,2 alcanes de formule:

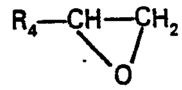

$R_4$ ayant la signification ci-dessus indiquée on obtient les composés de formule (VI)

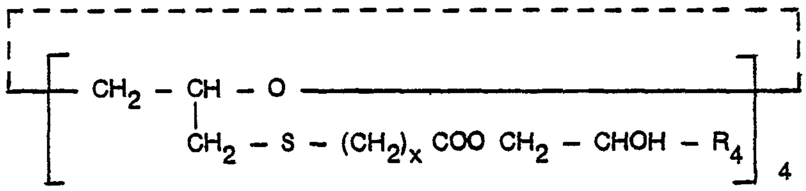

(VI)

Ces réactions sont réalisées en présence de catalyseurs alcalins tels que méthylate ou éthylate de sodium ou de potassium, à une température comprise entre 60°C et 140°C.

En faisant réagir le composé intermédiaire de formule (IV) sur une amine primaire de formule $R_4NH_2$, $R_4$ ayant la signification ci-dessus indiquée, on obtient, après élimination de méthanol ou d'éthanol le composé de formule:

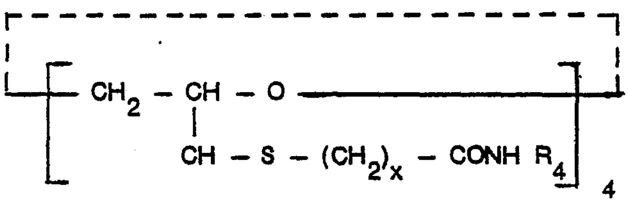

(VII)

En faisant réagir le composé intermédiaire de formule (IV) avec une amine secondaire de formule:

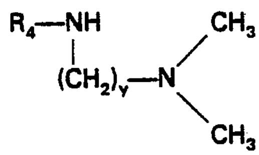

où $R_4$ et y ont les significations ci-dessus indiquées on obtient le composé tensio-actif de formule:

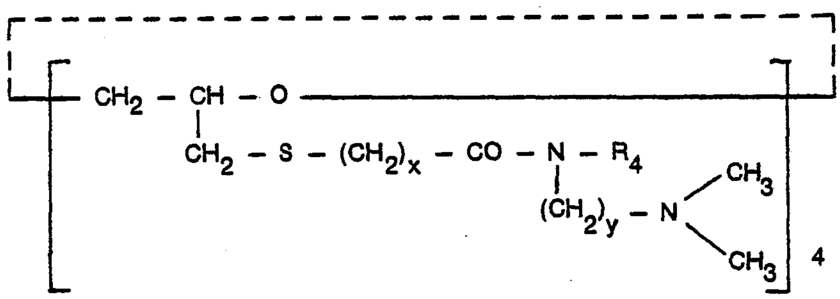

(VIII)

Les réactions du composé intermédiaire de formule (IV) avec une amine primaire ou secondaire s'effectuent généralement à une température comprise entre 20 et 120°C en présence éventuellement de méthylate de sodium ou de potassium.

D'une façon générale les composés du type I(e) répondant à la formule (I) dans laquelle A désigne le groupement

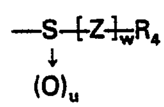

$R_4$, u et w ont les significations ci-dessus indiquées pour le groupement ($a_1$), Z désigne des groupements d'atomes à caractère hydrophile se présentant sous la forme d'un radical renfermant un ou plusieurs groupements éther, ester, amide, amine, ammonium et/ou hydroxyle peuvent être préparés à partir du tétramère de l'épichlorhydrine de formule (III) en un, deux, trois ou quatre stades.

Les composés de type I(e) à groupement thioéther ainsi préparés peuvent éventuellement être oxydés en sulfoxydes avec de l'eau oxygénée à 30—35%, en quantité stoechiométrique, à la

température de 20—50°C et de préférence de 30—35°C, en présence de 0,1 à 10% d'acide carboxylique ayant de 1 à 4 atomes de carbone et de préférence en présence d'acide acétique et éventuellement en présence d'un solvant usuel.

Les composés à groupement thioéther et sulfoxyde peuvent être transformés respectivement en composés sulfonium et sulfoxonium par alcoylation avec un agent alcoylant classique et de préférence avec le bromure ou iodure de méthyle ou le sulfate de diméthyle.

Les groupements amine tertiaires des composés du type I(e) peuvent être transformés en groupements ammonium quaternaires par alcoylation avec un agent alcoylant classique et de préférence avec les bromures ou iodures de méthyle ou le sulfate de diméthyle.

Les polyéthers cycliques tensio-actifs du type I($e_1$) répondant à la formule:

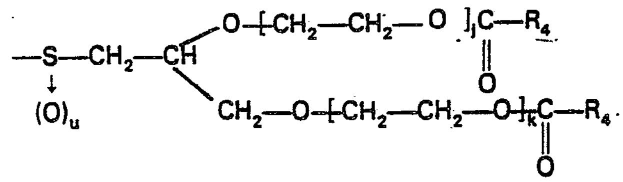

où j et k désignent chacun un nombre quelconque inférieur à 20 et la somme j + k est comprise entre 1 et 20;

$R_4$ a la signification ci-dessus indiquée,

peuvent être préparés par condensation du thioglycérol avec le tétramère de l'épichlorhydrine de formule (III) suivie d'une oxyéthylénation du dérivé obtenu puis estérification avec un acide de formule $R_4COOH$.

Les composés ainsi obtenus peuvent être oxydés et/ou alcoylés comme les composés du type I(e).

Les composés du type I(f) répondant à la formule (I) dans laquelle A désigne le groupement:

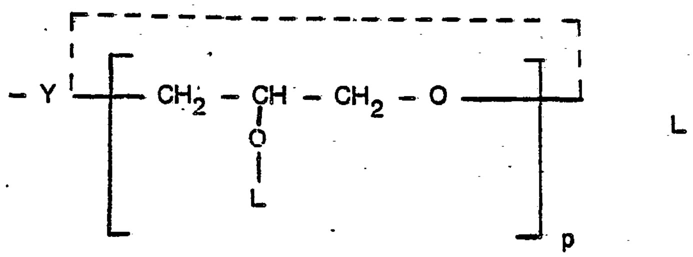

où Y, L et p ont les significations ci-dessus indiquées, peuvent être préparés de deux façons différentes selon la signification de Y.

Lorsque Y désigne un atome d'oxygène, les composés du type I(f) peuvent être préparés par réaction, en catalyse alcaline, du dérivé hydroxylé du tétramère de l'épichlorhydrine (composé de formule II figurant ci-après) avec le glycidol, le composé intermédiaire obtenu réagissant ensuite avec un oxyde d'alcoylène de formule:

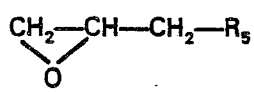

ou avec un alcoyle glycidyléther de formule:

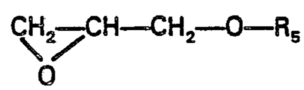

Lorsque Y désigne un atome de soufre ou un groupement sulfoxyde, les composés du type I(f) peuvent être préparés par un procédé en trois stades.

Dans un premier stade on prépare un composé intermédiaire de formule:

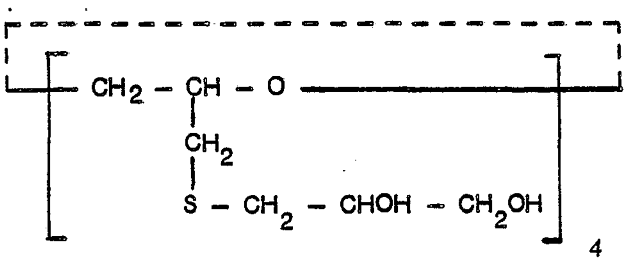

(IX)

à une température de 80—150°C, par réaction du tétramère de l'épichlorhydrine de formule (III) avec le thioglycérol, en présence d'un solvant choisi parmi les alcanols inférieurs ayant de 1 à 4 atomes de carbone et en présence d'un composé alcalin choisi avantageusement parmi les hydroxydes alcalins tels que l'hydroxyde de sodium ou de potassium et les alcoolates alcalins, par exemple le méthylate ou l'éthylate de sodium ou de potassium.

Dans un second stade on fait réagir sur ce composé intermédiaire (IX) 0 à 9 moles de glycidol et/ou d'oxydes d'alcoylène ayant de 8 à 20 atomes de carbone et/ou d'alcoyl glycidyl éther (tels que ceux mentionnes ci-dessus), à une température de 120—170°C et de préférence aux environs de 150°C, en présence d'un composé alcalin de même type que celui utilisé dans le premier stade, ce composé obtenu dans le second stade pouvant éventuellement être estérifié avec un acide sulfocarboxylique ayant de 8 à 20 atomes de carbone.

On utilise de préférence le glycidol qui réagit essentiellement avec le groupement alcool primaire du composé intermédiaire de formule (IX). Une petite fraction du glycidol peut réagir avec le groupement alcool secondaire.

Lorsque dans le second stade on fait réagir *r* moles de glycidol (*r* désigne un nombre entier ou décimal de 0 à 9) on obtient un composé tensio-actif représenté essentiellement par la formule:

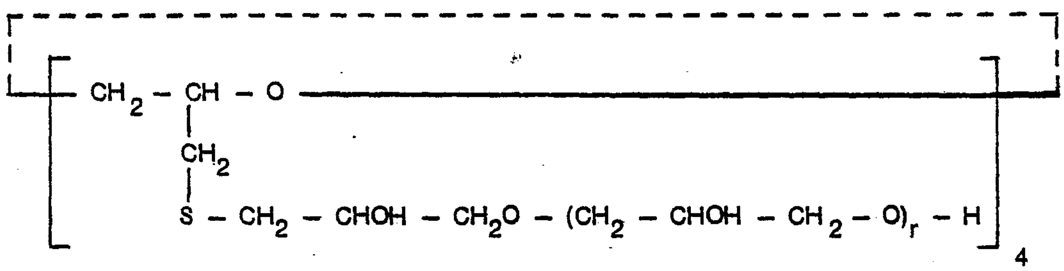

(X)

Il est possible de faire réagir, en un troisième stade, sur ce composé (X) *s* moles (*s* désigne un nombre entier ou décimal de 1 à 9) d'un oxyde d'alcoylène et/ou d'un alcoyl glycidyl éther et/ou d'un acide sulfocarboxylique et/ou d'un acide carboxylique ayant de 8 à 20 atomes de carbone.

Par réaction d'un oxyde d'alcoylène sur le composé intermédiaire de formule (X) on obtient le composé du type I(f) dans lequel L désigne $—CH_2—CHOH—CH_2—R_5$.

Par réaction d'un alcoylglycidyléther sur le compose de formule (X) on obtient le composé du type I(f) dans lequel L signifie

$$—CH_2—CHOH—CH_2—O—R_5.$$

Les fonctions hydroxyles peuvent être estérifiées par un acide carboxylique de formule $R_5—CH_2—COOH$ ou par un acide $\alpha$-sulfocarboxylique de formule:

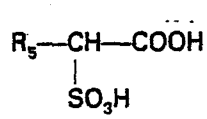

où $R_5$ a la signification ci-dessus indiquée.

Les composés du type I(f) à groupement thioéther peuvent éventuellement être oxydés en sulfoxydes avec de l'eau oxygénée.

Les composés du type I(f) ainsi que leurs sulfoxydes peuvent également être alcoylés et transformés respectivement en composés sulfonium ou sulfoxonium.

L'oxydation et l'alcoylation s'effectuent comme pour les composés du type I(e).

Par hydrolyse du polyéther cyclique polychloré tétramère de l'épichlorhydrine de formule (III) on obtient le composé de formule (II):

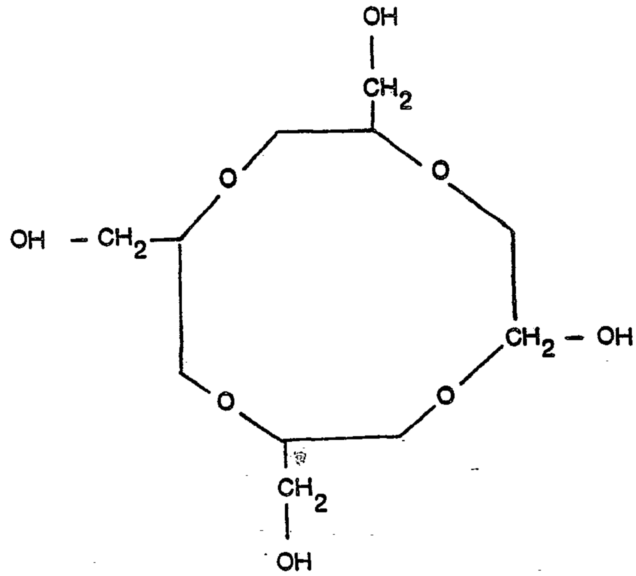

(II)

De façon simplifiée cette formule s'écrit:

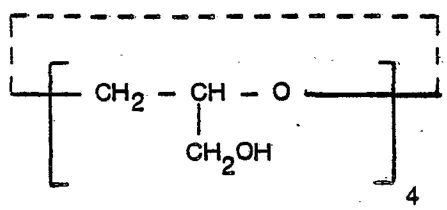

(IIa)

On réalise l'hydrolyse du polyéther cyclique polychloré de formule (III) en remplaçant les atomes de chlore par un groupement —OH, par réaction avec un sel alcalin d'acide carboxylique et de préférence avec l'acétate de sodium ou de potassium à une température de 100 à 190°C dans un solvant approprié choisi avantageusement parmi les glycols et les dérivés de glycol et de préférence parmi l'éthylèneglycol, le butylèneglycol, le diéthylèneglycol et ses éthers, le propylèneglycol, le dipropylèneglycol, l'hexylèneglycol et le 2-butoxyéthanol; l'ester acétique formé est ensuite décomposé par saponification au moyen d'hydroxyde de sodium ou de potassium ou par alcoolyse au moyen d'un alcool inférieur et de préférence au moyen de méthanol ou d'éthanol en présence d'un catalyseur basique choisi de préférence parmi le méthylate ou l'éthylate de sodium ou de potassium.

Par réaction de quatre fois *v* moles d'oxyde d'éthylène sur le dérivé hydroxylé du tétramère de l'épichlorhydrine de formule (II) on obtient un composé intermédiaire de formule:

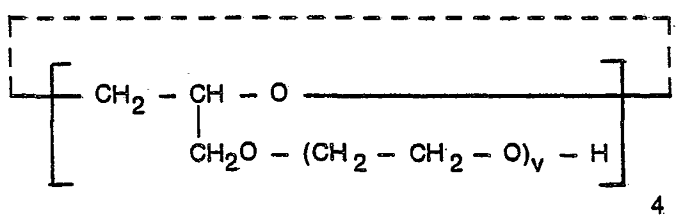

$(X_1)$

dont les fonctions hydroxyle peuvent être estérifiées soit avec un acide sulfocarboxylique de formule

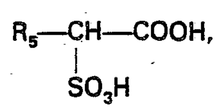

soit avec un acide carboxylique de formule $R_5$—$CH_2$—COOH, où $R_5$ désigne un radical alcoyle linéaire ayant de 6 à 18 atomes de carbone.

On obtient ainsi un composé du type I(g) répondant à la formule (I) dans laquelle A désigne le groupement

$$-O-\!\!\left[CH_2-CH_2O\right]_v\!\!-\underset{\underset{O}{\|}}{C}-\underset{\underset{R_6}{|}}{CH}-R_5$$

où $R_6$ désigne soit $SO_3H$ soit H, v désigne un nombre quelconque égal ou inférieur à 20, à l'exception du nombre zéro.

On peut également faire réagir sur le composé de formule (II) directement soit un acide sulfocarboxylique de formule:

$$R_5-\underset{\underset{SO_3H}{|}}{CH}-COOH$$

où $R_5$ désigne un radical alkyle linéaire ayant de 6 à 18 atomes de carbone. On obtient ainsi un composé du type I(g) dans lequel $R_6$ désigne $SO_3H$ et *v* désigne le nombre 0.

Cette réaction s'effectue à une température de 100°C à 120°C et on élimine l'eau formée.

Les polyéthers cycliques tensio-actifs de formule (I) selon l'invention se présentent sous la formule d'huile épaisse, de pâte ou de cire généralement solubles ou dispersibles dans l'eau.

Ils abaissent la tension superficielle de l'eau et permettent à de très faibles concentrations, la solubilisation de produits non hydrosolubles.

Parmi les composés non hydrosolubles pouvant être solubilisés par les polyéthers cycliques tensio-actifs de formule (I) il faut citer les colorants, les parfums, certains produits pharmaceutiques.

Outre la solubilisation de ces produits les composés tensio-actifs cycliques de formule (I) peuvent permettre de solubiliser ou de disperser des composés minéraux ou polaires en milieu organique ou des composés hydrophobes en milieu aqueux.

Les composés de l'invention peuvent être utilisés non seulement dans des compositions cosmétiques et pharmaceutiques, mais également dans les industries du textile, des teintures, des insecticides et des industries similaires.

Les compositions cosmétiques et pharmaceutiques renferment le plus souvent au moins $0{,}5^{-2}$ gramme par litre ou 0,5 $10^{-3}$% en poids de polyéther cyclique tensio-actif de formule (I).

Les compositions cosmétiques englobent notamment les compositions destinées aux soins de la peau, des ongles, des cheveux.

Les compositions pour les soins des cheveux visent notamment les shampooings et les compositions de conditionnement des cheveux, ainsi que les compositions tinctoriales.

Les compositions cosmétiques peuvent se présenter sous la forme d'une solution aqueuse ou hydroalcoolique, ou sous la forme d'une crème, d'un gel, d'une émulsion ou d'un aérosol.

Les solutions hydroalcooliques renferment généralement un alcool ayant de 1 à 4 atomes de carbone et de préférence l'éthanol ou l'isopropanol, avantageusement en une proportion de 5 à 70% du poids total de la composition.

Parmi les compositions cosmétiques il faut citer les compositions cosmétiques pour le traitement des cheveux, notamment les shampooings, les shampooings colorants et les compositions tinctoriales contenant en solution dans un solvant choisi dans un groupe formé par l'eau et une solution hydroalcoolique, une quantité cosmétiquement efficace d'un ou plusieurs polyéthers cycliques tensio-actifs de formule (I).

Les compositions cosmétiques pour le traitement des cheveux et en particulier les shampooings, les shampooings colorants et les compositions tinctoriales peuvent également contenir en plus d'un polyéther cyclique tensio-actif de formule (I), également un adjuvant choisi dans le groupe formé par les tensio-actifs anioniques, cationiques, amphotères, zwitterioniques ou non-ioniques et leurs mélanges, les parfums, les colorants, les conservateurs, les agents moussants, les stabilisateurs de mousse, les agents adoucissants, les agents de restructuration des cheveux, les agents anti-pelliculaires, les résines cosmétiques, les séquestrants, les épaississants, les synergistes de mousse, les électrolytes.

Les shampooings colorants et les compositions tinctoriales peuvent en outre contenir un ou plusieurs colorants directs et en particulier des colorants anthraquinoniques, des colorants azoïques, des colorants nitrés de la série benzénique, des indophénols, des indoanilines, des indamines.

Le pH des compositions est généralement compris entre 3 et 11.

L'invention a également pour objet un procédé de traitement des cheveux consistant à appliquer sur les cheveux une quantité efficace d'une composition aqueuse ou hydroalcoolique renfermant un ou plusieurs polyéthers cycliques tensio-actifs de formule (I) et éventuellement un ou plusieurs adjuvants ci-dessus définis.

L'invention a également pour objet les polyéthers cycliques tensio-actifs de formule générale:

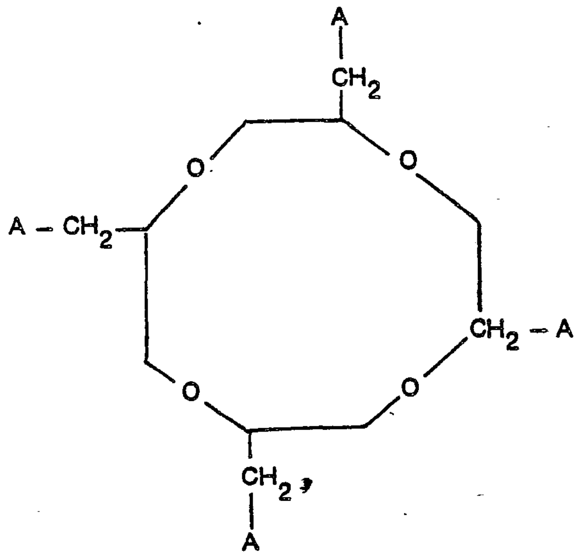

(I)

dans laquelle A désigne un ensemble amphiphile constitué d'une partie hydrophile et d'une partie lipophile, cet ensemble amphiphile étant relié par la partie hydrophile au cycle par liaison covalente; la partie hydrophile comporte un ou plusieurs groupements choisis parmi les suivants:

a) le groupement

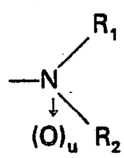

où $R_1$ et $R_2$, identiques ou différents, désignent des radicaux aliphatiques et de préférence alcoyle, hydroxyalcoyle ou alcényle ayant de 1 à 20 atomes de carbone, l'un des radicaux comportant au moins 8 atomes de carbone; ou des radicaux aliphatiques substitués ayant de 6 à 20 atomes de carbone ou des radicaux alcoylaryle ayant de 6 à 20 atomes de carbone; la somme des atomes de carbone de $R_1$ et $R_2$ étant, de préférence, inférieure ou égale à 28; l'un des radicaux peut, en outre, désigner un radical diméthylaminoéthyle ou propyle, diéthylaminoéthyle ou propyle, pipéridino éthyle ou propyle, morpholino éthyle ou propyle, alcoyl polyhydroxy propylène;

*u* désigne zéro ou 1;

$a_1$) le groupement

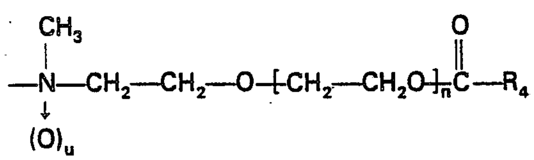

où $R_4$ désigne un radical aliphatique non substitué ou substitué ou un radical alcoylaryle ayant de 6 à 20, en particulier de 8 à 16 et plus particulièrement de 12 à 16 atomes de carbone et de préférence un radical alcoyle, hydroxyalcoyle, alcényle et alcoylphényle, la partie alcoyle du radical alcoylphényle ayant jusqu'à 14 atomes de carbone;

*n* désigne un nombre quelconque de 1 à 20, avantageusement de 1 à 10, en particulier de 2 à 8 et plus particulièrement de 4 à 6;

*u* désigne le nombre 0 ou 1;

$a_2$) le groupement

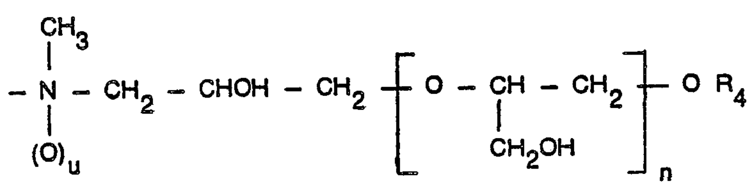

*u*, $R_4$ et *n* ayant la signification ci-dessus indiquée.

b) le groupement

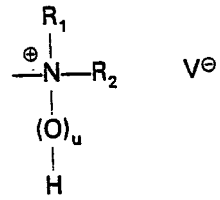

$b_1$) le groupement

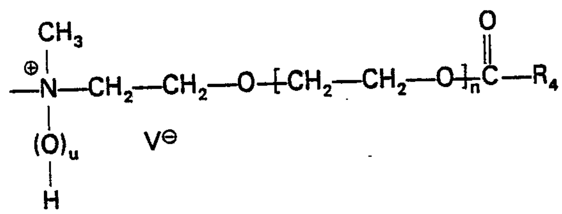

$b_2$) le groupement

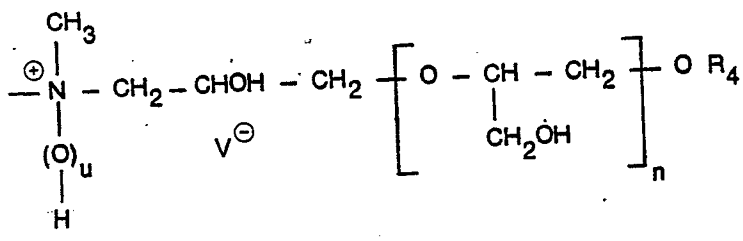

où *u* désigne le nombre 0 ou 1
*n* désigne un nombre quelconque de 1 à 20, avantageusement de 1 à 10, en particulier de 2 à 8 et plus particulièrement de 4 à 6;
$V^{\ominus}$ désigne un anion et de préférence un anion formiate, acétate, citrate ou lactate, $R_1$, $R_2$, $R_4$ ont les significations indiquées précédemment.

c) le groupement

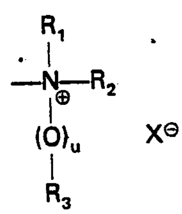

$R_1$, $R_2$ et *u* ayant la même signification que ci-dessus,
$R_3$ désigne un radical alcoyle ou hydroxyalcoyle ayant de 1 à 3 atomes de carbone et de préférence un radical méthyle, éthyle, hydroxyéthyle, dihydroxypropyle; ou un radical benzyle;
$X^{\ominus}$ désigne un anion et de préférence un chlorure, bromure, iodure, monométhyl sulfate, méthyl sulfonate, p.toluène sulfonate;

$c_1$) le groupement

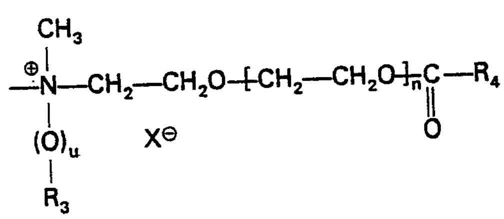

$c_2$) le groupement

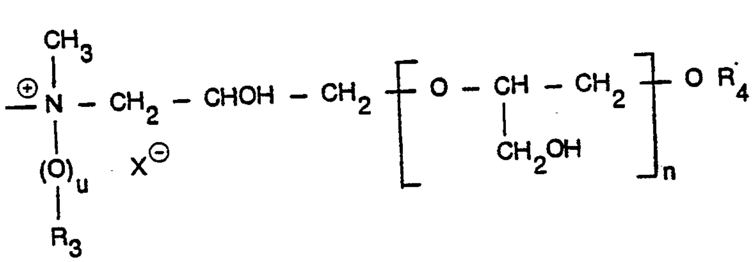

dans ces groupements ($c_1$) et ($c_2$)
*u* désigne le nombre 0 ou 1
*n* désigne un nombre quelconque de 1 à 20, avantageusement de 1 à 10, en particulier de 2 à 8 et plus particulièrement de 4 à 6;
$R_3$, $R_4$ et $X^\ominus$ ont les significations ci-dessus indiquées

d) le groupement

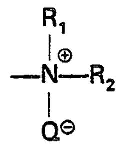

où $Q^\ominus$ désigne l'un des groupements suivants:

$$—(CH_2)_m—COO^\ominus \text{ où } m \text{ désigne 1, 2 ou 3}$$

$$—CH_2—CH_2—CH_2—SO_3^\ominus \qquad —CH_2—CH_2\ O\ SO_2^\ominus$$

d$_1$) le groupement

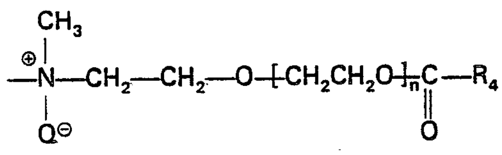

d$_2$) le groupement

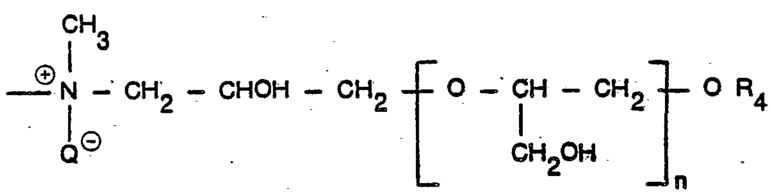

dans les groupements ($d_1$) et ($d_2$) *n* désigne un nombre quelconque de 1 à 20, avantageusement de 1 à 10, en particulier de 2 à 8 et plus particulièrement de 4 à 6;
$Q^\ominus$ a la signification indiquée pour le groupement (d).

e) le groupement

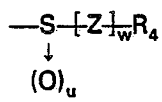

où $R_4$ a la signification indiquée pour le groupement ($a_1$);
*u* désigne zéro ou 1;
*w* désigne zéro ou 1;
Z désigne des groupements d'atomes à caractères hydrophile qui peuvent être éther, ester, amide, amine, ammonium et/ou hydroxyle.

Comme exemple de groupements Z on peut citer les suivants:

$—CH_2—CHOH—$ $\quad —(CH_2—CH_2O)_n—$ 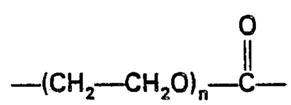

$—(CH_2)_x—COO—$ $\quad —(CH_2)_x—COOCH_2—CHOH—$

$—(CH_2)_x—CONH—$ 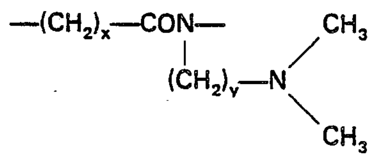

où x représente 1 ou 2; y représente 2 ou 3; *n* représente un nombre quelconque de 1 à 20.

$e_1$) le groupement

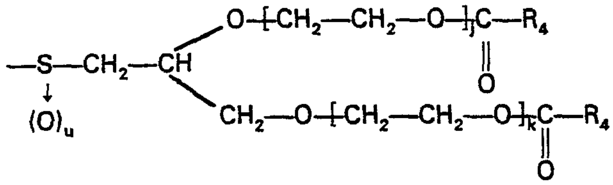

j et k, identiques ou différents, désignent chacun un nombre quelconque inférieur à 20, la somme j + k étant comprise entre 1 et 20.

f) le groupement

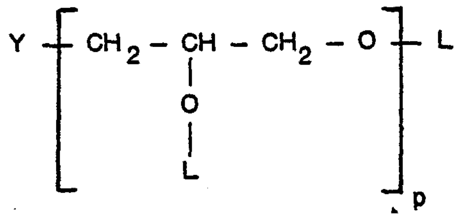

où L désigne en même temps un atome d'hydrogène et l'un des groupements:

$$-CH_2-CHOH-CH_2-(O)_q-R_5$$

où q désigne 0 ou 1

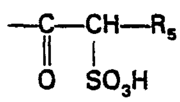
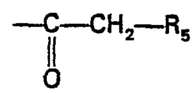

l'atome d'hydrogène et l'autre groupement étant répartis de façon statistique;
$R_5$ désigne un radical alcoyle linéaire ayant de 6 à 18 et en particulier de 8 à 16 atomes de carbone;
p désigne un nombre quelconque de 1 à 10, en particulier de 2 à 8, plus particulièrement de 3 à 6, et représente une valeur statistique moyenne;
Y désigne:

$-O-$; $-S-$ ou 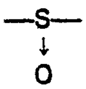

g) le groupement

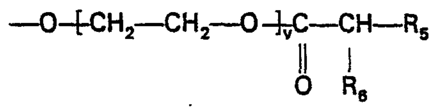

où v désigne un nombre quelconque de 0 à 20, en particulier de 0 à 10, plus particulièrement de 0 à 5;
$R_6$ désigne un atome d'hydrogène ou le radical $-SO_3H$; $R_5$ ayant la signification ci-dessus indiquée;

h) le groupement (e) ou ($e_1$) alcoylé par un agent d'alcoylation choisi dans le groupe formé par le bromure de méthyle, l'iodure de méthyle et le sulfate de diméthyle et de préférence par le sulfate de diméthyle;

i) le groupement (f) alcoylé par un agent d'alcoylation choisi dans le groupe formé par le bromure de méthyle, l'iodure de méthyle et le sulfate de diméthyle et de préférence par le sulfate de diméthyle;

Les polyéthers cycliques tensio-actifs de type Ia répondant à la formule (I) dans laquelle A désigne le groupement (a)

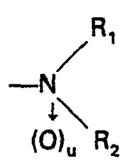

ne sont pas solubles dans l'eau sous cette forme, mais ils peuvent conduire à des produits solubles par salification ou alcoylation.

Les polyéthers cycliques tensio-actifs de formule (I) dans laquelle A désigne l'un des groupements ($a_1$), ($a_2$), (b), ($b_1$), ($b_2$), (c), ($c_1$), ($c_2$), (d), ($d_1$), ($d_2$), (e), ($e_1$), (f), (g), (h), (i) sont généralement solubles dans l'eau.

Les polyéthers cycliques tensio-actifs selon l'invention sont tous obtenus à partir du polyéther cyclique polychloré, tétramère de l'épichlorhydrine, de formule:

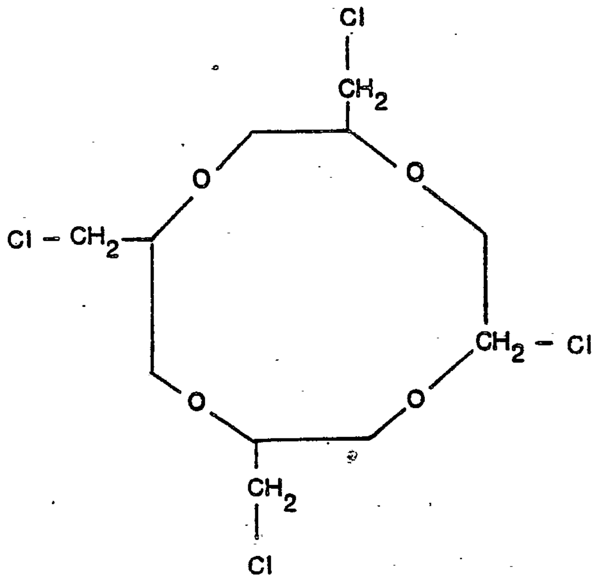

(III)

L'invention sera mieux comprise à l'aide des exemples non limitatifs ci-après.

Dans un but de simplification, on utilisera dans ces exemples, les formules simplifiées du type:

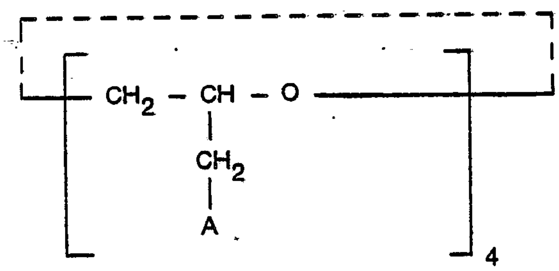

(Ia)

EXEMPLE 1

Préparation du composé tensio-actif cyclique du type I(a) de formule:

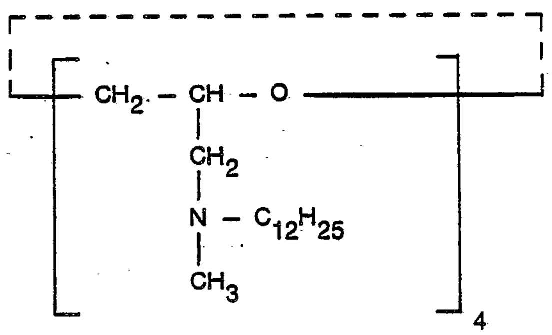

(XI)

Dans un premier stade on prépare le tétramère de l'épichlorhydrine de formule (III)

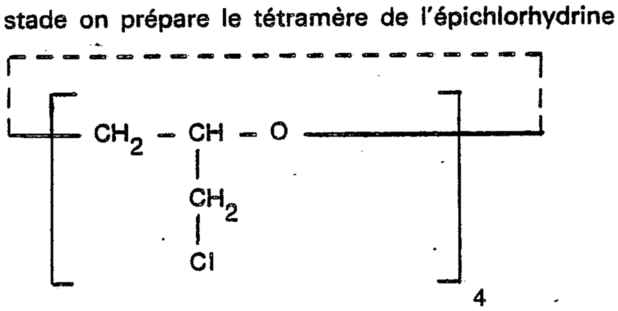

Dans un réacteur de 6 litres, on mélange: 1180 g d'épichlorhydrine et 500 ml de tétrachlorure de carbone.

On refroidit le mélange à 10°C par immersion dans un bain de glace.

Sous vive agitation, on ajoute, en 6 heures, 16 g d'éthérate de trifluorure de bore en solution dans 1 litre de tétrachlorure de carbone.

On maintient pendant ce temps le température entre 10 et 13°C.

On laisse ensuite monter la température et le mélange est porté, progressivement, au reflux pendant 1 heure.

On ajoute 50 g de carbonate de sodium sec, finement pulvérisé. Le mélange est agité 2 heures au reflux. Les sels minéraux sont séparés par filtration. Le tétrachlorure de carbone est évaporé sous pression réduite, puis, on fractionne par distillation.

On recueille ainsi, à 185°C, sous 0,2 mm de Hg, 210 g de produit qui se présente sous la forme d'une masse vitreuse incolore cristallisant au bout de quelques jours.

Dosage de chlore organique: 10,6 meq/g.

Dans un second stade on prépare le composé tensio-actif cyclique de formule (XI).

On chauffe sous atmosphère d'azote, à la température de 140°C, pendant 12 heures:

14,8 g (0,04 mole) du composé III et

64,8 g (0,32 mole) de N-méthyl laurylamine.

Après retour à la température du laboratoire, on ajoute, sous agitation, 150 ml de pentane. Le chlorhydrate de méthyl laurylamine est essoré, puis lavé avec deux fois 50 ml de pentane.

Le filtrat est concentré, d'abord à pression ordinaire, puis sous 30 mm de Hg en élevant la température du bain jusqu'à 90°C.

On obtient 43,5 g de produit que l'on sèche par chauffage pendant 6 heures à l'étuve à 105°C.

Indice de basicité: 3,84 meq/g Théorie: 3,90 meq/g.

Le composé tensio-actif ainsi obtenu est parfaitement soluble dans l'eau en présence d'acide lactique.

## EXEMPLE 2

Préparation du composé tensio-actif cyclique du type I(c), de formule:

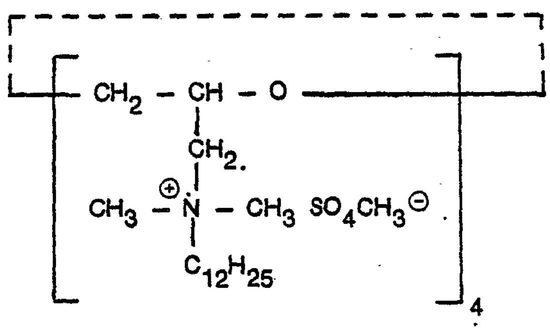

(XII)

On dissout 15 g de composé basique (0,051 mole) de l'exemple 1 dans 20 g de méthanol pur.

Sous agitation on ajoute 6,2 g (0,048 mole) de sulfate de diméthyle en 35 minutes. La température s'élève jusqu'à 43°C. Le mélange est agité pendant 4 heures à 45°C. Le solvant est éliminé par distillation sous pression réduite à 40°C.

On obtient une pâte brun clair, soluble à chaud dans l'eau, soluble à froid dans une solution hydroéthanolique contenant 50% d'éthanol.

## EXEMPLE 3

Préparation du composé tensio-actif cyclique de type I(a) de formule:

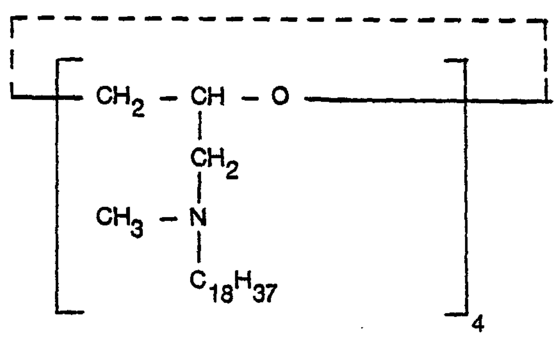

(XIII)

On dissout 9,25 g (0,025 mole) de tétramère d'épichlorhydrine de formule (III) et 28,3 g (0,1 mole) de N-méthyl stéarylamine, dans 30 g de diméthyléther du diéthylèneglycol.

Le mélange est chauffé à 135°C pendant 30 heures et l'acide est neutralisé au fur et à mesure de

son apparition avec du méthylate de sodium (0,090 meq/g). Le chlorure de sodium est séparé par filtration à chaud. Le solvant est éliminé par distillation sous pression réduite.

On obtient un solide brun, soluble dans l'eau en présence d'acide lactique, et présentant les caractéristiques suivantes:

| | | |
|---|---|---|
| Indice de basicité | : 2,63—2,64 meq/g | Théorie : 2,93 meq/g |
| Dosage de chlore | : 0 | |
| Point de goutte | : 33°C. | |

EXEMPLE 4

Préparation du composé tensio-actif cyclique du type I(c), de formule:

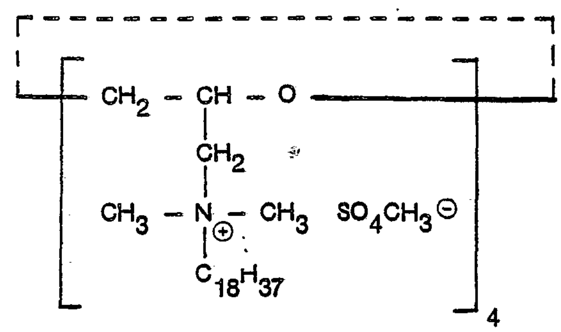

(XIV)

On ajoute 10 g de méthanol à 7,9 g (0,020 mole) de tétramère de l'épichlorhydrine préalablement fondu.

A la température de 30°C, on ajoute, sous vive agitation, 2,5 g (0,020 mole) de sulfate de diméthyle en solution dans 5 g de méthanol. La durée d'addition est de 5 minutes, la température s'élève à 45°C. Le mélange est agité pendant 2 heures à 45°C.

Le méthanol est éliminé par distillation sous une pression de 40 mm de Hg.

On obtient 10,4 g de produit tensio-actif de formule ci-dessus qui se présente sous la forme d'une pâte dure de couleur brune, soluble dans l'eau.

EXEMPLE 5

Préparation du composé tensio-actif cyclique du type I(a), de formule:

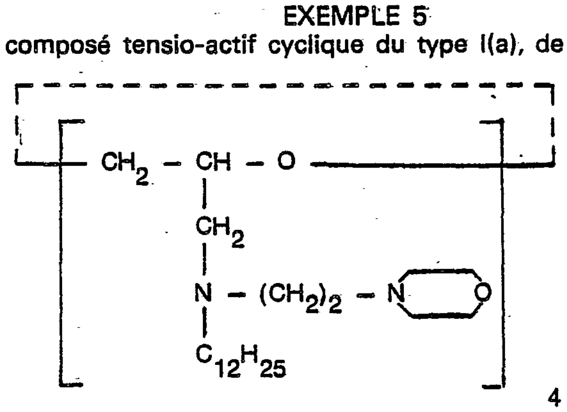

(XV)

A 7,4 g (0,02 mole) de tétramère de l'épichlorhydrine fondu, on ajoute 25 g (0,08 mole) de N-morpholino-éthyl laurylamine.

Le mélange est chauffé pendant 35 heures à 135°C.

Toutes les 3 heures un indice d'acide est effectué, et l'acide formé est neutralisé avec une quantité correspondante de méthylate de sodium. On ajoute ensuite 20 ml de benzène. Le chlorure de sodium est séparé par filtration et lavé avec deux fois 5 ml de benzène. Le solvant est éliminé par distillation sous pression réduite.

On obtient un produit tensio-actif qui est soluble dans l'eau en présence d'acide lactique.

EXEMPLE 6

Préparation du polyéther cyclique tensio-actif du type I(c), obtenu par quaternisation du composé de l'exemple 5 avec du sulfate de diméthyle.

A 6,4 g (36 meq en basicité) de composé (XV) préparé dans l'exemple 5, on ajoute, à 20°C, 2,26 g (18 meq) de sulfate de diméthyle.

Après 2 heures d'agitation, on obtient une huile brune épaisse soluble dans l'eau.

EXEMPLE 7

Préparation du composé tensio-actif cyclique du type I(i), de formule:

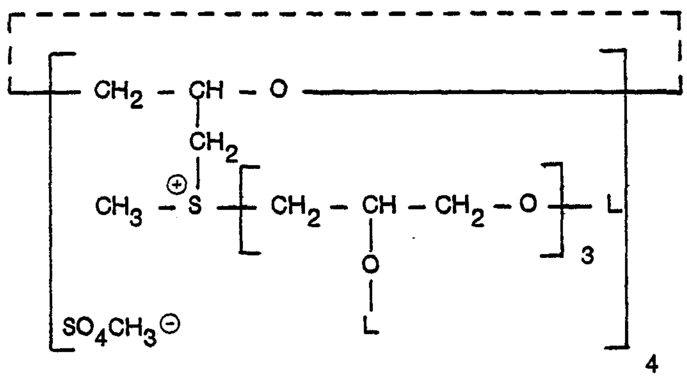

(XVI)

L désigne de l'hydrogène et le groupement $—CH_2—OH—C_{10}H_{21}$, répartis de façon statistique dans les proportions 3/1.

a) Dans un premier stade on prépare un composé intermédiaire de formule:

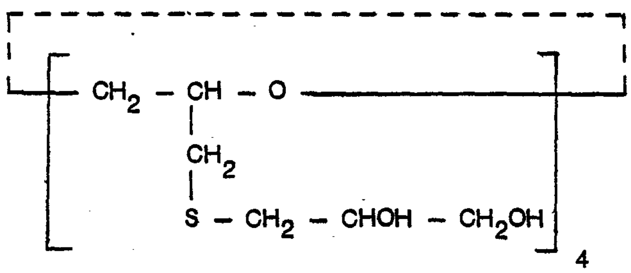

(XVII)

On dissout, dans 50 g d'éthanol absolu:
18,5 g (0,05 mole) de tétramère de l'épichlorhydrine
24,2 g (0,2 mole) de thioglycérol
32 g de liqueur méthanolique de méthylate de sodium à 6,3 meq/g.

Le mélange est porté à reflux pendant 24 heures. Le chlorure de sodium est séparé par filtration et lavé avec 30 ml d'éthanol absolu.

Après élimination du solvant par chauffage sous pression réduite, on obtient le composé recherché dont l'indice d'hydroxyle est de 11,2 meq/g et dont la teneur en soufre est de 19%.

b) Dans un second stade on ajoute à 14 g (170 meq de groupement hydroxyle) de composé intermédiaire préparé au premier stade, 0,42 g de méthylate de sodium dans le méthanol (contenant 5 meq/g de méthylate de sodium), puis à la température de 150°C, 12,5 g de glycidol (170 meq) en l'espace d'une heure.

c) Dans un troisième stade, à 5,65 g (0,005 mole) de composé intermédiaire préparé au second stade, on ajoute 0,2 g de méthylate de sodium, puis à la température de 150°C, 3,7 g d'époxy-1,2 dodécane (0,02 mole) en solution dans 7 ml de solution d'isopropanol à 85%, en distillant au fur et à mesure l'isopropanol et l'eau.

Le mélange réactionnel est chauffé pendant 7 heures à 150°C.

d) Dans un quatrième stade, on prépare le composé tensio-actif cyclique de formule (XVI).

On dissout, dans 7 ml de méthanol, 6,8 g de produit préparé précédemment puis on ajoute, à 45°C, en 10 minutes, 1,83 g de sulfate de diméthyle solubilisé dans 7 ml de méthanol.

Après 30 minutes de chauffage à reflux, le méthanol est éliminé par distillation sous pression réduite.

Le produit ainsi obtenu se présente sous la forme d'une pâte brune, soluble dans l'eau avec une légère opalescence.

EXEMPLE 8

Préparation du composé tensio-actif cyclique du type I(f), de formule:

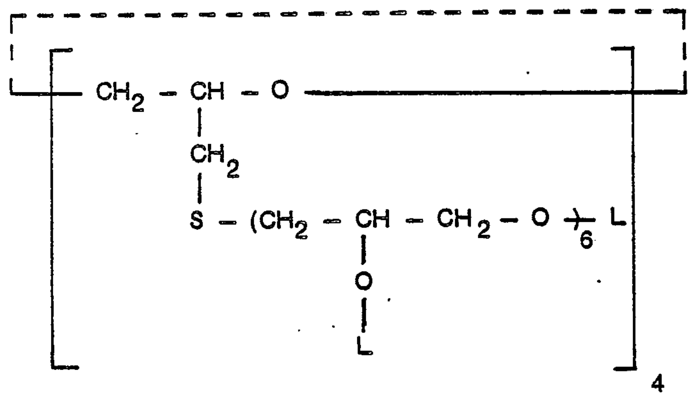

(XVIII)

L désigne de l'hydrogène et le groupement $—CH_2—CHOH—C_{10}H_{21}$ dans les proportions 6/1 répartis de façon statistique.

A 8,3 g de composé intermédiaire de formule (XVII) préparé dans l'exemple 7), contenant 100 meq (milliéquivalent) en groupement hydroxyle, on ajoute 2,2 g de méthylate de sodium à 4,6 meq/g, puis à 150°C, en l'espace de 1 heure 20 minutes, 19 g de glycidol (250 meq).

Le chauffage est maintenu encore pendant 20 minutes après la fin de l'addition.

On ajoute ensuite 9,2 g (50 meq) d'époxy-1,2 dodécane à la température de 150°C en l'espace de 1 heure 20 minutes. Le chauffage est ainsi maintenu pendant 6 heures.

Le produit obtenu se présente sous la forme d'une pâte brune soluble dans l'eau.

EXEMPLE 9

Procédé de préparation d'un composé tensio-actif cyclique du type I(c) de formule:

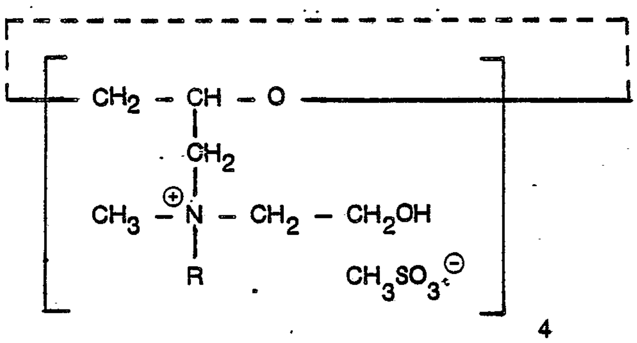

(XIX)

R = mélange de radicaux alcoyle en $C_{12}—C_{15}$ dérivés des alcools "Dobanol 25"® vendus par SHELL.

Le Dobanol 25 renferme des alcools primaires en $C_{12}—C_{15}$ dans les proportions en poids approximatives suivantes:

| | |
|---|---|
| Alcool en $C_{12}$ | 20% |
| $C_{13}$ | 33% |
| $C_{14}$ | 29% |
| $C_{15}$ | 18% |

a) A 7,4 g (0,02 mole) de tétramère de l'épichlorhydrine, on ajoute 16 g de méthyl éthanolamine (0,2 mole), puis on chauffe le mélange à 135°C pendant 20 heures. On neutralise l'acide formé avec 15 g de méthylate de sodium à 5 meq/g en présence de 25 ml de benzène. Le chlorure de sodium est séparé par filtration.

Après élimination du solvant par chauffage sous pression réduite, on obtient une huile brune dont l'indice de basicité est de 7,17 meq/g.

b) A 4,9 g (35,2 meq en amine) de dérivé ainsi obtenu, on ajoute 2 g d'éthanol absolu, puis en 15 minutes, 11 g d'ester méthane sulfonique d'un mélange d'alcools primaires en $C_{12}—C_{15}$ vendu sous la marque "Dobanol 25" dissous dans 5,5 g d'éthanol absolu.

On chauffe à 45°C pendant 10 heures, puis encore 10 heures à 90°C. Après élimination de l'éthanol on obtient une pâte brune soluble dans l'eau.

EXEMPLE 10

Préparation du composé tensio-actif cyclique du type I(g) de formule:

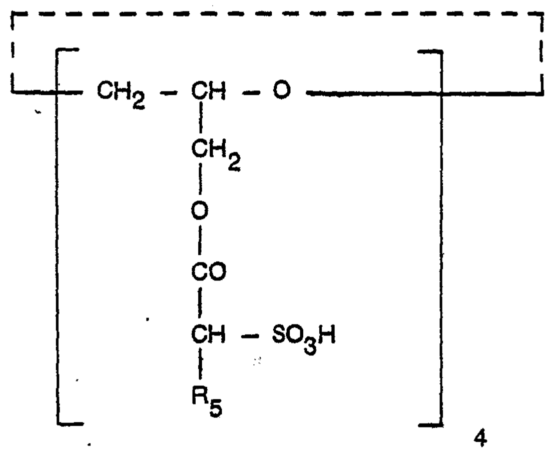

(XX)

où $R_5$ désigne le radical alkyle en $C_{14}$—$H_{29}$.

Dans un premier stade on prépare le composé intermédiaire hydroxylé de formule:

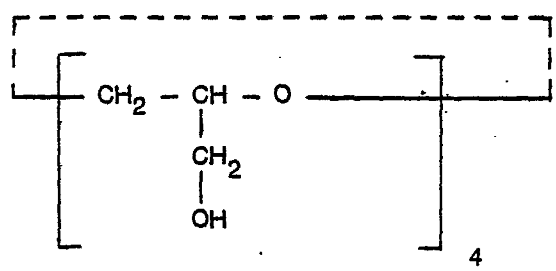

(IIA)

par "hydroxylation" (remplacement de l'atome d'halogène par un groupe hydroxyle) du tétramère de l'épichlorhydrine au moyen d'acétate de potassium et alcoolyse de l'ester acétique formé.

Sous atmosphère d'azote, on mélange:

7,4 g (0,02 mole) de tétramère de l'épichlorhydrine de formule (III)

7,85 g (0,08 mole) d'acétate de potassium pur sec

10 g de dipropylène glycol.

Le mélange est porté 5 heures à 145°C sous agitation. Le chlorure de potassium est essoré, puis lavé avec deux fois 10 ml d'éthanol absolu.

Les solvants sont éliminés par distillation sous une pression de 1 mm de Hg, en portant la température jusqu'à 130°C. Le poids obtenu est de 7,5 g. Le produit est mis en solution dans 10 ml d'éthanol absolu. On ajoute 0,1 g de méthylate de sodium à 6,2 meq/g. On laisse 24 heures à température ambiante.

La solution est neutralisée avec une solution éthanolique d'acide chlorhydrique.

L'ethanol est éliminé par distillation sous une pression de 40 mm de Hg.

Le produit est désolvaté par chauffage à l'étuve à 105°C pendant 8 heures.

On obtient le composé intermédiaire de formule IIA) ayant un indice d'hydroxyle de 13,5—14,05 meq/g.

(Théorie: 13,5 meq/g).

b) Dans un second stade on dissout 1 g de composé (IIA) et 4,6 g d'acide sulfopalmitique dans 18 g d'eau. On chauffe à 100°C et on distille l'eau à pression ordinaire puis sous pression réduite.

On reprend la masse réactionnelle avec 40 g de toluène que l'on ajoute progressivement pour entraîner l'eau formée au cours de la réaction. On obtient ainsi une pâte de couleur brun noir soluble dans l'eau.

EXEMPLE 11

Préparation d'un mélange de composés tensio-actifs cycliques du type I($a_1$) de formule:

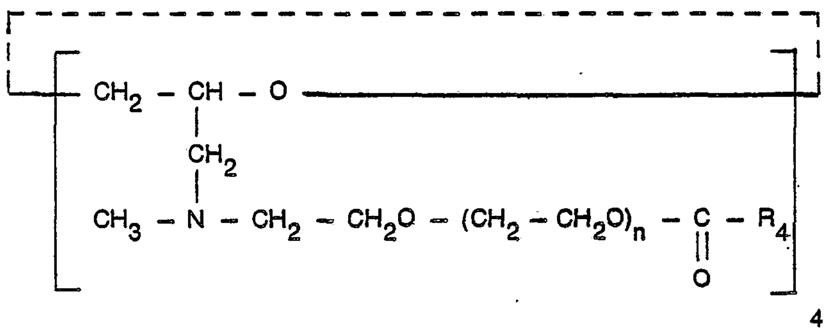

dans laquelle *n* représente une valeur statistique moyenne de 5,8 et $R_4$ désigne le radical $C_{11}H_{23}$.

a) A 20 g de composé préparé selon l'exemple 9a, on ajoute 1,2 g de solution méthanolique de méthylate de sodium à 5,85 meq/g.

Le méthanol est éliminé sous pression réduite.

On chauffe à la température de 180°C et fait passer sous agitation un courant d'oxyde d'éthylène pendant 14 heures. L'augmentation de poids correspond à une condensation statistique de 5,8 moles d'oxyde d'éthylène par groupement OH.

Le produit est dissous dans 100 ml d'isopropanol et le catalyseur neutralisé avec de l'acide chlorhydrique en solution isopropanolique. Après filtration le solvant est éliminé sous pression réduite.

On obtient une huile brune, soluble dans l'eau, dont l'indice de basicité est de 2,16 meq/g et l'indice d'hydroxyle: 2,8 meq/g.

b) 10,7 g de produit ainsi obtenu (30 meq en groupement OH) sont mélangés avec 6,45 g de laurate de méthyle (0,03 mole).

Après addition de 0,26 g de méthylate de sodium à 5,8 meq/g, on chauffe la masse réactionnelle pendant 3 heures à 100°C sous agitation.

On obtient ainsi une huile brune, soluble dans l'eau.

On peut, par addition d'un acide minéral ou organique, préparer les sels correspondants de formule $b_1$.

EXEMPLE 12

Préparation d'un composé tensio-actif cyclique du type I(a), de formule:

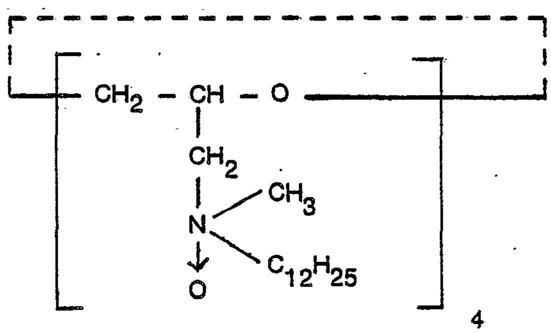

7,2 g de composé préparé selon l'exemple I (28 meq en groupement amine) sont dissous dans 7 g d'isopropanol, puis le mélange est chauffé sous agitation à 60°C.

On ajoute ensuite en 1 heure, 5,7 ml d'eau oxygénée à 130 volumes (39% en poids). Après 17 heures à 60°C, le taux de réaction est pratiquement quantitatif. Après concentration sous pression réduite, on obtient une pâte jaune clair, dispersible dans l'eau et soluble en présence d'acide.

Indice de basicité: 3,7 meq/g.

EXEMPLE 13

Préparation d'un composé tensio-actif cyclique de type I(e), de formule:

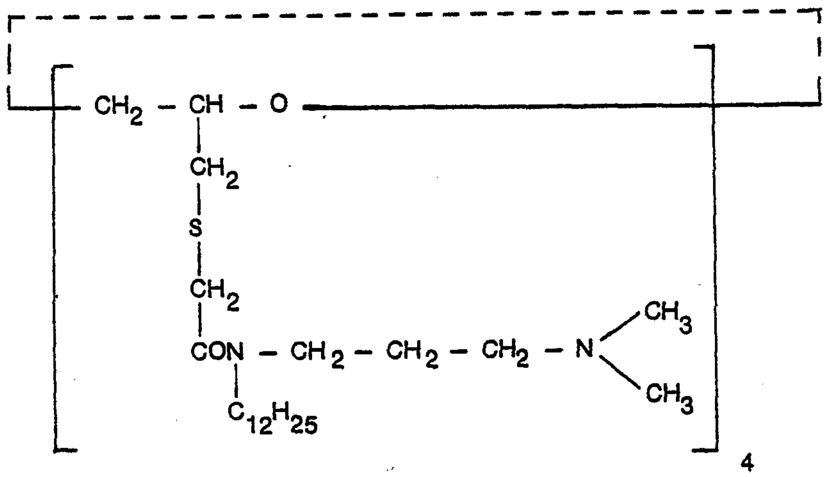

On ajoute, sous azote, à 9,25 g de tétramère de l'épichlorhydrine préparé selon l'exemple I (100 meq en chlore) dissous dans 60 g d'éthanol absolu, 12 g de thioglycolate d'éthyle (0,1 mole).

On chauffe ensuite la masse réactionnelle à 60°C et on ajoute 17,1 g de méthylate de sodium à 5,86 meq/g.

Après 16 heures de chauffage, la masse réactionnelle est diluée avec 30 ml d'acétate et le chlorure de sodium éliminé par filtration.

Le solvant est évaporé sous pression réduite.

On obtient ainsi une huile jaune dont l'indice de saponification est de 6,5 meq/g.

A 8,7 g de composé ainsi obtenu, on ajoute 13,7 g de N-dodécyl, N',N'-diméthyl propylène diamine-1,3 et 0,42 g de méthylate de sodium à 5,8 meq/g.

La masse réactionnelle est chauffée à 130°C pendant 45 heures.

On obtient ainsi une huile épaisse, brun foncé, soluble en milieu acide.

EXEMPLE 14

Préparation d'un mélange de composés tensio-actifs cycliques du type I($c_2$), de formule:

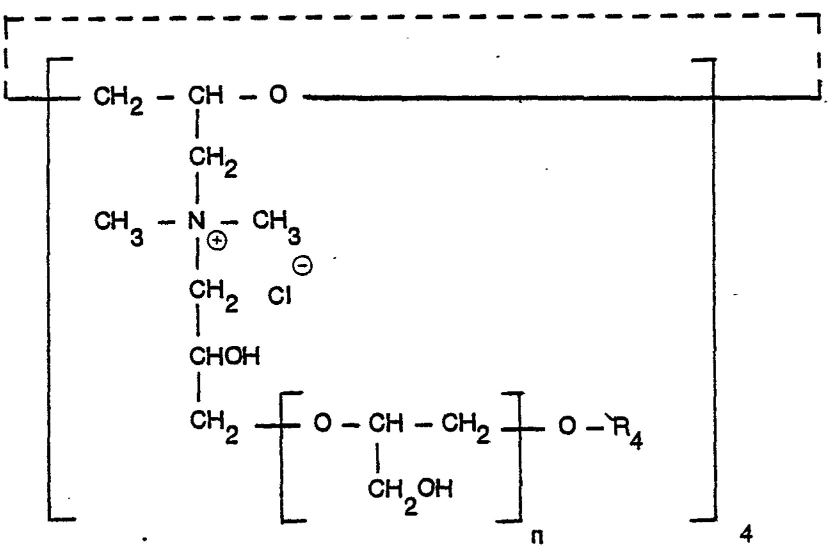

dans laquelle *n* désigne une valeur statistique moyenne de 3 et $R_4$ désigne $C_{12}H_{25}$.

A 39,5 g de composé polychloré préparés par polyaddition de 4 moles d'épichlorhydrine à 1 mole de dodécanol-1, en présence de trifluorure de bore (température de 50°C), on ajoute 40 g de diméthylamine pure, et 100 ml d'éthanol absolu.

Le mélange réactionnel est chauffé en autoclave à 80°C pendant 4 heures. Le produit est ensuite repris avec 100 ml d'acétate d'éthyle et lavé à l'eau; après séchage de la phase organique sur sulfate de sodium, on élimine le solvant sous pression réduite.

Indice de basicité: 2 meq/g.

31 g de composé ainsi obtenu sont chauffés avec un poids équivalent de dipropylène glycol et 16 g d'acétate de potassium pendant 6 heures à 180°C; après filtration des sels minéraux, le solvant est éliminé sous pression réduite.

La masse réactionnelle est ensuite reprise avec 70 ml d'éthanol absolu en présence de 0,5 g de méthylate de sodium à 5,8 meq/g.

'Après 48 heures à température ordinaire et neutralisation du catalyseur, le solvant est éliminé sous pression réduite.

On adjoute à 16 g de produit ainsi obtenu (2,48 meq/g) 3,7 g de tétramère de l'épichlorhydrine préparé selon l'exemple I (40 meq en chlore).

Le mélange est ensuite chaffé à 115°C pendant 70 heures.

On obtient ainsi une pâte brune très épaisse, soluble dans l'eau.

EXEMPLE 15

On prépare un shampooing de composition suivante:

| | |
|---|---|
| — Composé de l'exemple 3 | 0,5 g |
| — $R—CHOH—CH_2—O—[CH_2—CHOH—CH_2O]_{3,5}—H$ | 7 g |
| R désigne un mélange de radicaux alkyle ayant de 9 à 12 atomes de carbone | |
| — Acide lactique q.s.p. pH 3,5 | |
| — Eau q.s.p. | 100 g |

EXEMPLE 16

On prépare le shampooing de composition suivante:

| | |
|---|---|
| — Composé de l'exemple 11 | 0,7 g |
| — Laurylsulfate de triéthanolamine | 8 g |
| — Diéthanolamides d'acides gras de coprah | 3 g |
| — Acide lactique q.s.p. pH 6,5 | |
| — Eau q.s.p. | 100 g |

EXEMPLE 17

On prépare le shampooing de composition suivante:

| | |
|---|---|
| — Composé de l'exemple 8 | 1 g |
| — $R—CHOH—CH_2—O—[CH_2—CHOH—CH_2O]_{3,5}—H$ | 10 g |
| R désigne un mélange de radicaux alkyle ayant de 9 à 12 atomes de carbone | |
| — Diéthanolamides d'acides gras de coprah | 4 g |
| — Parfum | 0,03 g |
| — Acide lactique q.s.p. pH 6 | |
| — Eau q.s.p. | 100 g |

EXEMPLE 18

On prépare le shampooing de composition suivante:

| | |
|---|---|
| — Composé de l'exemple 14 | 0,6 g |
| — Laurylsulfate de triéthanolamine | 6 g |
| — Diéthanolamide laurique | 3 g |
| — Eau q.s.p. | 100 g |

Les shampooings des exemples 15 à 18 assurent un bon nettoyage des cheveux. Les cheveux lavés sont doux et se coiffent bien.

EXEMPLE 19

On prépare la composition tinctoriale suivante:

| | |
|---|---|
| — Composé de l'exemple 8 | 1 g |
| — N-β-amino éthyl N-méthyl amino-4 phényl azo-nitro-4'-phényl | 0,5 g |
| — Eau q.s.p. | 100 g |

Cette composition est appliquée pendant 30 minutes sur des cheveux décolorés. Après rinçage et shampooing on obtient une nuance abricot.

EXEMPLE 20

On prépare la composition tinctoriale suivante:

| | |
|---|---|
| — Composé de l'exemple 8 | 1 g |
| — N-(amino-3-propyl) amino-1 anthraquinone | 0,05 g |
| — Eau q.s.p. | 100 g |

Cette composition est appliquée pendant 30 minutes sur des cheveux décolorés. Après rinçage et shampooing on obtient une nuance orange clair.

EXEMPLE 21

On prépare la composition tinctoriale suivante:

| | |
|---|---|
| — Composé de l'exemple 8 | 1 g |
| — Diméthylamino-1,4 nitro-2 benzène | 0,03 g |
| — Eau q.s.p. | 100 g |

Cette composition est appliquée pendant 30 minutes sur des cheveux décolorés. Après rinçage et shampooing on obtient une nuance tamaris nacré.

**Revendications**

1. Composition cosmétique ou pharmaceutique, caractérisée par le fait qu'elle renferme, en présence d'un véhicule approprié, au moins un composé tensio-actif de formule générale:

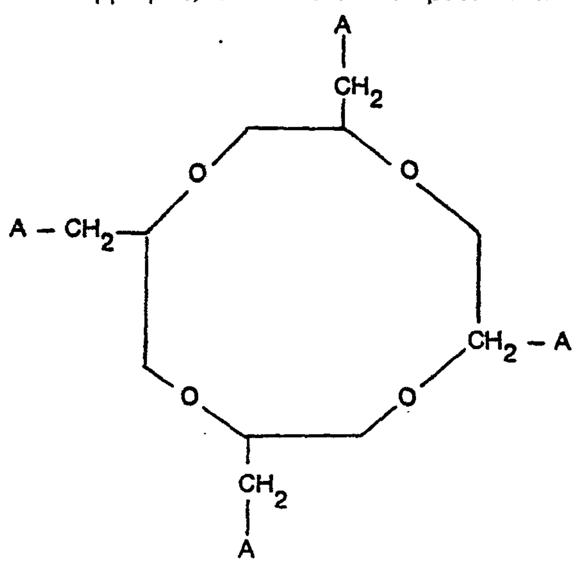

(I)

dans laquelle A désigne un ensemble amphiphile constitué d'une partie hydrophile et d'une partie lipophile, cet ensemble amphiphile étant relié par la partie hydrophile au cycle, par liaison covalente; la partie hydrophile comporte un ou plusieurs groupements amine, oxyde d'amine, ammonium, ammonio alcanoate, ammonio alcoyl sulfonate, ammonio alcoyl sulfinate, thioéther, sulfoxyde, sulfonium, sulfoxonium, éther, hydroxyle, ester, amide, acide; la partie lipophile est constituée d'un ou plusieurs groupements choisis parmi (i) les groupements aliphatiques ayant de 1 à 20 atomes de carbone, (ii) les groupements aliphatiques substituées ayant de 6 à 20 atomes de carbone, (iii) les groupements acoylaryle ayant de 6 à 20 atomes de carbone, la partie alcoyle du groupement alcoylaryle ayant jusqu'à 14 atomes de carbone.

2. Composition selon la revendication 1, caractérisée par le fait que le groupement amphiphile A est choisi parmi les suivants:

(a) le groupement:

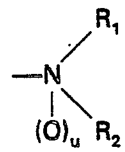

où $R_1$ et $R_2$, identiques ou différents, désignent des radicaux aliphatiques ayant de 1 à 20 atomes de carbone, l'une des radicaux comportant au moins 8 atomes de carbone, ou des radicaux aliphatiques substitués ayant de 6 à 20 atomes de carbone, ou des radicaux alcoylaryle ayant de 6 à 20 atomes de carbone; la somme des atomes de carbone de $R_1$ et $R_2$ étant, de préférence, inférieur ou égale à 28; l'un des radicaux peut, en outre, désigner un radical diméthylaminoéthyle ou propyle, diéthylaminoéthyle ou propyle, pipéridino éthyle ou propyle, morpholino éthyle ou propyle ou alcoyl polyhydroxy propylène;

*u* désigne le nombre zéro ou 1;

$a_1$) le groupement:

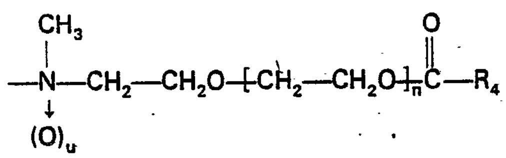

où $R_4$ désigne un radical aliphatique, non substitué ou substitué ou un radical alcoylaryle ayant de 6 à 20 atomes de carbone et de préférence un radical alcoyle, hydroxyalcoyle, alcényle ou alcoyl phényle, la partie alcoyle du radical alcoyl phényle ayant jusqu'à 14 atomes de carbone;

*n* désigne un nombre quelconque de 1 à 20;

*u* désigne le nombre 0 ou 1;

$a_2$) le groupement:

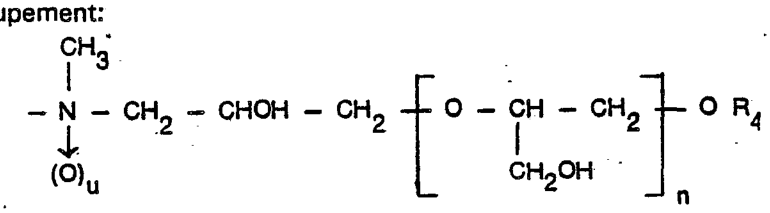

*u*, $R_4$ et *n* ayant la signification ci-dessus indiquée;

b) le groupement:

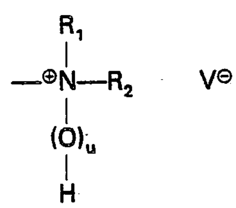

$b_1$) le groupement:

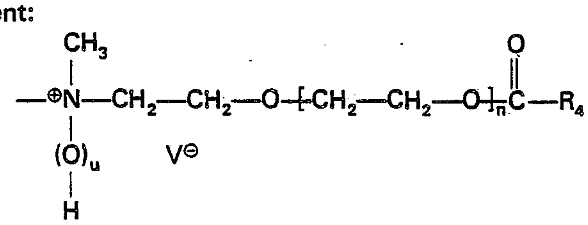

$b_2$) le groupement:

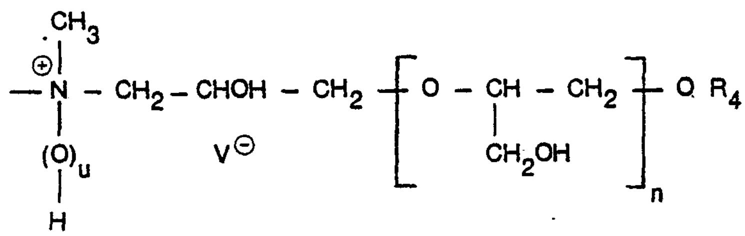

Dans les groupements (b), ($b_1$) et ($b_2$) $V^{\ominus}$ désigne un anion et de préférence un anion formiate, acétate, citrate ou lactate;
*u, n,* $R_1$, $R_2$ et $R_4$ ont les significations indiquées pour les groupements a et $a_1$;

c) le groupement:

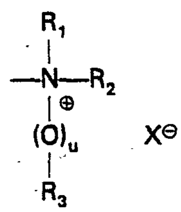

$R_1$, $R_2$ et *u* ayant la signification indiquée pour le groupement ($a_1$);
$R_3$ désigne un radical alcoyle ou hydroxyalcoyle ayant de 1 à 3 atomes de carbone et de préférence un radical méthyle, éthyle, hydroxyéthyle, dihydroxypropyle ou un radical benzyle;
$X^{\ominus}$ désigne un anion et de préférence un chlorure, bromure, iodure, monométhyl sulfate, méthyl sulfonate, p.toluène sulfonate;

$c_1$) le groupement:

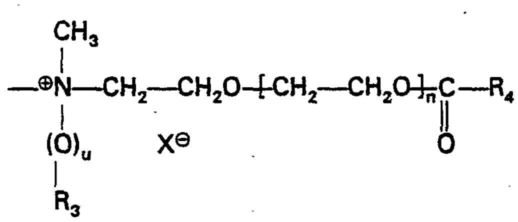

$c_2$) le groupement:

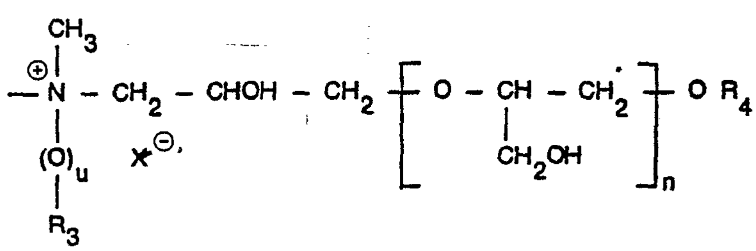

Dans ces groupements ($c_1$) et ($c_2$)
*u* désigne le nombre 0 ou 1
*n* désigne un nombre quelconque de 1 à 20
$R_4$ a les significations indiquées pour le groupement ($a_1$)
$R_3$ et $X^{\ominus}$ ont les significations indiquées pour le groupement (c);

d) le groupement:

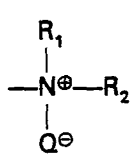

où $Q^{\ominus}$ désigne l'un des groupements suivants:

$-(CH_2)_m-COO^{\ominus}$ où *m* désigne 1, 2 ou 3

$-CH_2-CH_2-CH_2-SO_3^{\ominus}$

$-CH_2-CH_2-O-SO_2^{\ominus}$

$R_1$ et $R_2$ ont la signification indiquée pour le groupement (a);

$d_1$) le groupement:

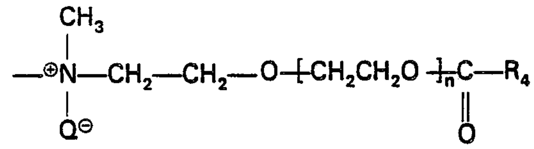

$d_2$) le groupement:

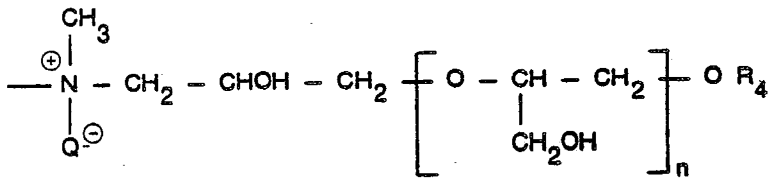

Dans les groupements ($d_1$) et ($d_2$) *n* désigne un nombre quelconque de 1 à 20, $Q^{\ominus}$ a la signification indiquée pour le groupement (d); $R_4$ a la signification indiquée pour le groupement ($a_1$);

e) le groupement:

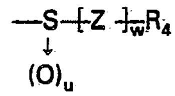

où $R_4$ a la signification indiquée pour le groupement $a_1$;
*u* désigne zéro ou 1; *w* désigne zéro ou 1;
Z désigne des groupements d'atomes à caractère hydrophile qui peuvent être éther, ester, amide, amine, ammonium et/ou hydroxyle;

$e_1$) le groupement:

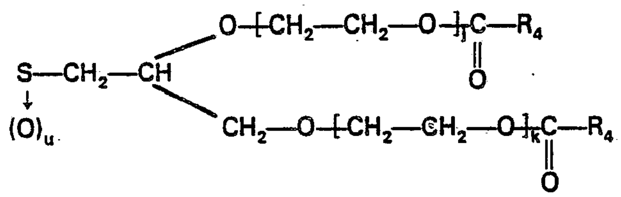

j et k désignent chacun un nombre quelconque inférieur à 20, la somme j + k étant comprise entre 1 et 20, *u* désigne 0 ou 1; $R_4$ a la signification indiquée pour le groupement $a_1$;

f) le groupement:

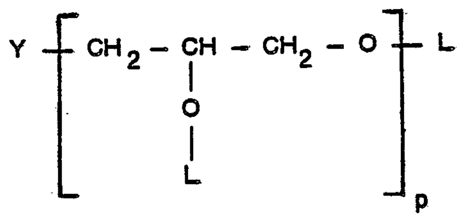

où L désigne en même temps un atome d'hydrogène et l'un des groupements:

$—CH_2—CHOH—CH_2—(O)_q—R_5$ où q désigne 0 ou 1

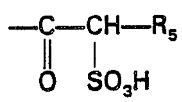 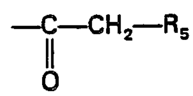

l'atome d'hydrogène et l'autre groupement étant répartis de façon statistique;
$R_5$ désigne un radical alcoyle linéaire ayant de 6 à 18 atomes de carbone;
p désigne un nombre quelconque de 1 à 10 et représente une valeur statistique moyenne
Y désigne: —O—; —S— ou 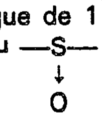

g) le groupement:

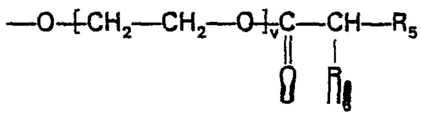

où v désigne un nombre quelconque de 0 à 20

$R_6$ désigne un atome d'hydrogène ou le radical $-SO_3H$; $R_5$ a la signification indiquée ci-dessus;

h) le groupement (e) ou ($e_1$) alcoylé par un agent d'alcoylation choisi dans le groupe formé par le bromure de méthyle, l'iodure de méthyle et le sulfate de diméthyle et de préférence par le sulfate de diméthyle;

i) le groupement (f) alcoylé par un agent d'alcoylation choisi dans le groupe formé par le bromure de méthyle, l'iodure de méthyle et le sulfate de diméthyle.

3. Composition selon les revendications 1 et 2, caractérisée par le fait que le véhicule est aqueux ou hydroalcoolique.

4. Composition cosmétique pour le traitement des cheveux contenant en solution dans un solvant choisi dans un groupe formé par l'eau et une solution hydroalcoolique, une quantité cosmétiquement efficace de un ou plusieurs composés de formule (I), selon la revendication 1 ou 2.

5. Composition de shampooing pour le traitement des cheveux contenant en solution dans un solvant choisi dans un groupe formé par l'eau et une solution hydroalcoolique, une quantité cosmétiquement efficace d'un ou plusieurs composés de formule (I), selon la revendication 1 ou 2.

6. Composition cosmétique pour le traitement des cheveux selon la revendication 3 contenant en solution dans un solvant choisi dans un groupe formé par l'eau et une solution hydroalcoolique, une quantité cosmétiquement efficace d'un ou plusieurs composés de formule (I) et contenant également un ou plusieurs adjuvants choisis dans le groupe formé par les tensio-actifs anioniques, cationiques, amphotères, zwitterioniques, non-ioniques, les parfums, les colorants, les conservateurs, les épaississants, les agents moussants, les stabilisateurs de mousse, les agents adoucissants, les agents de restructuration des cheveux, les agents antipelliculaires, les résines cosmétiques, les synergistes de mousse, les électrolytes.

7. Procédé de traitement des cheveux consistant à appliquer sur les cheveux humains une quantité efficace d'une composition comprenant en solution dans un solvant choisi dans un groupe formé par l'eau et une solution hydroalcoolique, une quantité cosmétiquement efficace d'un ou plusieurs polyéthers cycliques tensio-actifs de formule (I), selon la revendication 1 ou 2.

8. Composition tinctoriale pour cheveux contenant en solution dans un solvant choisi dans un groupe formé par l'eau et une solution hydroalcoolique une quantité cosmétiquement efficace d'un ou plusieurs composés de formule (I), selon la revendication 1 ou 2, ainsi qu'un colorant pour cheveux et éventuellement un ou plusieurs adjuvants choisis dans le groupe formé par les tensio-actifs anioniques, cationiques, non-ioniques, zwitterioniques, amphotères et leurs mélanges, les parfums, les conservateurs, les épaississants, les adoucissants, les résines cosmétiques, les séquestrants.

9. Composition selon la revendication 8, caractérisée par le fait que le colorant est choisi parmi les colorants anthraquinoniques, les colorants azoïques, les colorants nitrés de la série benzénique, les indophénols, les indoanilines, les indamines.

10. Polyéthers cycliques tensio-actifs de formule générale:

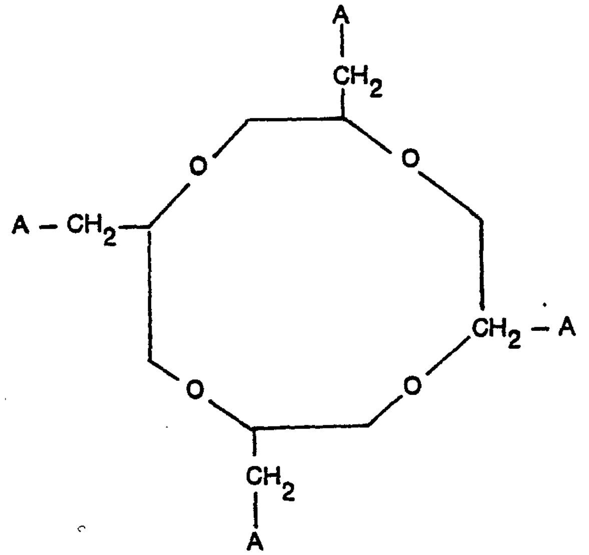

(I)

dans laquelle A désigne un ensemble amphiphile constitué d'une partie hydrophile et d'une partie lipophile, cet ensemble amphiphile étant relié par la partie hydrophile au cycle par liaison covalente; la

partie amphiphile comporte un ou plusieurs groupements choisis parmi les suivants:

a) le groupement:

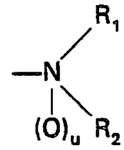

où $R_1$ et $R_2$, identiques ou différents, désignent des radicaux aliphatiques ayant de 1 à 20 atomes de carbone, l'une des radicaux comportant au moins 8 atomes de carbone, ou des radicaux aliphatiques substitués ayant de 6 à 20 atomes de carbone, ou des radicaux alcoylaryle ayant de 6 à 20 atomes de carbone; la somme des atomes de carbone de $R_1$ et $R_2$ étant, de préférence, inférieure ou égale à 28; l'un des radicaux peut, en outre, désigner un radical diméthylaminoéthyle ou propyle, diéthylaminoéthyle ou propyle, pipéridino éthyle ou propyle, morpholino éthyle ou propyle ou alcoyl polyhydroxy propylène;

*u* désigne le nombre zéro ou 1;

$a_1$) le groupement:

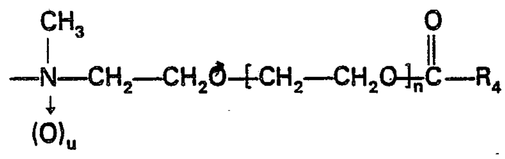

où $R_4$ désigne un radical aliphatique, non substitué ou substitué ou un radical alcoylaryle ayant de 6 à 20 atomes de carbone et de préférence un radical alcoyle, hydroxyalcoyle, alcényle ou alcoyl phényle, la partie alcoyle du radical alcoyl phényle ayant jusqu'à 14 atomes de carbone;

*n* désigne un nombre quelconque de 1 à 20;

*u* désigne le nombre 0 ou 1;

$a_2$) le groupement:

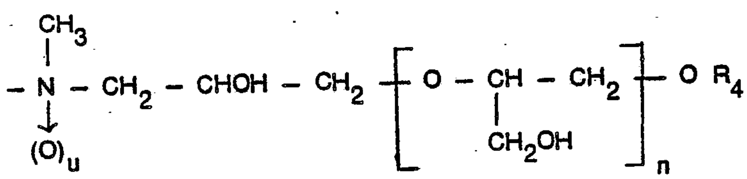

*u*, $R_4$ et *n* ayant la signification ci-dessus indiquée;

b) le groupement:

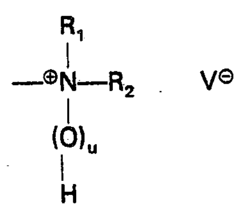

$b_1$) le groupement:

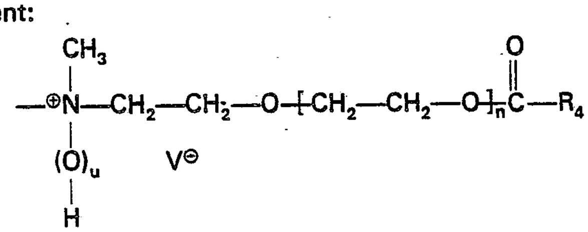

$b_2$) le groupement:

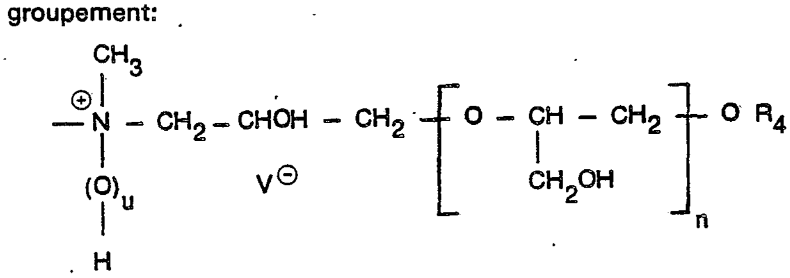

Dans les groupements (b), ($b_1$) et ($b_2$) $V^{\ominus}$ désigne un anion et de préférence un anion formiate, acétate, citrate ou lactate;
*u, n,* $R_1$, $R_2$ et $R_4$ ont les significations indiquées pour les groupements a et $a_1$;

c) le groupement:

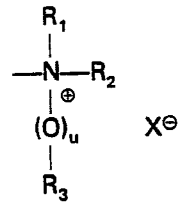

$R_1$, $R_2$ et *u* ayant la signification indiquée pour le groupement ($a_1$);
$R_3$ désigne un radical alcoyle ou hydroxyalcoyle ayant de 1 à 3 atomes de carbone et de préférence un radical méthyle, éthyle, hydroxyéthyle, dihydroxypropyle ou un radical benzyle;
$X^{\ominus}$ désigne un anion et de préférence un chlorure, bromure, iodure, monométhyl sulfate, méthyl sulfonate, p.toluène sulfonate;

$c_1$) le groupement:

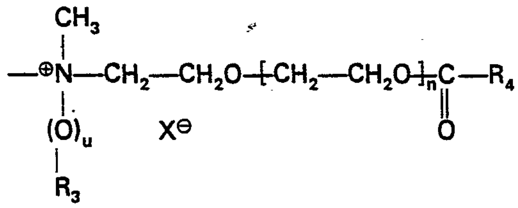

$c_2$) le groupement:

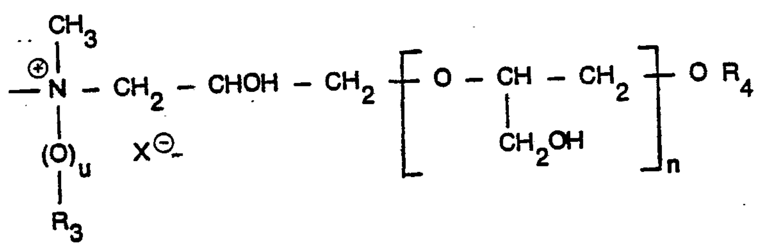

Dans ces groupements ($c_1$) et ($c_2$)
*u* désigne le nombre 0 ou 1
*n* désigne un nombre quelconque de 1 à 20
$R_4$ a les significations indiquées pour le groupement ($a_1$)
$R_3$ et $X^{\ominus}$ ont les significations indiquées pour le groupement (c);

d) le groupement:

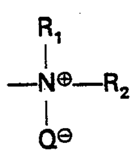

où $Q^{\ominus}$ désigne l'un des groupements suivants:

$-(CH_2)_m-COO^{\ominus}$ où *m* désigne 1, 2 ou 3

$-CH_2-CH_2-CH_2-SO_3^{\ominus}$

$-CH_2-CH_2-O-SO_2^{\ominus}$

$R_1$ et $R_2$ ont la signification indiquée pour le groupement (a);

$d_1$) le groupement:

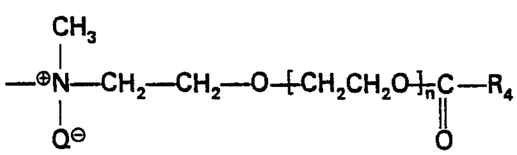

$d_2$) le groupement:

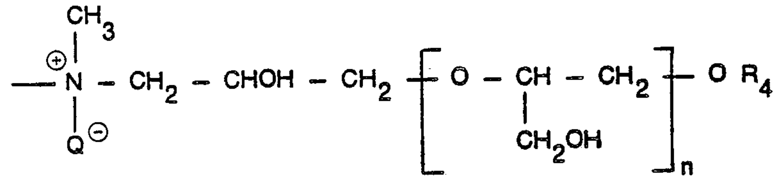

Dans les groupements ($d_1$) et ($d_2$) *n* désigne un nombre quelconque de 1 à 20, $Q^{\ominus}$ a la signification indiquée pour le groupement (d); $R_4$ a la signification indiquée pour le groupement ($a_1$);

e) le groupement:

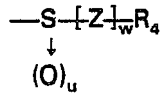

où $R_4$ a la signification indiquée pour le groupement ($a_1$);
*u* désigne zéro ou 1; *w* désigne zéro ou 1;
Z désigne des groupements d'atomes à caractère hydrophile qui peuvent être éther, ester, amide, amine, ammonium et/ou hydroxyle;

$e_1$) le groupement:

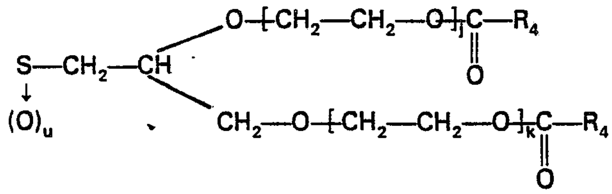

j et k désignent chacun un nombre quelconque inférieur à 20, la somme j + k étant comprise entre 1 et 20, *u* désigne 0 ou 1; $R_4$ a la signification indiquée pour le groupement $a_1$;

f) le groupement:

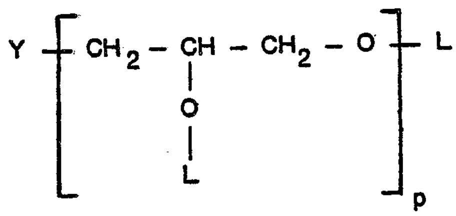

où L désigne en même temps un atome d'hydrogène et l'un des groupements:

$—CH_2—CHOH—CH_2—(O)_q—R_5$ où q désigne 0 ou 1

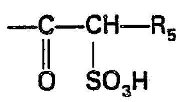

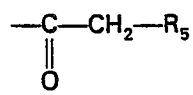

l'atome d'hydrogène et l'autre groupement étant répartis de façon statistique;
$R_5$ désigne un radical alcoyle linéaire ayant de 6 à 18 atomes de carbone;
p désigne un nombre quelconque de 1 à 10 et représente une valeur statistique moyenne
Y désigne: —O—; —S— ou 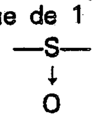

g) le groupement:

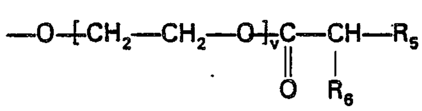

où v désigne un nombre quelconque de 0 à 20
$R_6$ désigne un atome d'hydrogène ou le radical $—SO_3H$; $R_5$ a la signification indiquée ci-dessus;

h) le groupement (e) ou ($e_1$) alcoylé par un agent d'alcoylation choisi dans le groupe formé par le bromure de méthyle, l'iodure de méthyle et le sulfate de diméthyle et de préférence par le sulfate de diméthyle;

i) le groupement (f) alcoylé par un agent d'alcoylation choisi dans le groupe formé par le bromure de méthyle, l'iodure de méthyle et le sulfate de diméthyle.

11. Polyéther cyclique tensio-actif selon la revendication 10, caractérisé par le fait que le groupement amphiphile A est choisi parmi les suivants:

a) le groupement:

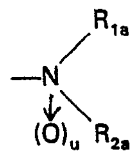

où $R_{1a}$ et $R_{2a}$, identiques ou différents, désignent des radicaux alcoyle, hydroxyalcoyle ou alcényle ayant de 1 à 20 atomes de carbone, l'un des radicaux comportant au moins 8 atomes de carbone, ou des radicaux alcoylaryle ayant de 6 à 20 atomes de carbone, la somme des atomes de carbone de $R_{1a}$ et $R_{2a}$ étant, de préférence, inférieure ou égale à 28;
l'un des radicaux peut, en outre, désigner un radical diméthylaminoéthyle ou propyle, diéthylaminoéthyle ou propyle, pipéridino éthyle ou propyle, morpholino éthyle ou propyle ou alcoyl polyhydroxy propylène;
*u* désigne le nombre zéro ou 1;

$a_1$) le groupement:

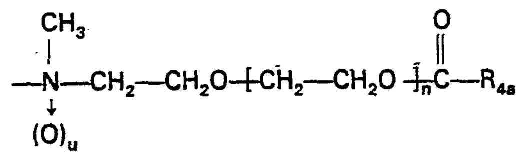

où $R_{4a}$ désigne un radical alcoyle, hydroxyalcoyle, alcényle ou alcoyl phényle ayant de 6 à 20 atomes de carbone, la partie alcoyle du radical alcoyl phényle ayant jusqu'à 14 atomes de carbone;
*n* désigne un nombre quelconque de 1 à 20;
*u* désigne le nombre 0 ou 1;

$a_2$) le groupement:

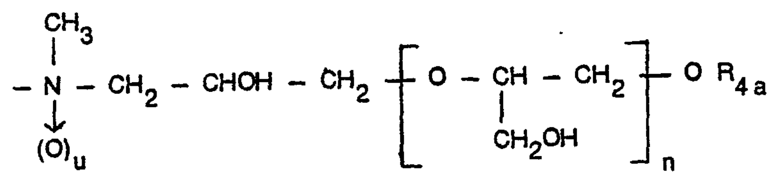

*u*, $R_{4a}$ et *n* ayant la signification ci-dessus indiquée;

b) le groupement:

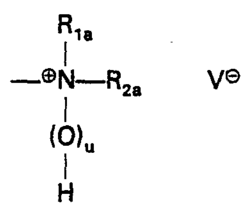

$b_1$) le groupement:

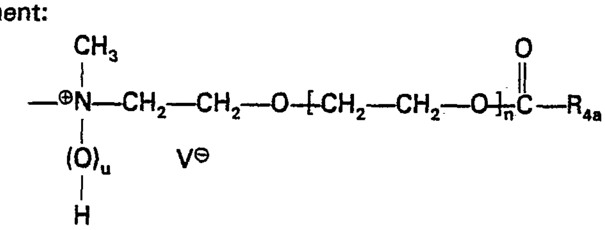

$b_2$) le groupement:

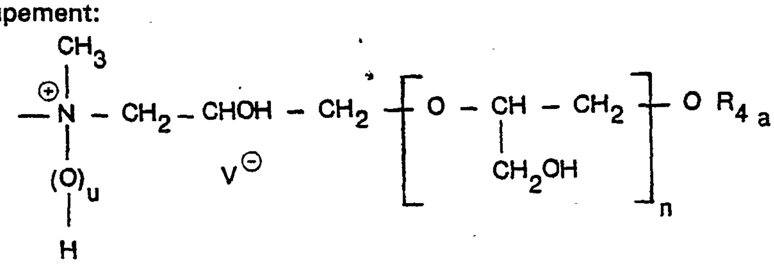

Dans le groupements (b), ($b_1$) et ($b_2$) $V^{\ominus}$ désigne un anion et de préférence un anion formiate, acétate, citrate ou lactate;

*u, n,* $R_{1a}$, $R_{2a}$ et $R_{4a}$ ont les significations indiquées pour les groupements a et $a_1$;

c) le groupement:

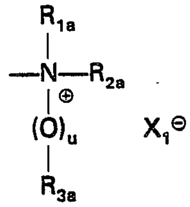

$R_{1a}$, $R_{2a}$ et *u* ayant la signification indiquée pour le groupement (a);

$R_{3a}$ désigne un radical méthyle, éthyle, hydroxyéthyle, dihydroxypropyle ou un radical benzyle;

$X_1^{\ominus}$ désigne un anion chlorure, bromure, iodure, monométhyl sulfate, méthyl sulfonate, p.toluène sulfonate;

$c_1$) le groupement:

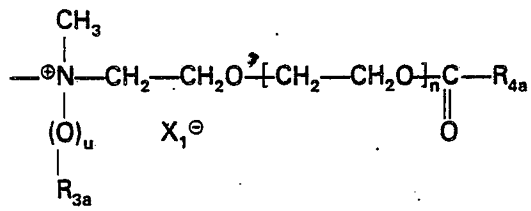

$c_2$) le groupement:

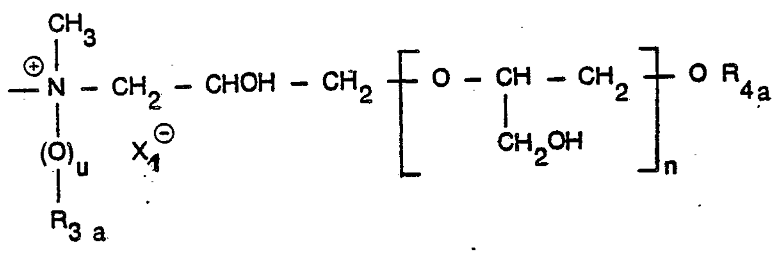

Dans ces groupements ($c_1$) et ($c_2$)

*u* désigne le nombre 0 ou 1

*n* désigne un nombre quelconque de 1 à 20

$R_{4a}$ a les significations indiquées pour le groupement ($a_1$)

$R_3$ et $X_1^{\ominus}$ ont les significations indiquées pour le groupement (c);

d) le groupement:

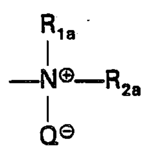

où $Q^{\ominus}$ désigne l'un des groupements suivants:

$—(CH_2)_m COO^{\ominus}$ où *m* désigne 1, 2 ou 3

$—CH_2—CH_2—CH_2—SO_3^{\ominus}$

$—CH_2—CH_2—O—SO_3^{\ominus}$

$R_{1a}$ et $R_{2a}$ ont la signification indiquée pour le groupement (a);

$d_1$) le groupement:

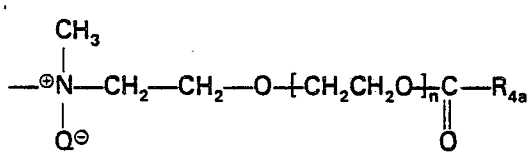

$d_2$) le groupement:

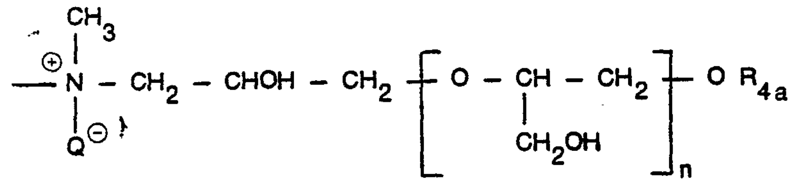

Dans les groupements ($d_1$) et ($d_2$) *n* désigne un nombre quelconque de 1 à 20, $R_{4a}$ a les significations indiquées pour le groupement ($a_1$); $Q^{\ominus}$ a la signification indiquée pour le groupement (d);

e) le groupement:

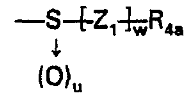

où $R_{4a}$ a les significations indiquées pour le groupement ($a_1$);
*u* désigne zéro ou 1; *w* désigne zéro ou 1;
$Z_1$ désigne un groupement d'atomes à caractère hydrophile choisi dans le groupe formé par:

$—CH_2—CHOH—$; $—(CH_2—CH_2O)_n—$; 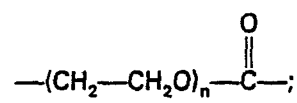 $—(CH_2)_x—COO—$;

$—(CH_2)_x—COOCH_2—CHOH—$; $—(CH_2)_x—CONH—$; 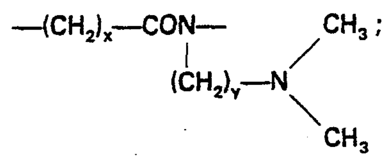

où x représente 1 ou 2, y représente 2 ou 3; *n* représente un nombre quelconque de 1 à 20.

$e_1$) le groupement:

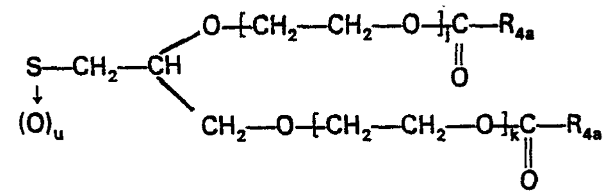

j et k désignent chacun un nombre quelconque inférieur à 20, la somme j + k étant comprise entre 1 et 20, *u* désigne 0 ou 1; $R_{4a}$ a la signification indiquée pour le groupement $a_1$;

f) le groupement:

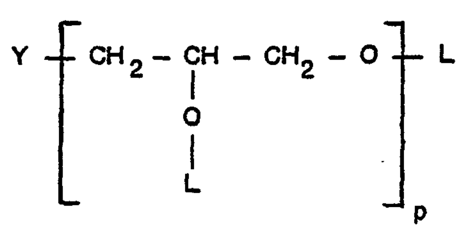

L désigne en même temps un atome d'hydrogène et l'un des groupements:

$—CH_2—CHOH—CH_2—(O)_q—R_5$ où q désigne 0 ou 1

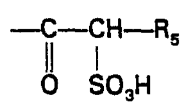 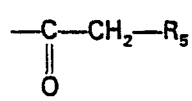

l'atome d'hydrogène et l'autre groupement étant répartis de façon statistique;
$R_5$ désigne un radical alcoyle linéaire ayant de 6 à 18 atomes de carbone;

p désigne un nombre quelconque de 1 à 10 et représente une valeur statistique moyenne

Y désigne: —O—; —S— ou 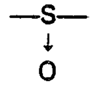

g) le groupement:

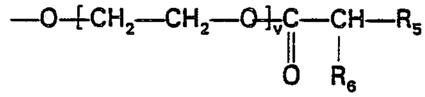

où v désigne un nombre quelconque de 0 à 20

$R_6$ désigne un atome d'hydrogène ou le radical $—SO_3H$;

$R_5$ a la signification indiquée ci-dessus;

h) le groupement (e) ou ($e_1$) alcoylé par un agent d'alcoylation choisi dans le groupe formé par le bromure de méthyle, l'iodure de méthyle et le sulfate de diméthyle et de préférence par le sulfate de diméthyle;

i) le groupement (f) alcoylé par un agent d'alcoylation choisi dans le groupe formé par le bromure de méthyle, l'iodure de méthyle et le sulfate de diméthyle.

12. Polyéther cyclique tensio-actif selon la revendication 11, caractérisé par le fait que le groupement amphiphile A est le groupement (a)

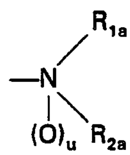

où $R_{1a}$ et $R_{2a}$, identiques ou différents, désignent des radicaux alcoyle ou hydroxyalcoyle ayant de 1 à 20 atomes de carbone, l'un des radicaux comportant au moins 8 atomes de carbone, la somme des atomes de carbone de $R_{1a}$ et $R_{2a}$ étant, de préférence, inférieure ou égale à 28; l'un des radicaux peut, en outre, désigner un radical diméthylaminoéthyle ou propyle, diéthylaminoéthyle ou propyle, pipéridino éthyle ou propyle, morpholino éthyle ou propyle ou alcoyl polyhydroxy propylène;

*u* désigne le nombre zéro ou un.

13. Polyéther cyclique tensio-actif selon la revendication 11, caractérisé par le fait que le groupement amphiphile A est le groupement ($a_1$)

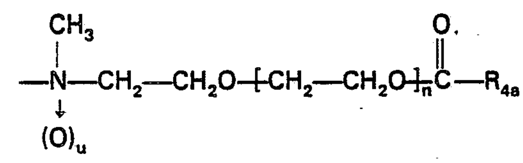

où $R_{4a}$ désigne un radical alcoyle, hydroxyalcoyle, alcényle ou alcoyl phényle ayant de 6 à 20 atomes de carbone, la partie alcoyle du radical alcoyl phényle ayant jusqu'à 14 atomes de carbone;

*n* désigne un nombre quelconque de 1 à 20; *u* désigne le nombre 0 ou 1.

14. Polyéther cyclique tensio-actif selon la revendication 11, caractérisé par le fait que le groupement amphiphile A est le groupement (b)

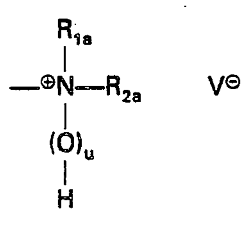

où $R_{1a}$ et $R_{2a}$, identiques ou différents, désignent des radicaux alcoyle ou hydroxyalcoyle ayant de 1 à 20 atomes de carbone, l'un des radicaux comportant au moins 8 atomes de carbone, la somme des atomes de carbone de $R_{1a}$ et $R_{2a}$ étant, inférieure ou égale à 28, l'un des radicaux $R_{1a}$ ou $R_{2a}$ peut, en outre, désigner un radical choisi dans le groupe formé par les radicaux diméthylaminoéthyle, diméthylamino propyle, diéthylamino éthyle, diéthylamino propyle, pipéridino éthyle, pipéridino propyle, morpholino éthyle, morpholino propyle et alcoyl polyhydroxy propylène; *u* désigne le nombre zéro ou 1; V désigne un anion choisi dans le groupe formé par les anions formiate, acétate, citrate et lactate.

15. Polyéther cyclique tensio-actif selon la revendication 11, caractérisé par le fait que le groupement amphiphile A est le groupement (c)

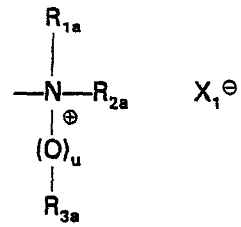

où $R_{1a}$ et $R_{2a}$, identiques ou différents, désignent des radicaux alcoyle ou hydroxyalcoyle ayant de 1 à 20 atomes de carbone, l'un des radicaux comportant au moins 8 atomes de carbone, la somme des atomes de carbone de $R_{1a}$ et $R_{2a}$ étant inférieure ou égale à 28, l'un des radicaux peut, en outre, désigner un radical choisi dans le groupe formé par diméthylaminoéthyle, diméthylamino propyle, diéthylamino éthyle, diéthylamino propyle, pipéridino éthyle, pipéridino propyle, morpholino éthyle, morpholino propyle et alcoyl polyhydroxy propylène;
$R_{3a}$ désigne un radical méthyle, éthyle, hydroxyéthyle, dihydroxypropyle ou un radical benzyle; $X_1^{\ominus}$ désigne un anion choisi dans le groupe formé par chlorure, bromure, iodure, monométhyl sulfate, méthyl sulfonate, p.toluène sulfonate; *u* désigne le nombre 0 ou 1.

16. Polyéther cyclique tensio-actif selon la revendication 11, caractérisé par le fait que le groupement amphiphile A est le groupement ($c_2$)

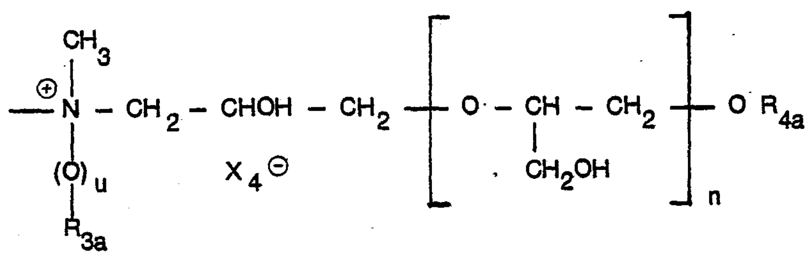

où *u* désigne le nombre 0 ou 1;
*n* désigne un nombre quelconque de 1 à 20
$R_{3a}$ désigne un radical méthyle, éthyle, hydroxyéthyle, dihydroxypropyle ou un radical benzyle;
$R_{4a}$ désigne un radical alcoyle, hydroxyalcoyle, alcényle ou alcoyl phényle ayant de 6 à 20 atomes de carbone, la partie alcoyle du radical alcoyl phényle ayant jusqu'à 14 atomes de carbone.

17. Polyéther cyclique tensio-actif selon la revendication 11, caractérisé par le fait que le groupement amphiphile A est le groupement (e)

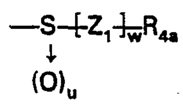

où $R_{4a}$ désigne un radical alcoyle hydroxyalcoyle, alcényle ou alcoyl phényle, ayant de 6 à 20 atomes de carbone, la partie alcoyle du radical alcoyl phényle ayant jusqu'à 14 atomes de carbone;
*u* désigne 0 ou 1; w désigne 0 ou 1;
$Z_1$ désigne un groupement choisi dans le groupe formé par

$-CH_2-CHOH-$; $-(CH_2-CH_2-O)_n-$; 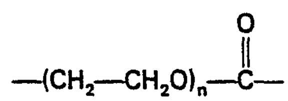 ;

$-(CH_2)_x-COO-$; $-(CH_2)_x-COOCH_2-CHOH-$;

$-(CH_2)_x-CONH-$; 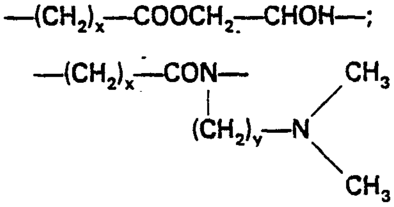 ;

où *x* représente 1 ou 2; y représente 2 ou 3; *n* représente un nombre quelconque de 1 à 20.

18. Polyéther cyclique tensio-actif selon la revendication 11, caractérisé par le fait que le groupement amphiphile A est le groupement (f)

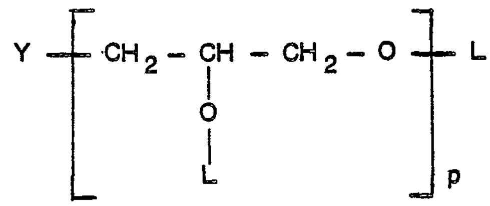

où L désigne en même temps un atome d'hydrogène et l'un des groupements:

$—CH_2—CHOH—CH_2—(O)_q—R_5$ où q désigne 0 ou 1

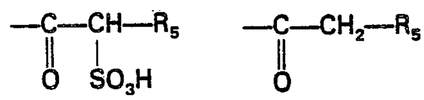

l'atome d'hydrogène et l'autre groupement étant répartis de façon statistique;
$R_5$ désigne un radical alcoyle linéaire ayant de 6 à 18 atomes de carbone;
p désigne un nombre quelconque de 1 à 10 et représente une valeur statistique moyenne;
Y désigne: —O—; —S— ou

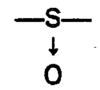

ce groupement pouvant être alcoylé par un agent d'alcoylation choisi de préférence dans le groupe formé par le bromure de méthyle, l'iodure de méthyle et le sulfate de diméthyle.

19. Polyéther cyclique tensio-actif selon la revendication 11, caractérisé par le fait que le groupement amphiphile A est le groupement (g)

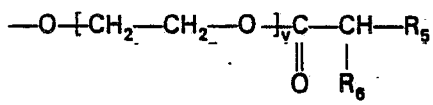

où *v* désigne un nombre quelconque de 0 à 20;
$R_5$ désigne un radical alcoyle linéaire ayant de 6 à 18 atomes de carbone.
$R_6$ désigne un atome d'hydrogène ou le radical $—SO_3H$.

20. Procédé de préparation d'un polyéther cyclique tensio-actif de formule (I) dans laquelle A désigne:

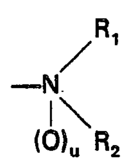 (a)

où $R_1$ et $R_2$, identiques ou différents, désignent des radicaux aliphatiques ayant de 1 à 20 atomes de carbone, l'un des radicaux comportant au moins 8 atomes de carbone, ou des radicaux aliphatiques substitués ayant de 6 à 20 atomes de carbone ou des radicaux alcoylaryle ayant de 6 à 20 atomes de carbone; la somme des atomes de carbone de $R_1$ et $R_2$ étant de préférence inférieure ou égale à 28; l'un des radicaux peut, en outre, désigner un radical diméthylaminoéthyle ou propyle, diéthylaminoéthyle ou propyle, pipéridino éthyle ou propyle, morpholino éthyle ou propyle ou alcoyl polyhydroxy propylène;
*u* désigne zéro ou 1

par réaction du tétramère de l'épichlorhydrine de formule (III)

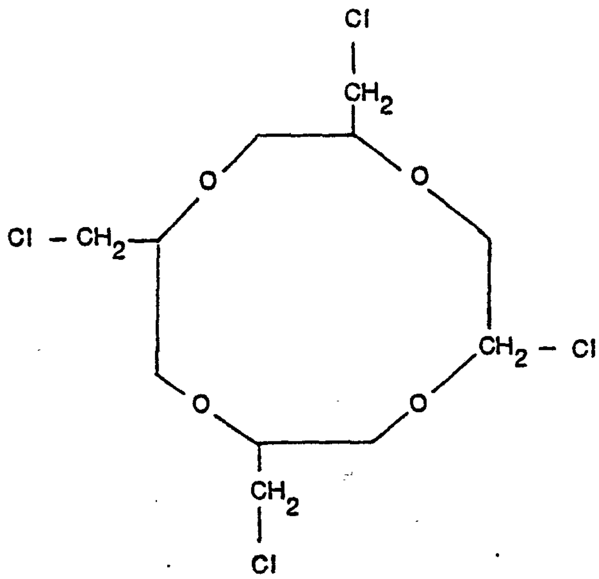

(III)

avec une amine secondaire $HNR_1R_2$, $R_1$ et $R_2$ ayant la signification ci-dessus indiquée, entre 80 et 150°C, éventuellement en présence d'un solvant.

21. Procédé de préparation d'un polyéther cyclique tensio-actif de formule (I) dans laquelle A désigne:

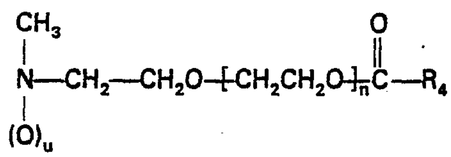

($a_1$)

où *n* désigne un nombre quelconque de 1 à 20; *u* désigne zéro ou 1; $R_4$ désigne un radical aliphatique non substitué ou substitué ou un radical alcoylaryle ayant de 6 à 20 atomes de carbone, par réaction du tétramère de l'épichlorhydrine de formule (III) sur la méthyléthanolamine, suivie de l'addition de *n* moles d'oxyde d'éthylène sur le produit obtenu et enfin estérification des fonctions OH avec un acide de formule $R_4COOH$.

22. Procédé de préparation d'un polyéther cyclique tensio-actif de formule I dans laquelle A désigne:

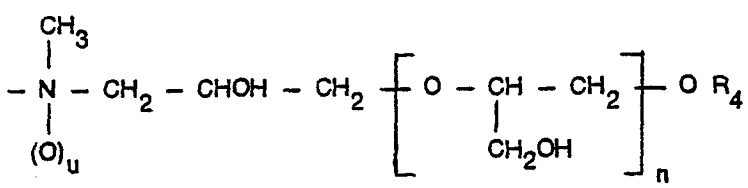

($a_2$)

où *n*, *u*, $R_4$ ont les significations indiquées dans la revendication 10, caractérisé par le fait que (1) on fait réagir le tétramère de l'épichlorhydrine de formule (III) avec la méthylamine, (2) on condense sur le produit résultant un composé de formule:

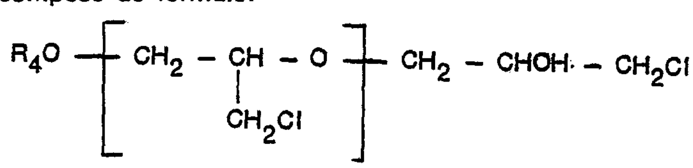

(3) on remplace dans le composé résultant les atomes de chlore par des groupes hydroxyle à l'aide d'acétate de sodium ou de potassium suivi d'hydrolyse ou alcoolyse de l'ester acétique formé.

23. Procédé de préparation d'un polyéther cyclique tensio-actif de formule (I) dans laquelle A désigne le groupement:

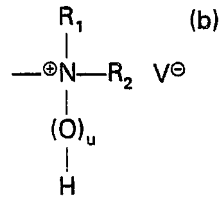 (b) ou 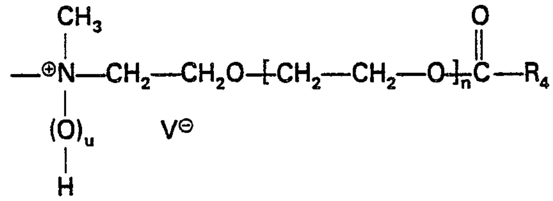 ($b_1$) ou

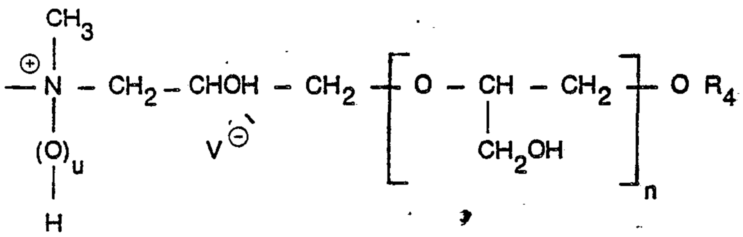 ($b_2$)

où V⊖ désigne un anion et de préférence un formiate, acétate, citrate ou lactate; *u*, $R_1$, $R_2$, $R_4$, *n* ont les significations indiquées dans la revendication 10, par salification d'un composé de formule (I) dans laquelle A désigne respectivement le groupement

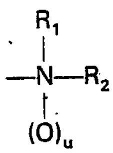 (a) ou 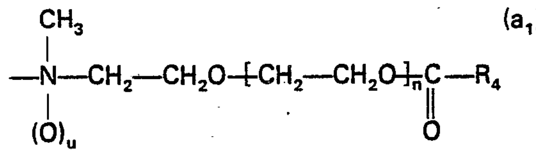 ($a_1$) ou

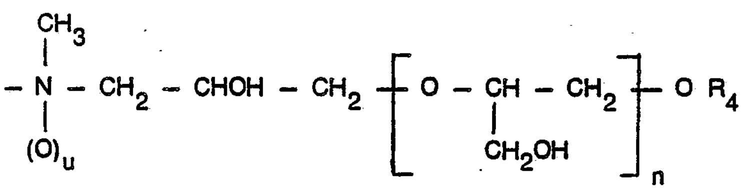 ($a_2$)

avec un acide organique et de préférence avec l'acide formique, citrique, lactique ou tartrique.

24. Procédé de préparation d'un polyéther cyclique tensio-actif de formule (I) dans laquelle A désigne le groupement

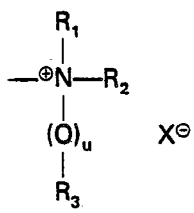 (c) ou

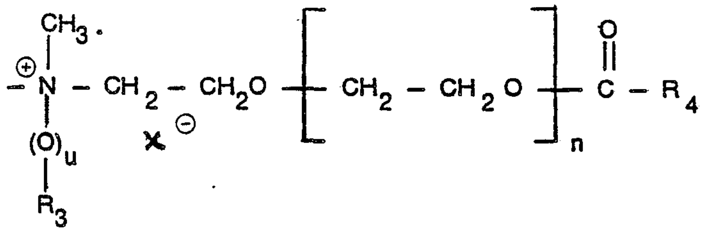 ($c_1$) ou

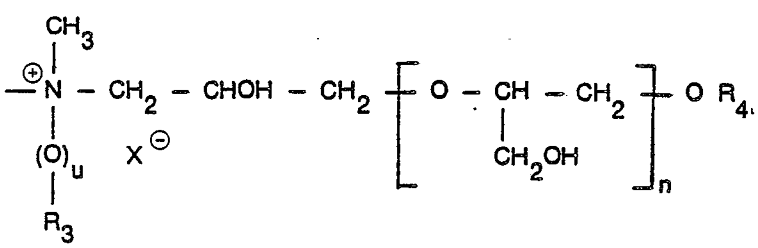 ($c_2$)

où $R_3$ désigne un radical alcoyle ou hydroxyalcoyle ayant de 1 à 3 atomes de carbone et de préférence un radical méthyle, éthyle, hydroxyéthyle, dihydroxypropyle; ou un radical benzyle;
$X^{\ominus}$ désigne un anion et de préférence un chlorure, bromure, iodure, monométhyl sulfate, méthyl sulfonate, p.toluène sulfonate;
*u*, $R_1$, $R_2$, $R_4$, *n* ont les significations indiquées dans la revendication 10, par alcoylation d'un composé de formule (I) dans laquelle A désigne respectivement le groupement:

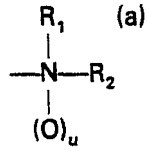 (a) ou 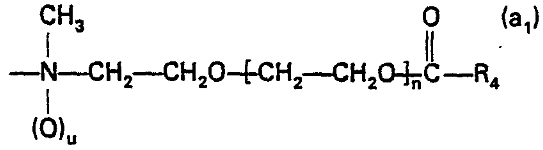 ($a_1$) ou

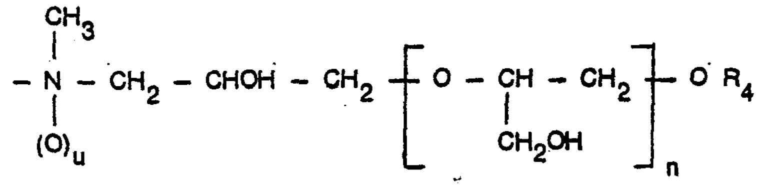 ($a_2$)

avec un agent alcoylant $XR_3$ où X et $R_3$ ont la signification ci-dessus.

25. Procédé de préparation d'un polyéther cyclique tensio-actif de formule (I) dans laquelle A désigné le groupement:

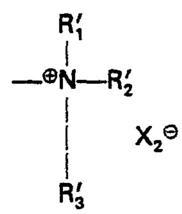 (c')

où $R'_1$ et $R'_2$ désignent un radical alkyle ou hydroxyalkyle ayant de 1 à 3 atomes de carbone, $R'_3$ désigne un radical alkyle ayant de 8 à 20 atomes de carbone et $X_2^{\ominus}$ désigne un groupement mésylate ou tosylate par réaction du tétramère de l'épichlorhydrine de formule (III) avec une amine secondaire de formule:

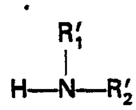

où $R'_1$ et $R'_2$ ont les significations indiquées ci-dessus suivie d'une alcoylation avec un mésylate ou tosylate d'un alcool ou d'un mélange d'alcools ayant de 8 à 20 atomes de carbone.

26. Procédé de préparation d'un polyéther cyclique tensio-actif de formule (I) dans laquelle A désigne le groupement:

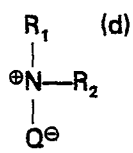 (d) ou 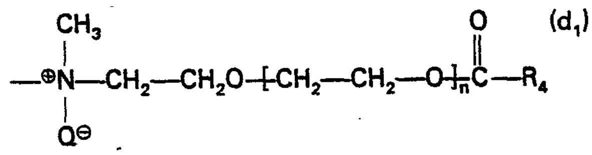 ($d_1$) ou

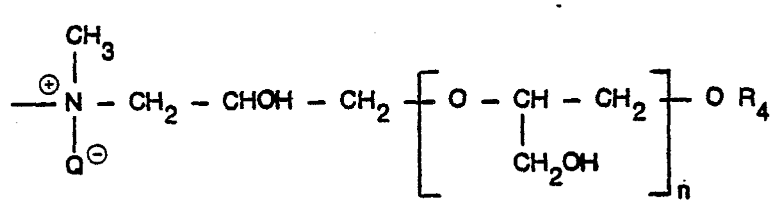 ($d_2$)

où $Q^{\ominus}$ désigne l'un des groupements suivants:

$-(CH_2)_m-COO^-$ ou *m* désigne 1, 2 ou 3

$-CH_2-CH_2-CH_2-SO_3^-$

$-CH_2-CH_2OSO_2^-$

$R_1$, $R_2$, $R_4$, *n* ont les significations indiquées dans la revendication 10 par alcoylation d'un composé de formule (I) dans laquelle A désigne respectivement le groupement

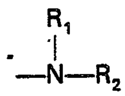 (a') ou

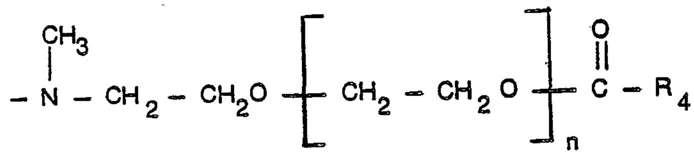 ($a_1'$) ou

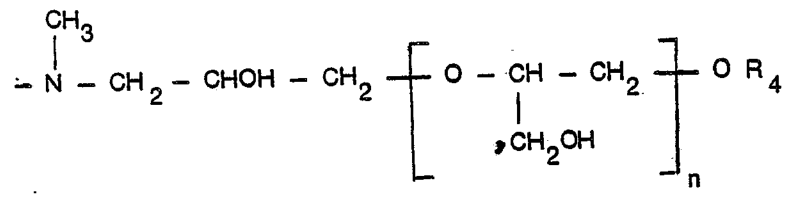 ($a_2'$)

$R_1$, $R_2$, $R_4$ et n ayant les significations indiquées ci-dessus avec l'un des acides de formule:

$Cl(CH_2)_mCOOH$; $Br(CH_2)_mCOOH$ où *m* désigne 1, 2 ou 3

$ClCH_2—CH_2—CH_2—SO_3H$; $BrCH_2CH_2CH_2—SO_3H$

ou avec le sel de sodium ou de potassium d'un tel acide; ou avec la propane sultone ou le sulfite de glycol.

27. Procédé de préparation d'un polyéther cyclique tensio-actif de formule (I) dans laquelle A désigne:

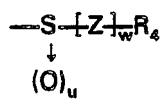 (e)

où Z désigne des groupements d'atomes à caractère hydrophile se présentant sous la forme d'un radical renfermant un ou plusieurs groupements éther, ester, amide, amine, ammonium et/ou hydroxyle; $R_4$ a la signification indiquée dans la revendication 10; *u* désigne zéro ou 1; *w* désigne zéro ou 1, caractérisé par le fait qu'au premier stade on fait réagir avec le tétramère de l'épichlorhydrine de formule (III) un réactif choisi dans le groupe formé par les alcoylmercaptans, les hydroxyalcoylmercaptans, les alcoyl mono- ou polyéthoxymercaptans, les esters méthylique ou éthylique d'un acide mercapto-acétique ou mercapto-propionique, à une température comprise entre 80 et 150°C, en présence d'un composé alcalin choisi dans le groupe formé par l'hydroxyde de sodium, l'hydroxyde de potassium, le méthylate ou l'éthylate de sodium ou de potassium et qu'éventuellement en un second stade on transforme le groupement ester obtenu au premier stade en groupement acide correspondant, par saponification ou hydrolyse, ou en un ester différent par transestérification ou en un groupement amide par réaction avec une amine primaire ou une diamine secondaire-tertiaire et qu'éventuellement on oxyde le groupement thioéther en sulfoxyde au moyen d'eau oxygénée et/ou transforme le groupement thioéther ou sulfoxyde en groupement sulfonium ou sulfoxonium et/ou on transforme le groupement amine tertiaire en groupement ammonium par alcoylation avec un agent alcoylant.

28. Procédé selon la revendication 27, caractérisé par le fait que Z se présente sous la forme d'un radical renfermant un ou plusieurs groupements éther et/ou hydroxyle; qu'au premier stade l'on fait réagir sur le tétramère de l'épichlorhydrine de formule (III) un réactif choisi parmi les alcoyl-mercaptans, les hydroxyalcoylmercaptans, les alcoyl-mono- ou poly-éthoxymercaptans.

29. Procédé selon la revendication 27, caractérisé par le fait que *w* désigne 1; Z désigne le groupement:

$—(CH_2)_x—COO—$ ou $—(CH_2)_x—CONH—$ ou 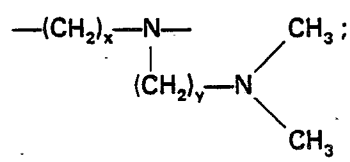

où *x* désigne 1 ou 2; y désigne 2 ou 3

que dans un premier stade on prépare un composé intermédiaire de formule:

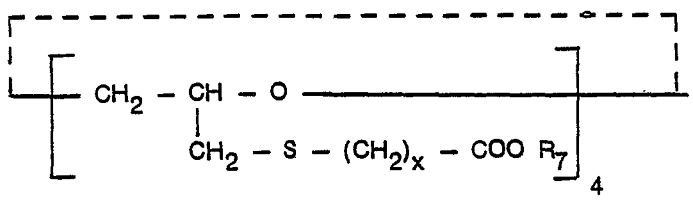

(IV)

où *x* désigne 1 ou 2
$R_7$ désigne $CH_3$ ou $C_2H_5$
par réaction du tétramère de l'épichlorhydrine de formule (III) avec un ester de formule:

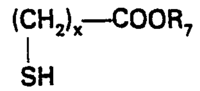

où x et $R_7$ ont les significations ci-dessus indiquées et que dans un second stade on fait réagir ce composé intermédiaire de formule (IV) avec un alcool de formule $R_4OH$ ou avec une amine primaire de formule $R_4NH_2$ ou avec une diamine secondaire-tertiaire de formule:

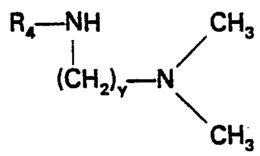

où $R_4$ a la signification indiquée dans la revendication 10;
y désigne 2 ou 3.

30. Procédé selon les revendications 27 et 29 caractérisé par le fait que w désigne 1;
Z désigne le groupement:

$$—(CH_2)_x—COO—CH_2—CHOH$$

où x désigne 1 ou 2
que dans un premier stade on prépare un composé intermédiaire de formule:

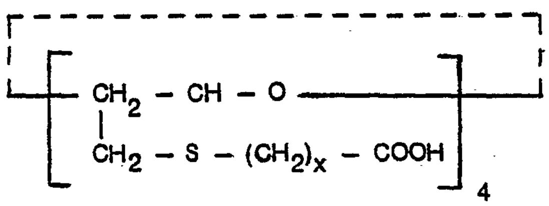

(IV A)

par saponification du composé de formule (IV);
que dans un second stade l'on fait réagir sur le composé intermédiaire de formule (IV A) un époxy 1,2-alcane de formule:

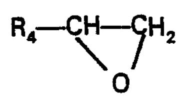

où $R_4$ a la signification indiquée dans la revendication 10.

31. Procédé selon la revendication 27, caractérisé par le fait que
*w* désigne 1
Z désigne le groupement:

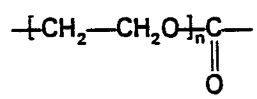

où *n* désigne un nombre quelconque de 1 à 20
que dans un premier stade l'on condense le thioéthanol avec le tétramère de l'épichlorhydrine de formule (III),
que dans un second stade on additionne 1 à 20 moles d'oxyde d'éthylène sur le composé obtenu au premier stade,
que dans un troisième stade on estérifie le composé obtenu au deuxième stade par un acide de formule $R_4—COOH$
où $R_4$ a la signification indiquée dans la revendication 10.

32. Procédé de préparation d'un polyéther cyclique tensio-actif de formule (I) dans laquelle A désigne:

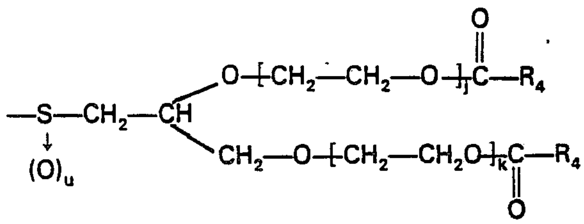

(e$_1$)

où j et k désignent chacun un nombre quelconque inférieur à 20, la somme j + k est comprise entre 1 et 20, *u* désigne 0 ou 1, $R_4$ a la signification indiquée dans la revendication 10, caractérisé par le fait que dans un premier stade on condense le thioglycérol sur le tétramère de l'épichlorhydrine de formule (III), que dans un deuxième stade on effectue l'oxyéthylénation du composé obtenu dans le premier stade, que dans un troisième stade on estérifie avec un acide de formule $R_4COOH$ et qu'éventuellement dans un quatrième stade on oxyde le groupement thioéther en sulfoxyde avec de l'eau oyxgénée et/ou on transforme le groupement thioéther ou sulfoxyde en groupement sulfonium ou sulfoxonium avec un agent alcoylant et de préférence avec le bromure ou iodure de méthyle ou le sulfate de diméthyle.

33. Procédé de préparation d'un polyéther cyclique tensio-actif de formule (I) dans laqulle A désigne:

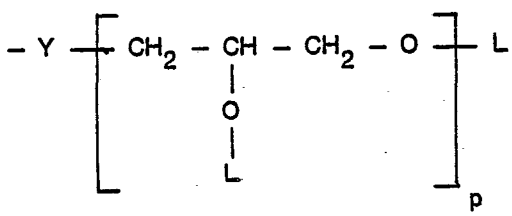

(f)

où L désigne en même temps un atome d'hydrogène et l'un des groupements:

$-CH_2-CHOH-CH_2-(O)_q-R_5$ ou 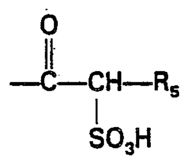 ou 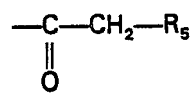

($R_5$ étant un radical alcoyle linéaire ayant de 6 à 18 atomes de carbone, et q désigne zéro ou 1), l'atome d'hydrogène et l'autre groupement étant répartis de façon statistique; Y désigne l'oxygène; p désigne un nombre entier ou décimal de 1 à 10 et représente une valeur statistique moyenne, caractérisé par le fait que dans un premier stade l'on fait réagir le glycidol en catalyse alcaline avec le dérivé hydroxylé du tétramère de l'épichlorhydrine de formule (II)

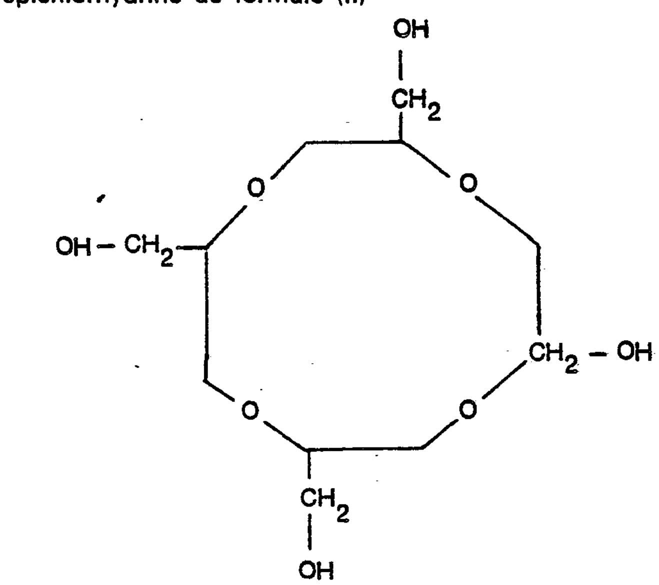

(II)

et que dans un deuxième stade l'on fait réagir le composé obtenu dans le premier stade avec un oxyde d'alcoylène de formule

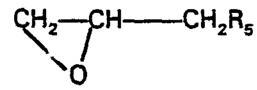

ou avec un alcoyle glycidyléther de formule

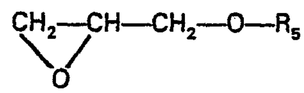

où $R_5$ a la signification ci-dessus indiquée.

34. Procédé de préparation d'un polyéther cyclique tensio-actif de formule (I) dans laquelle A désigne:

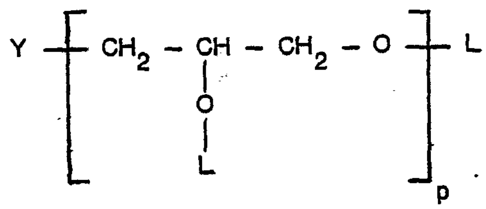

(f)

où L désigne en même temps un atome d'hydrogène et l'un des groupements:

$$-CH_2-CHOH-CH_2-(O)_q-R_5$$

ou 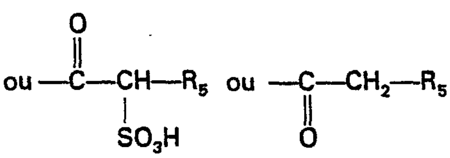 ou

($R_5$ étant un radical alkyle linéaire ayant de 6 à 18 atomes de carbone, et q désigne zéro ou 1), l'atome d'hydrogène et l'autre groupement étant répartis de façon statistique; Y désigne —S— ou 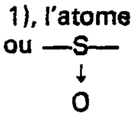

p désigne un nombre entier ou décimal de 1 à 10 et représente une valeur statistique moyenne, caractérisé par le fait que la réaction s'effectue en un ou plusieurs stades; que dans un premier stade on prépare un composé intermédiaire de formule:

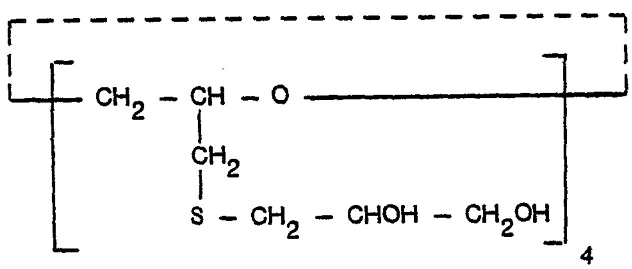

(IX)

par réaction du tétramère de l'épichlorhydrine de formule (III) avec le thioglycérol, en présence d'un solvant et d'un composé alcalin; qu'on fait ensuite réagir sur ce composé intermédiaire de formule (IX), également en présence d'un composé alcalin, 0 à 9 moles de glycidol et/ou d'oxyde d'alcoylène ayant 8 à 20 atomes de carbone et/ou d'alcoyl glycidyl éther; et qu'éventuellement ensuite on estérifie les fonctions hydroxyle avec un acide $\alpha$-sulfocarboxylique ou carboxylique ayant de 8 à 20 atomes de carbone et/ou l'on oxyde le groupement thioéther en groupement sulfoxyde avec de l'eau oxygénée et/ou on alcoyle le groupement thioéther ou sulfoxyde en groupement sulfonium ou sulfoxonium avec un agent alcoylant connu et de préférence avec le bromure ou iodure de méthyle ou le sulfate de diméthyle.

35. Procédé de préparation d'un polyéther cyclique tensio-actif de formule (I), dans laquelle A désigne le groupement:

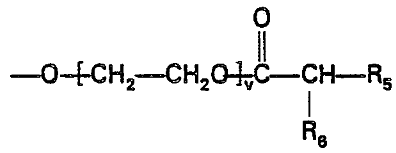

(g)

où v désigne un nombre quelconque égal ou inférieur à 20, à l'exception du nombre zéro; $R_5$ désigne un radical alcoyle linéaire ayant de 6 à 18 atomes de carbone; $R_6$ désigne H ou $SO_3H$, caractérisé par le fait que dans un premier stade on fait réagir sur le dérivé hydroxylé du tétramère de l'épichlorhydrine de formule (II) quatre fois *v* moles d'oxyde d'éthylène et que dans un second stade on estérifie les fonctions hydroxyle avec un acide sulfocarboxylique de formule:

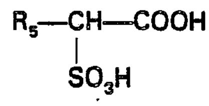

ou avec un acide carboxylique de formule:

$$R_5—CH_2—COOH$$

36. Procédé de préparation d'un polyéther cyclique tensioactif de formule (I) dans laquelle A désigne le groupement:

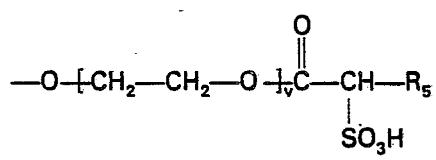

(g)

où *v* désigne zéro, $R_5$ a la signification indiquée dans la revendication 19, caractérisé par le fait qu'on estérifie les fonctions hydroxyle du dérivé hydroxylé du tétramère de l'épichlorhydrine de formule (II) avec un acide sulfocarboxylique de formule:

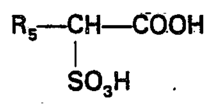

**Claims**

1. Cosmetic or pharmaceutical composition, characterized in that it contains, in the presence of a suitable vehicle, at least one surface-active compound of the general formula:

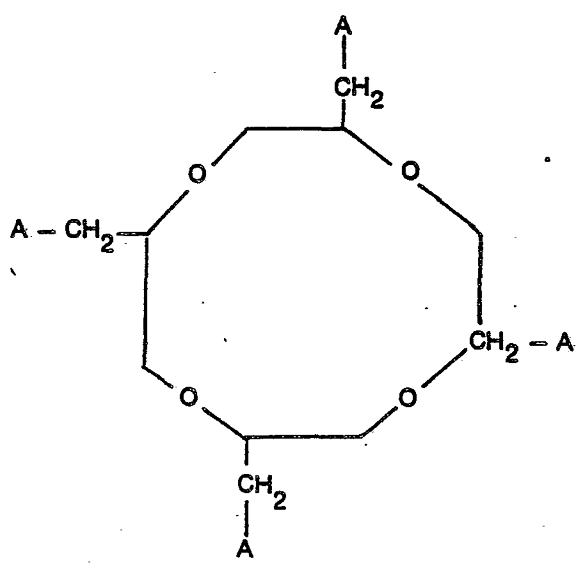

(I)

in which A denotes an amphiphilic unit consisting of a hydrophilic part and a lipophilic part, this

amphiphilic unit being joined to the ring by the hydrophilic part, by means of a covalent bond; the hydrophilic part contains one or more amine, amine oxide, ammonium, ammonioalkanoate, ammonioalkylsulphonate, ammonioalkylsulphinate, thioether, sulphoxide, sulphonium, sulphoxonium, ether, hydroxl, ester, amide or acid groups; the lipophilic part consists of one or more groups chosen from amongst (i) aliphatic groups having from 1 to 20 carbon atoms, (ii) substituted aliphatic groups having from 6 to 20 carbon atoms, and (iii) alkylaryl groups having from 6 to 20 carbon atoms, the alkyl part of the alkylaryl group having up to 14 carbon atoms.

2. Composition according to Claim 1, characterised in that the amphiphilic group A is chosen from amongst the following groups:

a) the group:

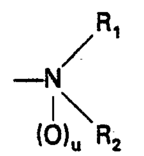

in which $R_1$ and $R_2$, which are identical or different, denote aliphatic radicals having from 1 to 20 carbon atoms, one of the radicals containing at least 8 carbon atoms, or substituted aliphatic radicals having from 6 to 20 carbon atoms, or alkylaryl radicals having from 6 to 20 carbon atoms, the sum of the carbon atoms in $R_1$ and $R_2$ preferably being less than or equal to 28; one of the radicals can also denote a dimethylaminoethyl or dimethylaminopropyl radical, a diethylaminoethyl or diethylaminopropyl radical, a piperidinoethyl or piperidinopropyl radical; a morpholinoethyl or morpholinopropyl radical or an alkylpolyhydroxypropylene radical; *u* denotes the number zero or 1;

$a_1$) the group:

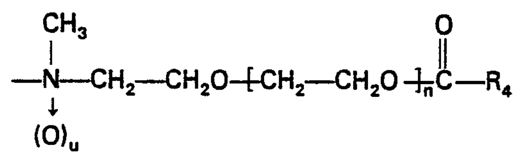

in which $R_4$ denotes an unsubstituted or substituted aliphatic radical or an alkylaryl radical having from 6 to 20 carbon atoms, and preferably an alkyl, hydroxyalkyl, alkenyl or alkylphenyl radical, the alkyl part of the alkylphenyl radical having up to 14 carbon atoms, *n* denotes any number from 1 to 20, and *u* denotes the number 0 or 1;

$a_2$) the group:

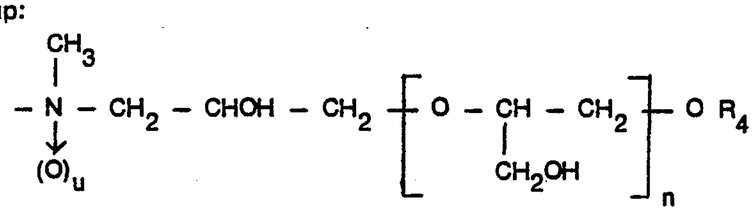

*u*, $R_4$ and *n* having the meaning indicated above;

b) the group:

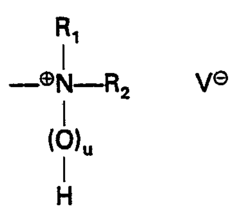

$b_1$) the group:

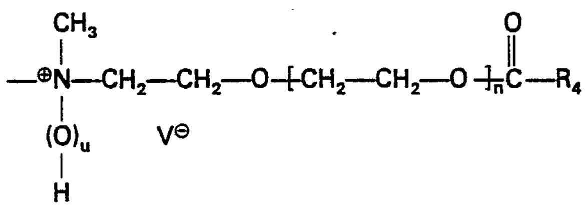

$b_2$) the group:

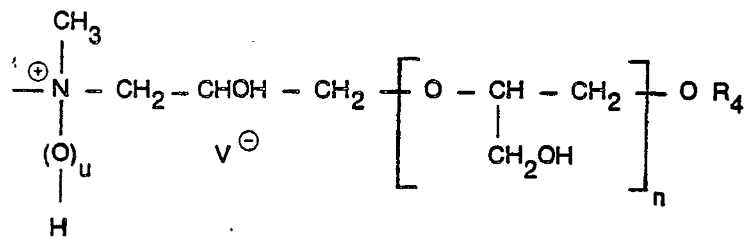

In the groups (b), ($b_1$) and ($b_2$), $V^{\ominus}$ denotes an anion and preferably a formate, acetate, citrate or lactate anion; *u, n,* $R_1$, $R_2$ and $R_4$ have the meanings indicated for the groups a and $a_1$:

c) the group:

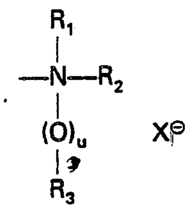

$R_1$, $R_2$ and *u* having the meaning indicated for the group ($a_1$); $R_3$ denotes an alkyl or hydroxyalkyl radical having from 1 to 3 carbon atoms, and preferably a methyl, ethyl, hydroxyethyl or dihydroxypropyl radical or a benzyl radical, and $X^{\ominus}$ denotes an anion, and preferably a chloride, bromide, iodide, monomethyl-sulphate, methyl-sulphonate or p-toluenesulphonate;

$c_1$) the group:

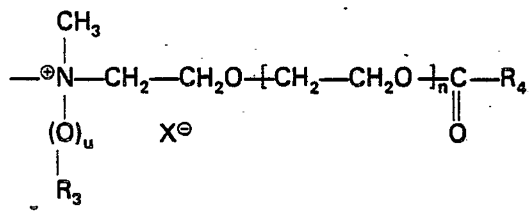

$c_2$) the group:

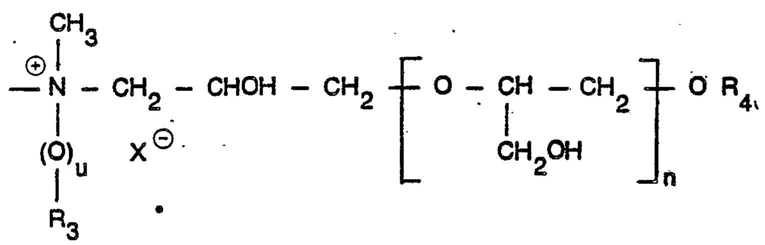

In these groups ($c_1$) and ($c_2$), *u* denotes the number 0 or 1, *n* denotes any number from 1 to 20, $R_4$ has the meanings indicated for the group ($a_1$), and $R_3$ and $X^{\ominus}$ have the meanings indicated for the group (c);

d) the group:

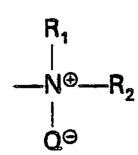

in which $Q^{\ominus}$ denotes one of the following groups:

$-(CH_2)_m COO^{\ominus}$, in which *m* denotes 1, 2 or 3

$-CH_2-CH_2-CH_2-SO_3^{\ominus}$

$-CH_2-CH_2-O-SO_2^{\ominus}$

and $R_1$ and $R_2$ have the meaning indicated for the group (a);

$d_1$) the group:

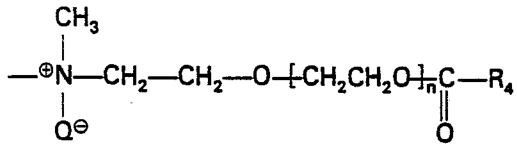

$d_2$) the group:

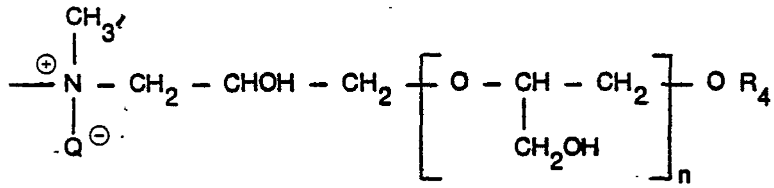

In the groups ($d_1$) and ($d_2$) *n* denotes any number from 1 to 20, $Q^{\ominus}$ has the meaning indicated for the group (d), and $R_4$ has the meaning indicated for the group ($a_1$);

e) the group:

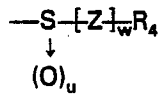

in which $R_4$ has the meaning indicated for the group $a_1$, *u* denotes zero or 1, *w* denotes zero or 1, and Z denotes groups of atoms of hydrophilic character, which can be ether, ester, amide, amine, ammonium and/or hydroxyl groups:

$e_1$) the group:

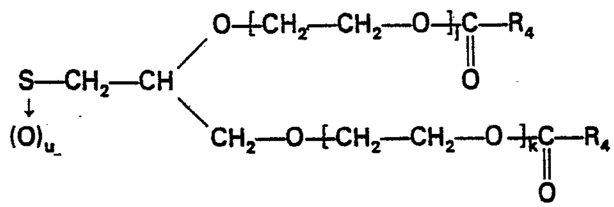

j and k each denote any number less than 20, the sum j+k being between 1 and 20, *u* denotes 0 or 1, and $R_4$ has the meaning indicated for the group $a_1$);

f) the group:

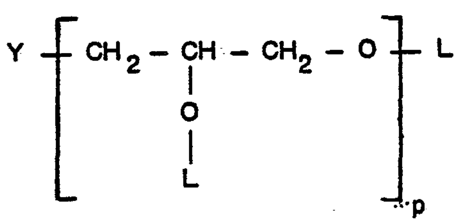

in which L simultaneously denotes a hydrogen atom and one of the following groups:

$—CH_2—CHOH—CH_2—(O)_q—R_5$, in which q denotes 0 or 1

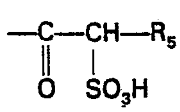

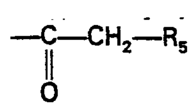

the hydrogen atom and the other group being randomly distributed, $R_5$ denotes a linear alkyl radical having from 6 to 18 carbon atoms, p denotes any number from 1 to 10 and represents an average statistical value, and Y denotes:

—O—, —S— or 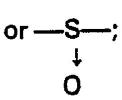

g) the group:

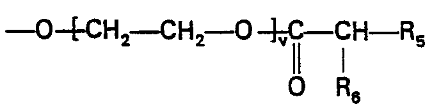

in which v denotes any number from 0 to 20, $R_6$ denotes a hydrogen atom or the radical $—SO_3H$, and $R_5$ has the meaning indicated above;

h) the group (e) or ($e_1$) alkylated by an alkylating agent chosen from the group comprising methyl bromide, methyl iodide and dimethyl sulphate, and preferably dimethyl sulphate;

i) the group (f) alkylated by an alkylating agent chosen from the group comprising methyl bromide, methyl iodide and dimethyl sulphate.

3. Composition according to Claims 1 and 2, characterised in that the vehicle is aqueous or aqueous-alcoholic.

4. Cosmetic composition for treating the hair, containing a cosmetically effective amount of one or more compounds of the formula (I), according to Claim 1 or 2, in solution in a solvent chosen from a group comprising water and an aqueous-alcoholic solution.

5. Shampoo composition for treating the hair, containing a cosmetically effective amount of one or more compounds of the formula (I), according to Claim 1 or 2, in solution in a solvent chosen from a group comprising water and an aqueous-alcoholic solution.

6. Cosmetic composition for treating the hair, according to Claim 3, containing a cosmetically effective amount of one or more compounds of the formula (I), in solution in a solvent chosen from a group comprising water and an aqueous-alcoholic solution, and also containing one or more adjuvants chosen from the group comprising anionic, cationic, amphoteric, zwitterionic and non-ionic surface-active agents, perfumes, dyestuffs, preservatives, thickeners, foaming agents, foam stabilisers, softeners, hair-restructuring agents, anti-dandruff agents, cosmetic resins, foam synergistic agents and electrolytes.

7. Process for treating the hair, consisting in applying, to human hair, an effective amount of a composition containing a cosmetically effective amount of one or more surface-active cyclic polyethers of the formula (I), according to Claim 1 or 2, in solution in a solvent chosen from a group comprising water and an aqueous-alcoholic solution.

8. Hair-dyeing composition containing a cosmetically effective amount of one or more compounds of the formula (I), according to Claim 1 or 2, in solution in a solvent chosen from a group comprising water and an aqueous-alcoholic solution, and also containing a dyestuff for the hair and, if appropriate, one or more adjuvants chosen from the group comprising anionic, cationic, non-ionic, zwitterionic and amphoteric surface-active agents and mixtures thereof, perfumes, preservatives, thickeners, softeners, cosmetic resins and sequestering agents.

9. Composition according to Claim 8, characterised in that the dyestuff is chosen from amongst anthraquinone dyestuffs, azo dyestuffs, nitro dyestuffs of the benzene series, indophenols, indoanilines and indamines.

10. Surface-active cyclic polyethers of the general formula:

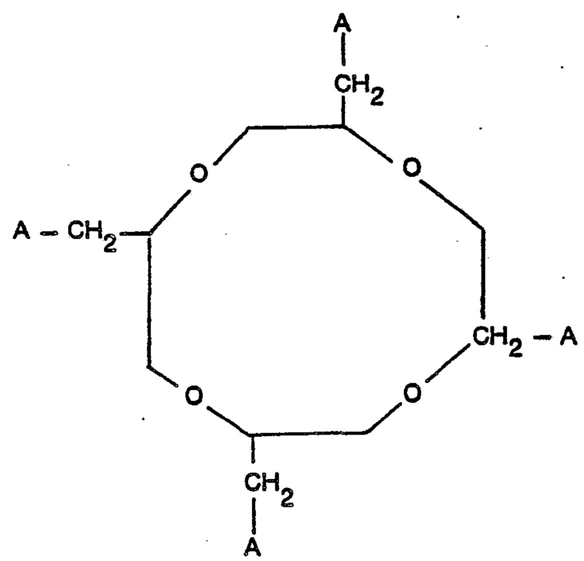

(I)

in which A denotes an amphiphilic unit consisting of a hydrophilic part and a lipophilic part, this amphiphilic unit being joined to the ring by the hydrophilic part, by means of a covalent bond; the amphiphilic part contains one or more groups chosen from amongst the following groups:

a) the group:

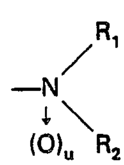

in which $R_1$ and $R_2$, which are identical or different, denote aliphatic radicals having from 1 to 20 carbon atoms, one of the radicals containing at least 8 carbon atoms, or substituted aliphatic radicals having from 6 to 20 carbon atoms, or alkylaryl radicals having from 6 to 20 carbon atoms, the sum of the carbon atoms in $R_1$ and $R_2$ preferably being less than or equal to 28; one of the radicals can also denote a dimethylaminoethyl or dimethylaminopropyl radical, a diethylaminoethyl or diethylaminopropyl radical, a piperidinoethyl or piperidinopropyl radical, a morpholinoethyl or morpholinopropyl radical or an alkylpolyhydroxypropylene radical; *u* denotes the number zero or 1;

$a_1$) the group:

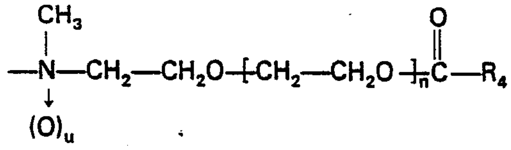

in which $R_4$ denotes an unsubstituted or substituted aliphatic radical or an alkylaryl radical having from 6 to 20 carbon atoms, and preferably an alkyl, hydroxyalkyl, alkenyl or alkylphenyl radical, the alkyl part of the alkylphenyl radical having up to 14 carbon atoms, *n* denotes any number from 1 to 20, and *u* denotes the number 0 or 1;

$a_2$) the group:

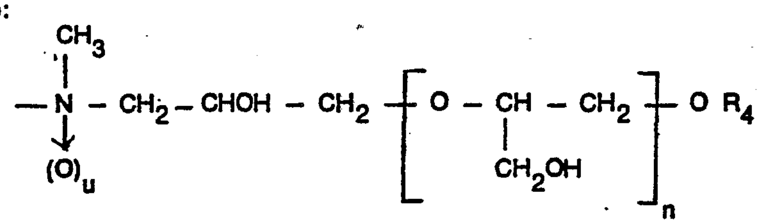

*u*, $R_4$ and *n* having the meaning indicated above;

b) the group:

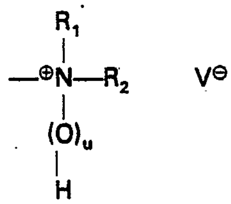

$b_1$) the group:

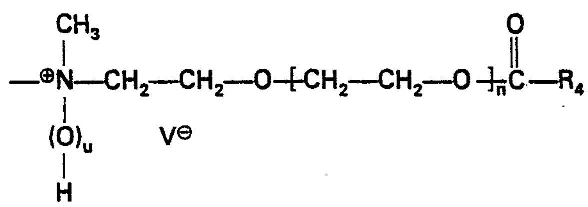

$b_2$) the group:

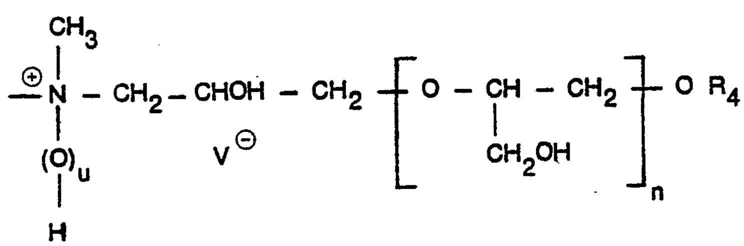

In the groups (b), ($b_1$) and ($b_2$), $V^\ominus$ denotes an anion and preferably a formate, acetate, citrate or lactate anion; *u*, *n*, $R_1$, $R_2$ and $R_4$ have the meanings indicated for the groups a and $a_1$:

c) the group:

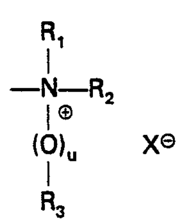

$R_1$, $R_2$ and *u* having the meaning indicated for the group ($a_1$); $R_3$ denotes an alkyl or hydroxyalkyl radical having from 1 to 3 carbon atoms, and preferably a methyl, ethyl, hydroxyethyl or dihydroxypropyl radical or a benzyl radical, and $X^{\ominus}$ denotes an anion, and preferably a chloride, bromide, iodide, monomethyl-sulphate, methylsulphonate or p-toluenesulphonate;

$c_1$) the group:

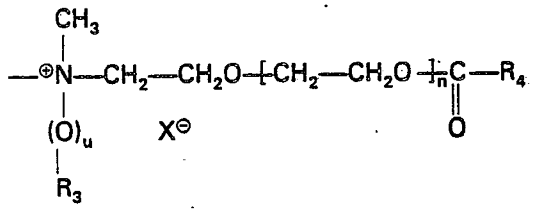

$c_2$) the group:

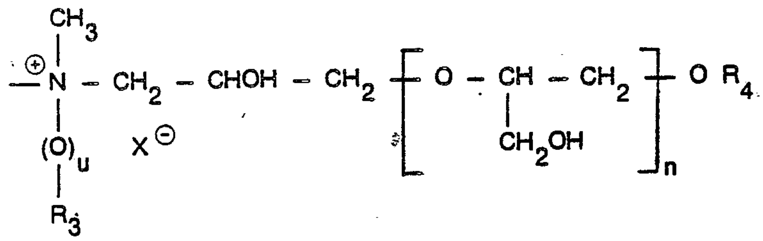

In these groups ($c_1$) and ($c_2$), *u* denotes the number 0 or 1, *n* denotes any number from 1 to 20, $R_4$ has the meanings indicated for the group ($a_1$), and $R_3$ and $X^{\ominus}$ have the meanings indicated for the group (c);

d) the group:

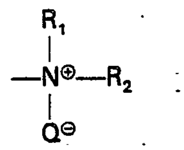

in which $Q^{\ominus}$ denotes one of the following groups:

$-(CH_2)_m-COO^{\ominus}$, in which *m* denotes 1, 2 or 3

$-CH_2-CH_2-CH_2-SO_3^{\ominus}$

$-CH_2-CH_2-O-SO_2^{\ominus}$

and $R_1$ and $R_2$ have the meaning indicated for the group (a);

$d_1$) the group:

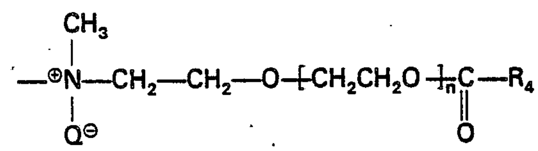

$d_2$) the group:

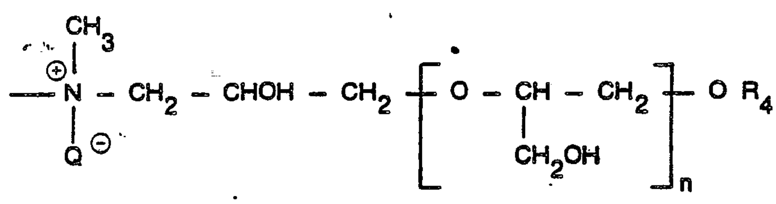

In the groups ($d_1$) and ($d_2$) *n* denotes any number from 1 to 20, $Q^{\ominus}$ has the meaning indicated for the group (d), and $R_4$ has the meaning indicated for the group ($a_1$);

e) the group:

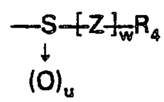

in which $R_4$ has the meaning indicated for the group ($a_1$), *u* denotes zero or 1, *w* denotes zero or 1, and Z denotes groups of atoms of hydrophilic character, which can be ether, ester, amide, amine, ammonium and/or hydroxyl groups;

$e_1$) the group:

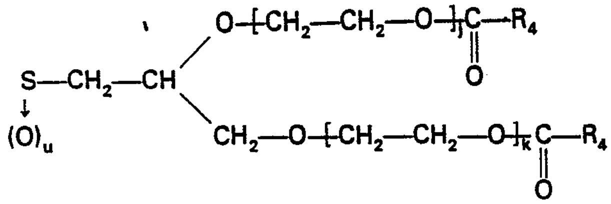

j and k each denote any number less than 20, the sum j+k being between 1 and 20, *u* denotes 0 or 1, and $R_4$ has the meaning indicated for the group $a_1$);

f) the group:

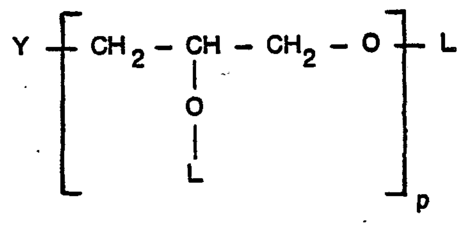

in which L simultaneously denotes a hydrogen atom and one of the following groups:

$—CH_2—CHOH—CH_2—(O)_q—R_5$, in which q denotes 0 or 1

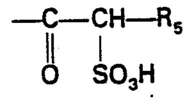 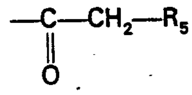

the hydrogen atom and the other group being randomly distributed, $R_5$ denotes a linear alkyl radical having from 6 to 18 carbon atoms, p denotes any number from 1 to 10 and represents an average statistical value, and Y denotes:

—O—, —S— or 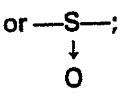;

g) the group:

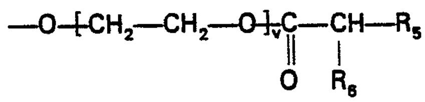

in which v denotes any number from 0 to 20, $R_6$ denotes a hydrogen atom or the radical $—SO_3H$, and $R_5$ has the meaning indicated above;

h) the group (e) or ($e_1$) alkylated by an alkylating agent chosen from the group comprising methyl bromide, methyl iodide and dimethyl sulphate, and preferably dimethyl sulphate;

i) the group (f) alkylated by an alkylating agent chosen from the group comprising methyl bromide, methyl iodide and dimethyl sulphate.

11. Surface-active cyclic polyether according to Claim 10, characterised in that the amphiphilic group A is chosen from amongst the following groups:

a) the group:

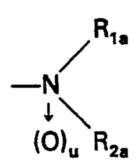

in which $R_{1a}$ and $R_{2a}$, which are identical or different, denote alkyl, hydroxyalkyl or alkenyl radicals having 1 to 20 carbon atoms, one of the radicals containing at least 8 carbon atoms, or alkylaryl radicals having from 6 to 20 carbon atoms, the sum of the carbon atoms in $R_{1a}$ and $R_{2a}$ preferably being less than or equal to 28; one of the radicals can also denote a dimethylaminoethyl or dimethylaminopropyl radical, a diethylaminoethyl or diethylaminopropyl radical, a piperidinoethyl or

piperidinopropyl radical, a morpholinoethyl or morpholinopropyl radical or an alkylpolyhydroxypropylene radical; *u* denotes the number zero or 1;

$a_1$) the group:

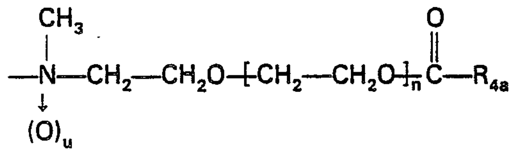

in which $R_{4a}$ denotes an alkyl, hydroxyalkyl, alkenyl or alkylphenyl radical having from 6 to 20 carbon atoms, the alkyl part of the alkylphenyl radical having up to 14 carbon atoms, *n* denotes any number from 1 to 20, and *u* denotes the number 0 or 1;

$a_2$) the group:

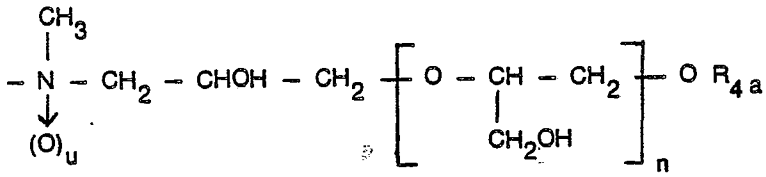

*u*, $R_{4a}$ and *n* having the meaning indicated above;

b) the group:

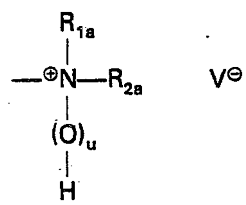

$b_1$) the group:

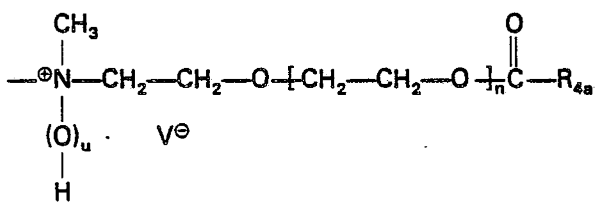

$b_2$) the group:

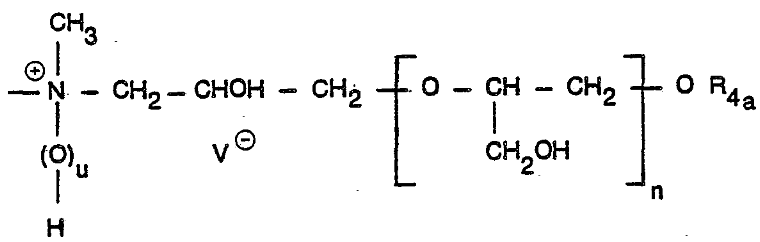

In the groups (b), ($b_1$) and ($b_2$), $V^{\ominus}$ denotes an anion, and preferably a formate, acetate, citrate or lactate anion, and u, n, $R_{1a}$, $R_{2a}$ and $R_{4a}$ have the meanings indicated for the groups a and $a_1$;

c) the group:

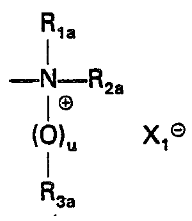

$R_{1a}$, $R_{2a}$ and *u* having the meaning indicated for the group (a); $R_{3a}$ denotes a methyl, ethyl, hydroxyethyl or dihydroxypropyl radical or a benzyl radical, and $X_1^{\ominus}$ denotes a chloride, bromide, iodide, monomethyl-sulphate, methylsulphonate or p-toluenesulphonate anion;

$c_1$) the group:

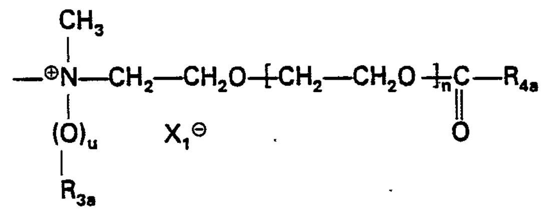

$c_2$) the group:

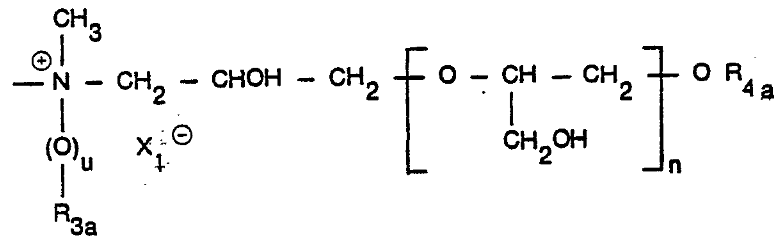

In these groups ($c_1$) and ($c_2$), *u* denotes the number 0 or 1, *n* denotes any number from 1 to 20, $R_{4a}$ has the meanings indicated for the group ($a_1$), and $R_3$ and $X_1^{\ominus}$ have the meanings indicated for the group (c);

d) the group:

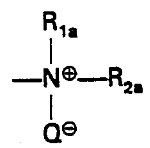

in which $Q^{\ominus}$ denotes one of the following groups:

$-(CH_2)_m-COO^{\ominus}$, in which *m* denotes 1, 2 or 3

$-CH_2-CH_2-CH_2-SO_3^{\ominus}$

$-CH_2-CH_2-O-SO_2^{\ominus}$

and $R_{1a}$ and $R_{2a}$ have the meaning indicated for the group (a);

$d_1$) the group:

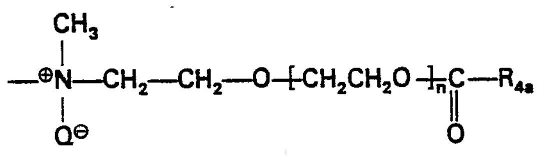

$d_2$) the group:

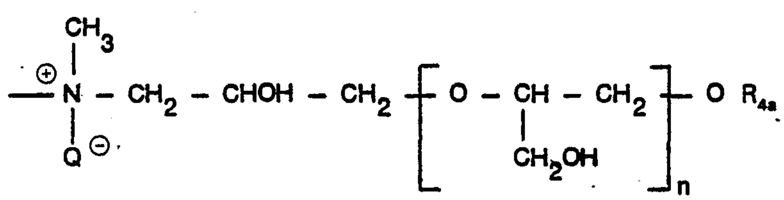

In the groups ($d_1$) and ($d_2$), *n* denotes any number from 1 to 20, $R_{4a}$ has the meanings indicated for the group ($a_1$), and $Q^{\ominus}$ has the meaning indicated for the group (d);

e) the group:

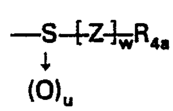

in which $R_{4a}$ has the meanings indicated for the group ($a_1$), *u* denotes zero or 1, w denotes zero or 1, and $Z_1$ denotes a group of atoms of hydrophilic character, chosen from the group comprising:

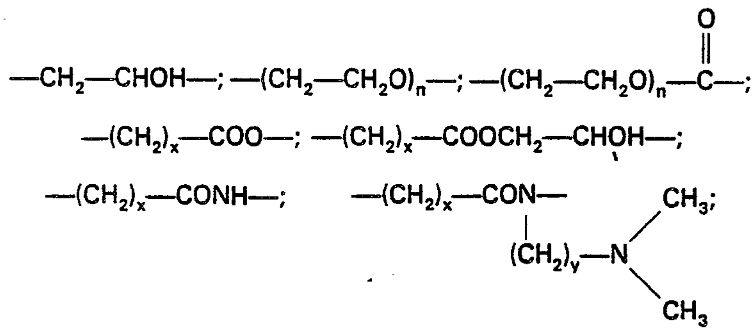

in which x represents 1 or 2, y represents 2 or 3, and *n* represents any number from 1 to 20.

$e_1$) the group:

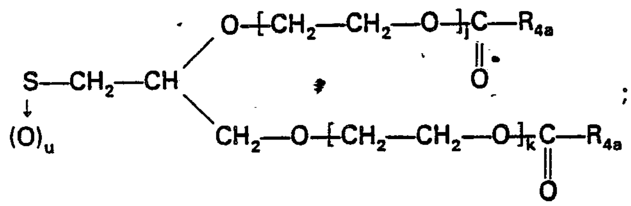

;

j and k each denote any number less than 20, the sum j + k being between 1 and 20, *u* denotes 0 or 1, and $R_{4a}$ has the meaning indicated for the group $a_1$;

f) the group:

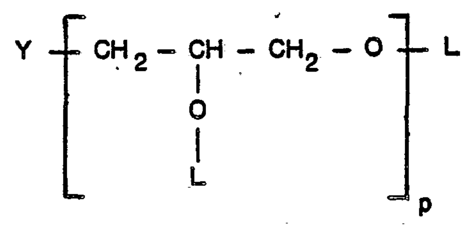

;

L simultaneously denotes a hydrogen atom and one of the groups:

$-CH_2-CHOH-CH_2-(O)_q-R_5$, in which q denotes 0 or 1

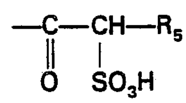

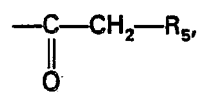

the hydrogen atom and the other group being randomly distributed, $R_5$ denotes a linear alkyl radical having from 6 to 18 carbon atoms, P denotes any number from 1 to 10 and represents an average statistical value, and y denotes:

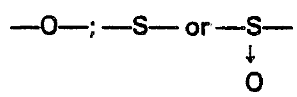

g) the group:

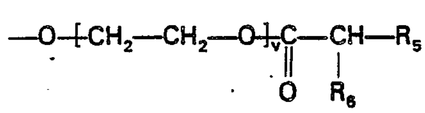

in which *v* denotes any number from 0 to 20, $R_6$ denotes a hydrogen atom or the radical $-SO_3H$, $R_5$ has the meaning indicated above;

h) the groups (e) or ($e_1$) alkylated by an alkylating agent chosen from the group comprising methyl bromide, methyl iodide and dimethyl sulphate, and preferably dimethyl sulphate;

i) the group (f) alkylated by an alkylating agent chosen from the group comprising methyl bromide, methyl iodide and dimethyl sulphate.

12. Surface-active cyclic polyether according to Claim 11, characterised in that the amphiphilic group A is the group (a):

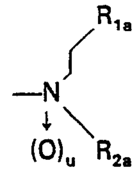

in which $R_{1a}$ and $R_{2a}$, which are identical or different, denote alkyl or hydroxyalkyl radicals having from 1 to 20 carbon atoms, one of the radicals containing at least 8 carbon atoms, the sum of the carbon atoms in $R_{1a}$ and $R_{2a}$ preferably being less than or equal to 28; one of the radicals can also denote a dimethylaminoethyl or dimethylaminopropyl radical, a diethylaminoethyl or diethylaminopropyl radical, a piperidinoethyl or piperidinopropyl radical, a morpholinoethyl or morpholinopropyl radical or an alkylpolyhydroxypropylene radical; *u* denotes the number zero or one.

13. Surface-active cyclic polyether according to Claim 11, characterised in that the amphiphilic group A is the group ($a_1$)

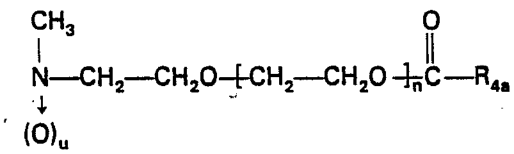

in which $R_{4a}$ denotes an alkyl, hydroxyalkyl, alkenyl or alkylphenyl radical having from 6 to 20 carbon atoms, the alkyl part of the alkylphenyl radical having up to 14 carbon atoms, *n* denotes any number from 1 to 20, and *u* denotes thenumber 0 or 1.

14. Surface-active cyclic polyether according to Claim 11, characterised in that the amphiphilic group A is the group (b):

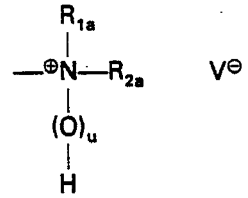

in which $R_{1a}$ and $R_{2a}$, which are identical or different, denote alkyl or hydroxyalkyl radicals having from 1 to 20 carbon atoms, one of the radicals containing at least 8 carbon atoms, the sum of the carbon atoms in $R_{1a}$ and $R_{2a}$ being less than or equal to 28; one of the radicals $R_{1a}$ or $R_{2a}$ can also denote a radical chosen from the group comprising dimethylaminoethyl, dimethylaminopropyl, diethylaminoethyl, diethylaminopropyl, piperidinoethyl, piperidinopropyl, morpholinoethyl, morpholinopropyl and alkylpolyhydroxypropylene radicals; *u* denotes the number zero or 1; V denotes an anion chosen from the group comprising formate, acetate, citrate and lactate anions.

15. Surface-active cyclic polyether according to Claim 11, characterized in that the amphiphilic group A is the group (c):

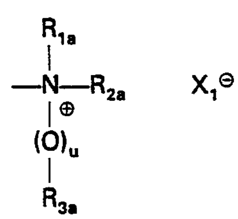

in which $R_{1a}$ and $R_{2a}$, which are identical or different, denote alkyl or hydroxyalkyl radicals having from 1 to 20 carbon atoms, one of the radicals containing at least 8 carbon atoms, the sum of the carbon atoms in $R_{1a}$ and $R_{2a}$ being less than or equal to 28; one of the radicals can also denote a radical chosen from the group comprising dimethylaminoethyl, dimethylaminopropyl, diethylaminoethyl, diethylaminopropyl, piperidinoethyl, piperidinopropyl, morpholinoethyl, morpholinopropyl and alkylpolyhydroxypropylene; $R_{3a}$ denotes a methyl, ethyl, hydroxyethyl or dihydroxypropyl radical or a benzyl radical; $X_1^{\ominus}$ denotes an anion chosen from the group comprising chloride, bromide, iodide, monomethyl-sulphate, methylsulphonate and p-toluenesulphonate; *u* denotes the number 0 or 1.

16. Surface-active cyclic polyether according to Claim 11, characterised in that the amphiphilic group A is the group ($c_2$):

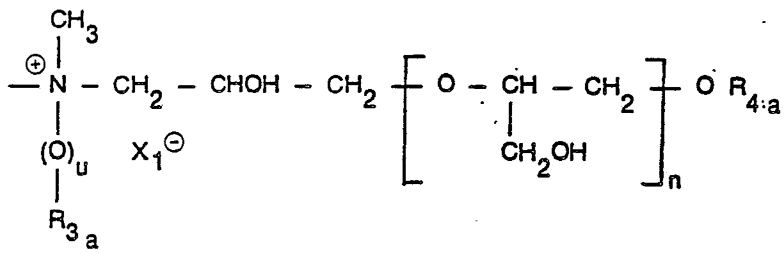

in which *u* denotes the number 0 or 1, *n* denotes any number from 1 to 20, $R_{3a}$ denotes a methyl, ethyl, hydroxyethyl or dihydroxypropyl radical or a benzyl radical, and $R_{4a}$ denotes an alkyl, hydroxyalkyl, alkenyl or alkylphenyl radical having from 6 to 20 carbon atoms, the alkyl part of the alkylphenyl radical having up to 14 carbon atoms.

17. Surface-active cyclic polyether according to Claim 11, characterised in that the amphiphilic group A is the group (e):

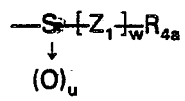

in which $R_{4a}$ denotes an alkyl, hydroxyalkyl, alkenyl or alkylphenyl radical having from 6 to 20 carbon atoms, the alkyl part of the alkylphenyl radical having up to 14 carbon atoms, *u* denotes 0 or 1, w denotes 0 or 1, and $Z_1$ denotes a group chosen from the group comprising:

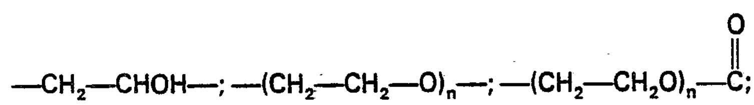

$-(CH_2)_x-COO-$; $-(CH_2)_x-COOCH_2-CHOH-$;

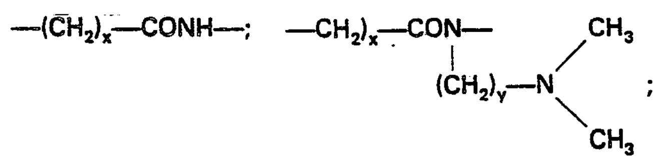

in which *x* represents 1 or 2, y represents 2 or 3, and *n* represents any number from 1 to 20.

18. Surface-active cyclic polyether according to Claim 11, characterised in that the amphiphilic group A is the group (f):

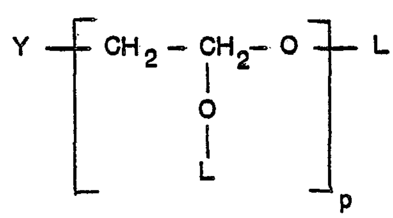

in which L simultaneously denotes a hydrogen atom and one of the groups:

$-CH_2-CHOH-CH_2-(O)_q-R_5$, in which q denotes 0 or 1

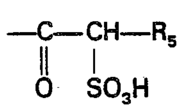

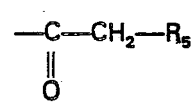

the hydrogen atom and the other group being randomly distributed, $R_5$ denotes a linear alkyl radical having from 6 to 18 carbon atoms, p denotes any number from 1 to 10 and represents an average statistical value, and Y denotes:

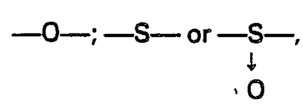

it being possible for this group to be alkylated by an alkylating agent preferably chosen from the group comprising methyl bromide, methyl iodide and dimethyl sulphate.

19. Surface-active cyclic polyether according to Claim 11, characterised in that the amphiphilic group A is the group (g):

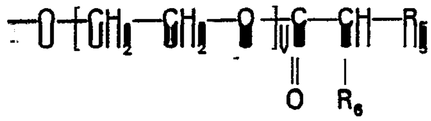

in which $v$ denotes any number from 0 to 20, $R_5$ denotes a linear alkyl radical having from 6 to 18 carbon atoms, and $R_6$ denotes a hydrogen atom or the radical $—SO_3H$.

20. Process for the prepareation of a surface active cyclic polyether of the formula (I) in which A denotes:

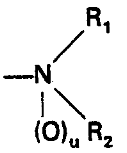
(a)

in which $R_1$ and $R_2$, which are identical or different, denote aliphatic radicals having from 1 to 20 carbon atoms, one of the radicals containing at least 8 carbon atoms, or substituted aliphatic radicals having from 6 to 20 carbon atoms, or alkylaryl radicals having from 6 to 20 carbon atoms, the sum of the carbon atoms in $R_1$ and $R_2$ preferably being less than or equal to 28, it also being possible for one of the radicals to denote a dimethylaminoethyl or dimethylaminopropyl radical, a diethylaminoethyl or diethylaminopropyl radical, a piperidinoethyl or piperidinopropyl radical, a morpholinoethyl or morpholinopropyl radical or an alkylpolyhydroxypropylene radical, and $u$ denotes zero or 1, by reacting epichlorohydrin tetramer of the formula (III)

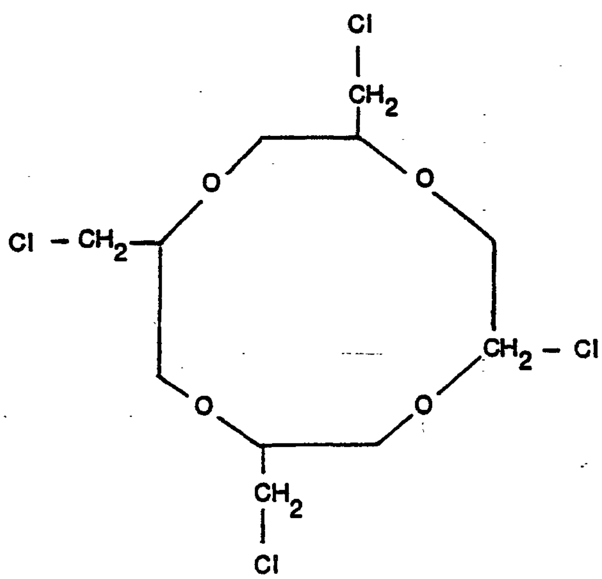
(III)

with a secondary amine $HNR_1R_2$, $R_1$ and $R_2$ having the meaning indicated above, at between 80 and 150°C, if appropriate in the presence of a solvent.

21. Process for the preparation of a surface-active cyclic polyether of the formula (I) in which A denotes:

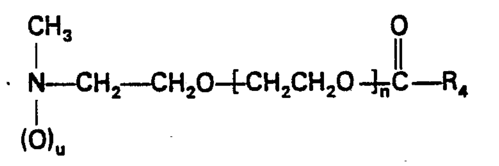
($a_1$)

in which $n$ denotes any number from 1 to 20, $u$ denotes zero or 1, and $R_4$ denotes an unsubstituted or substituted aliphatic radical or an alkylaryl radical having from 6 to 20 carbon atoms, by reacting epichlorohydrin tetramer of the formula (III) with methylethanolamine, then adding $n$ mols of ethylene oxide onto the product obtained, and finally esterifying the OH groups with an acid of the formula $R_4COOH$.

22. Process for the preparation of a surface-active cyclic polyether of the formula I in which A denotes:

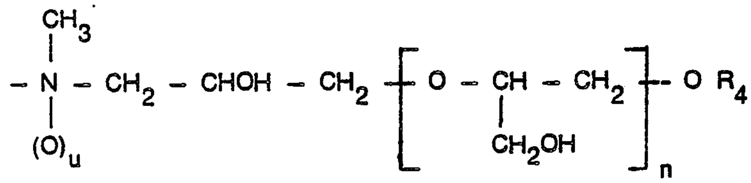

$(a_2)$

in which *n, u* and $R_4$ have the meanings indicated in Claim 10, characterised in that (1) epichlorohydrin tetramer of the formula (III) is reacted with methylamine, (2) a compound of the formula:

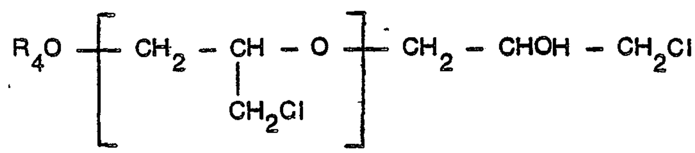

is condensed with the resulting product, and (3) the chlorine atoms are replaced in the resulting compound by hydroxyl groups with the aid of sodium acetate or potassium acetate, this being followed by hydrolysis or alcoholysis of the acetic acid ester formed.

23. Process for the preparation of a surface-active cyclic polyether of the formula (I) in which A denotes the group:

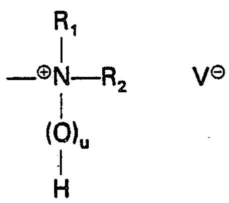

(b) or

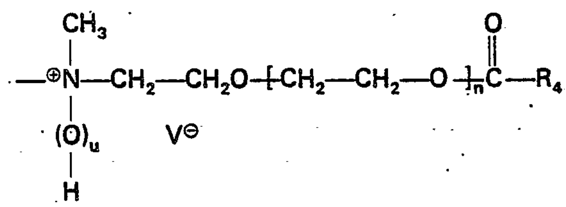

$(b_1)$ or

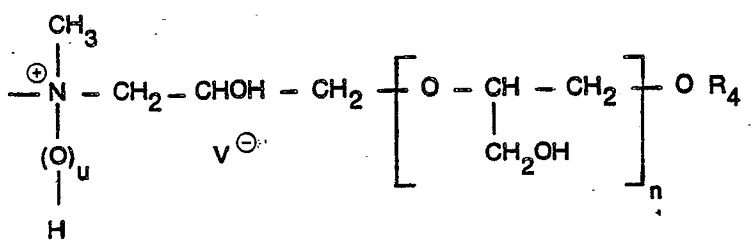

$(b_2)$

in which V⊖ denotes an anion, and preferably a formate, acetate, citrate or lactate, and *u*, $R_1$, $R_2$, $R_4$ and *n* have the meanings indicated in Claim 10, by salifying a compound of the formula (I) in which A respectively denotes the group:

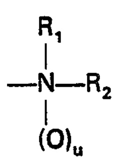

(a) or

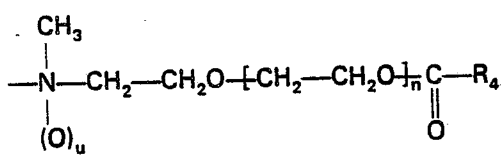

$(a_1)$ or

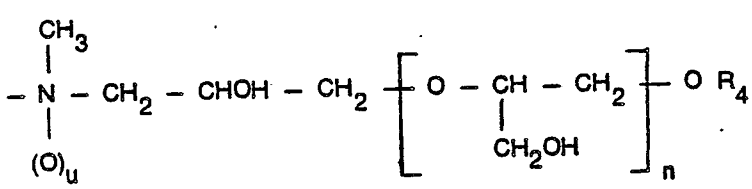

$(a_2)$

with an organic acid, and preferably with formic, citric, lactic or tartaric acid.

24. Process for the preparation of a surface-active cyclic polyether of the formula (I) in which A denotes the group:

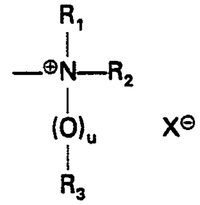 (c) or

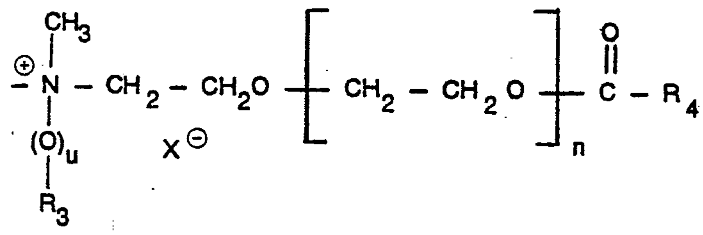 $(c_1)$

or 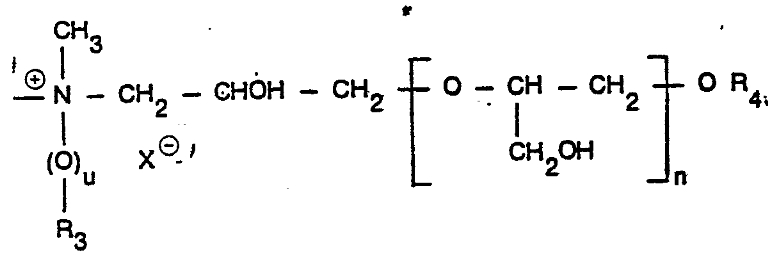 $(c_2)$

in which $R_3$ denotes an alkyl or hydroxyalkyl radical having from 1 to 3 carbon atoms, and preferably a methyl, ethyl, hydroxyethyl or dihydroxypropyl radical or a benzyl radical, $X^\ominus$ denotes an anion, and preferably a chloride, bromide, iodide, monomethyl-sulphate, methylsulphonate or p-toluenesulphonate, and *u*, $R_1$, $R_2$, $R_4$ and *n* have the meanings indicated in Claim 10, by alkylating a compound of the formula (I) in which A respectively denotes the group:

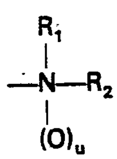 (a) or 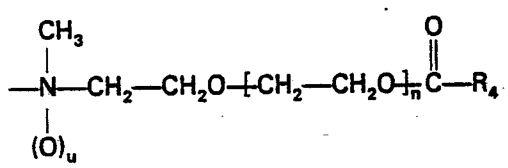 $(a_1)$ or

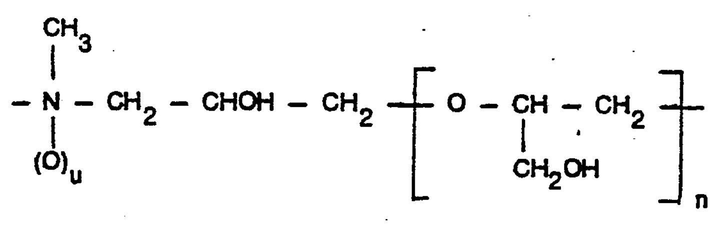 $(a_2)$

with an alkylating agent $XR_3$, in which X and $R_3$ have the above meaning.

25. Process for the preparation of a surface-active cyclic polyether of the formula I in which A denotes the group:

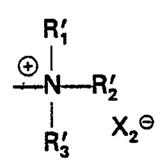 (c')

in which $R'_1$ and $R'_2$ denote an alkyl or hydroxyalkyl radical having from 1 to 3 carbon atoms, $R'_3$ denotes an alkyl radical having from 8 to 20 carbon atoms, and $X_2^\ominus$ denotes a mesylate or tosylate group, by reacting epichlorohydrin tetramer of the formula III with a secondary amine of the formula:

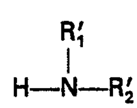

in which $R_1'$ and $R_2'$ have the meanings indicated above, this being followed by alkylation with a mesylate or tosylate of an alcohol having from 8 to 20 carbon atoms or of a mixture of such alcohols.

26. Process for the preparation of a surface-active cyclic polyether of the formula (I) in which A denotes the group:

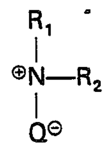 (d) 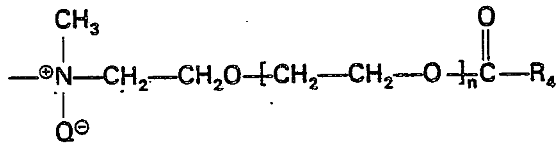 ($d_1$) or

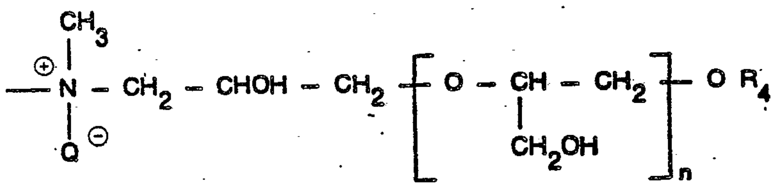 ($d_2$)

in which $Q^{\ominus}$ denotes one of the following groups:

$-(CH_2)_m COO^-$, in which *m* denotes 1, 2 or 3

$-CH_2-CH_2-CH_2-SO_3^-$

$-CH_2-CH_2OSO_2^-$

and $R_1$, $R_2$, $R_4$ and *n* have the meanings indicated in Claim 10, by alkylating a compound of the formula (I) in which A respectively denotes the group:

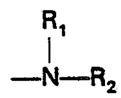 (a′) or

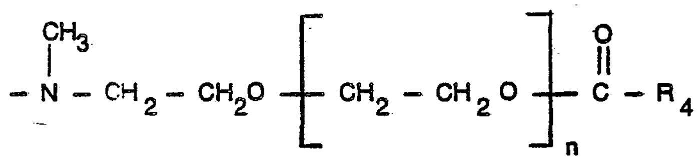 ($a_1'$) or

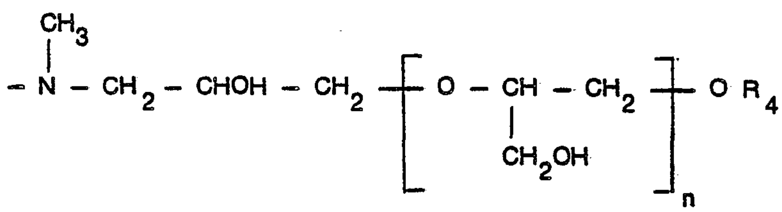 ($a_2'$)

$R_1$, $R_2$, $R_4$ and *n* having the meanings indicated above, with one of the acids of the formulae:

$Cl(CH_2)_m COOH$; $Br(CH_2)_m COOH$, in which *m* denotes 1, 2 or 3

$ClCH_2-CH_2-CH_2-SO_3H$; $BrCH_2CH_2CH_2-SO_3H$

or with the sodium or potassium salt of such an acid, or with propane-sultone or glycol sulphite.

27. Process for the preparation of a surface-active cyclic polyether of the formula (I) in which A denotes

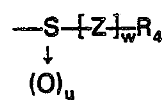 (e)

in which Z denotes groups of atoms of hydrophilic character, present in the form of a radical containing one or more ether, ester, amide, amine, ammonium and/or hydroxyl groups, $R_4$ has the meaning indicated in Claim 10, *u* denotes zero or 1, and *w* denotes zero or 1, characterised in that, in the first

stage, a reactant chosen from the group comprising alkylmercaptans, hydroxyalkylmercaptans, alkylmonoethoxymercaptans or alkylpolyethoxymercaptans, and the methyl or ethyl esters of a mercaptoacetic or mercaptopropionic acid is reacted with epichlorohydrin tetramer of the formula (III), at a temperature between 80 and 150°C, in the presence of an alkaline compound chosen from the group comprising sodium hydroxide, potassium hydroxide, sodium methylate or ethylate or potassium methylate or ethylate, and in that, if appropriate, in a second stage, the ester group obtained in the first stage is converted to the corresponding acid group by saponification or hydrolysis, or to a different ester by transesterification, or to an amide group by reaction with a primary amine or a secondary-tertiary diamine, and in that, if appropriate, the thioether group is oxidised to sulphoxide by means of hydrogen peroxide, and/or the thioether or sulphoxide group is converted to a sulphonium or sulphoxonium group, and/or the tertiary amine group is converted to an ammonium group by alkylation with an alkylating agent.

28. Process according to Claim 27, characterised in that Z is in the form of a radical containing one or more ether and/or hydroxyl groups, and in that, in the first stage, a reactant chosen from amongst alkylmercaptans, hydroxyalkylmercaptans, alkylmonoethoxymercaptans and alkylpolyethoxymercaptans is reacted with epichlorohydrin tetramer of the formula (III).

29. Process according to Claim 27, characterised in that *w* denotes 1, and Z denotes the group:

$-(CH_2)_x-COO-$ or $-(CH_2)_x-CONH-$ or 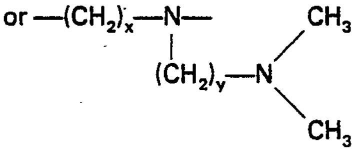

in which *x* denotes 1 or 2, and y denotes 2 or 3, in that, in a first stage, an intermediate of the formula:

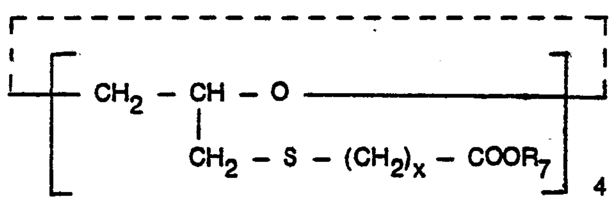

(IV)

in which *x* denotes 1 or 2, and $R_7$ denotes $CH_3$ or $C_2H_5$, is prepared by reacting epichlorohydrin tetramer of the formula (III) with an ester of the formula:

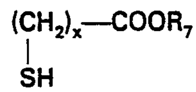

in which x and $R_7$ have the meanings indicated above, and in that, in a second stage, this intermediate of the formula (IV) is reacted with an alcohol of the formula $R_4OH$, or with a primary amine of the formula $R_4NH_2$, or with a secondary-tertiary diamine of the formula:

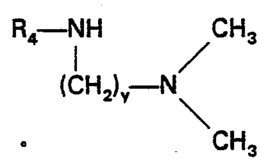

in which $R_4$ has the meaning indicated in Claim 10 and y denotes 2 or 3.

30. Process according to Claims 27 and 29, characterised in that w denotes 1, and Z denotes the group:

$$-(CH_2)_x-COO-CH_2-CHOH$$

in which x denotes 1 or 2, in that, in a first stage, an intermediate of the formula:

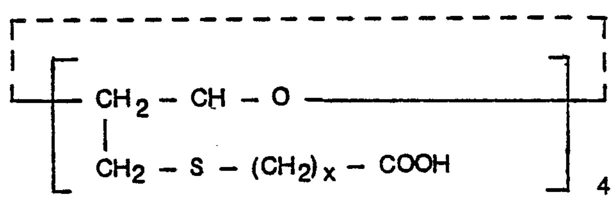

is prepared by saponifying the compound of the formula (IV), and in that, in a second stage, a 1,2-epoxyalkane of the formula:

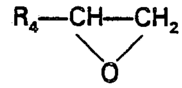

in which $R_4$ has the meaning indicated in Claim 10, is reacted with the intermediate of the formula (IVA).

31. Process according to Claim 27, characterised in that *w* denotes 1, and Z denotes the group:

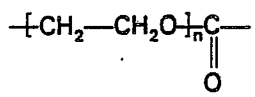

in which *n* denotes any number from 1 to 20, in that, in a first stage, thioethanol is condensed with epichlorohydrin tetramer of the formula (III), in that, in a second stage, 1 to 20 mols of ethylene oxide are added onto the compound obtained in the first stage, and in that, in a third stage, the compound obtained in the second stage is esterified by an acid of the formula $R_4$—COOH, in which $R_4$ has the meaning indicated in Claim 10.

32. Process for the preparation of a surface-active cyclic polyether of the formula (I) in which A denotes:

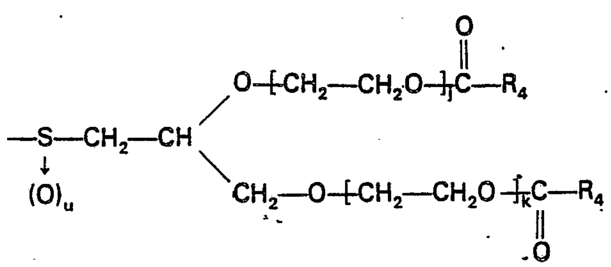

($e_1$)

in which j and k each denote any number less than 20, the sum j + k is between 1 and 20, *u* denotes 0 or 1, and $R_4$ has the meaning indicated in Claim 10, characterised in that, in a first stage, thioglycerol is condensed with epichlorohydrin tetramer of the formula (III), in that, in a second stage, the compound obtained in the first stage is oxyethylenated, in that, in a third stage, esterification is carried out with an acid of the formula $R_4COOH$, and in that, if appropriate, in a fourth stage, the thioether group is oxidised to sulphoxide with hydrogen peroxide, and/or the thioether or sulphoxide group is converted to a sulphonium or sulphoxonium group with an alkylating agent, and preferably with methyl bromide or iodide or dimethyl sulphate.

33. Process for the preparation of a surface-active cyclic polyether of the formula (I) in which A denotes:

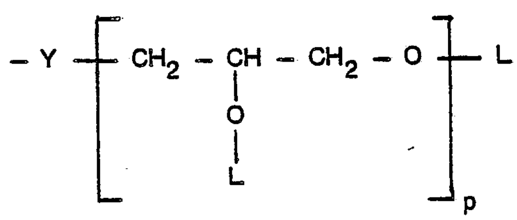

(f)

in which L simultaneously denotes a hydrogen atom and one of the groups:

$—CH_2—CHOH—CH_2—(O)_q—R_5$ or 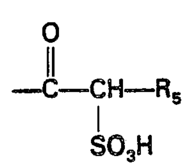 or 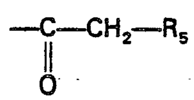

($R_5$ being a linear alkyl radical having from 6 to 18 carbon atoms, and q denoting zero or 1), the hydrogen atom and the other group being randomly distributed, Y denotes oxygen and p denotes an integer or decimal number from 1 to 10 and represents an average statistical value, characterised in

that, in a first stage, glycidol is reacted, under alkaline catalysis, with the hydroxyl derivative of epichlorohydrin tetramer, of the formula (II):

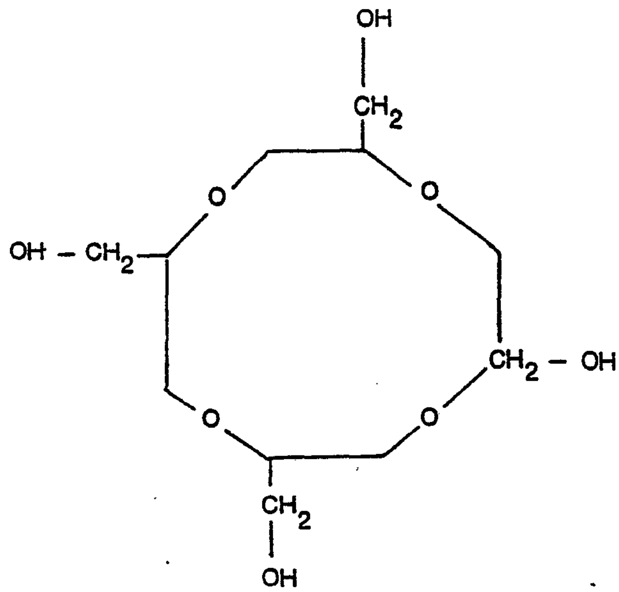

(II)

and in that, in a second stage, the compound obtained in the first stage is reacted with an alkylene oxide of the formula

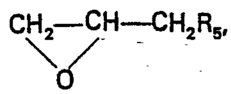

or with an alkyl glycidyl ether of the formula

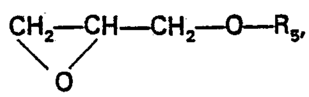

in which $R_5$ has the meaning indicated above.

34. Process for the preparation of a surface-active cyclic polyether of the formula (I) in which A denotes:

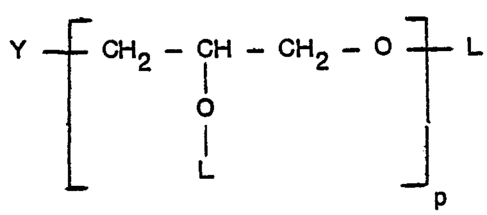

(f)

in which L simultaneously denotes a hydrogen atom and one of the groups:

$-CH_2-CHOH-CH_2-(O)_q-R_5$ or 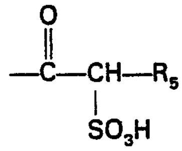 or 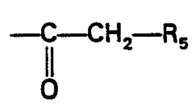

($R_5$ being a linear alkyl radical having from 6 to 18 carbon atoms, and q denoting zero or 1), the hydrogen atom and the other group being randomly distributed, Y denotes

—S— or 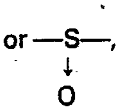

and p denotes an integer or decimal number from 1 to 10 and represents an average statistical value, characterised in that the reaction is carried out in one or more stage, in that, in a first stage, an intermediate of the formula:

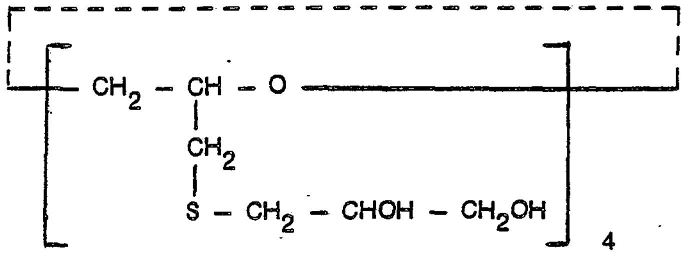

(IX)

is prepared by reacting epichlorohydrin tetramer of the formula (III) with thioglycerol, in the presence of a solvent and an alkaline compound, in that 0 to 9 mols of glycidol and/or of an alkylene oxide having 8 to 20 carbon atoms, and/or of an alkyl glycidyl ether are then reacted with this intermediate of the formula (IX), also in the presence of an alkaline compound, and in that, if appropriate, the hydroxyl groups are then esterified with a carboxylic or $\alpha$-sulphocarboxylic acid having from 8 to 20 carbon atoms, and/or the thioether group is oxidised to a sulphoxide group with hydrogen peroxide, and/or the thioether or sulphoxide group is alkylated to a sulphonium or sulphoxonium group with a known alkylating agent, and preferably with methyl bromide or iodine or dimethyl sulphate.

35. Process for the preparation of a surface-active cyclic polyether of the formula (I) in which A denotes the group:

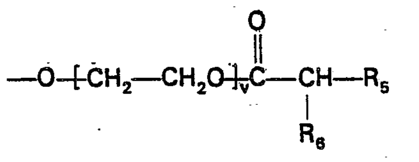

(g)

in which v denotes any number which is equal to or less than 20, except for the number zero, $R_5$ denotes a linear alkyl radical having from 6 to 18 carbon atoms, and $R_6$ denotes H or $SO_3H$, characterised in that, in a first stage, four times *v* mols of ethylene oxide are reacted with the hydroxyl derivative of epichlorohydrin tetramer, of the formula (II), and in that, in a second stage, the hydroxyl groups are esterified with a sulphocarboxylic acid of the formula:

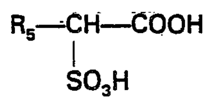

or with a carboxylic acid of the formula:

$R_5$—$CH_2$—COOH

36. Process for the preparation of a surface-active cyclic polyether of the formula (I) in which A denotes the group:

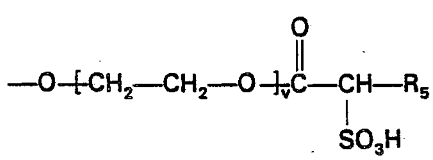

(g)

in which *v* denotes zero and $R_5$ has the meaning indicated in Claim 19, characterised in that the hydroxyl groups of the hydroxyl derivative of epichlorohydrin tetramer, of the formula (II), are esterified with a sulphocarboxylic acid of the formula:

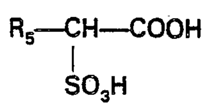

**Patentansprüche**

1. Kosmetisches oder pharmazeutisches Mittel, dadurch gekennzeichnet, daß es in Gegenwart eines geeigneten Trägers mindestens eine oberflächenaktive Verbindung der allgemeinen Formel enthält:

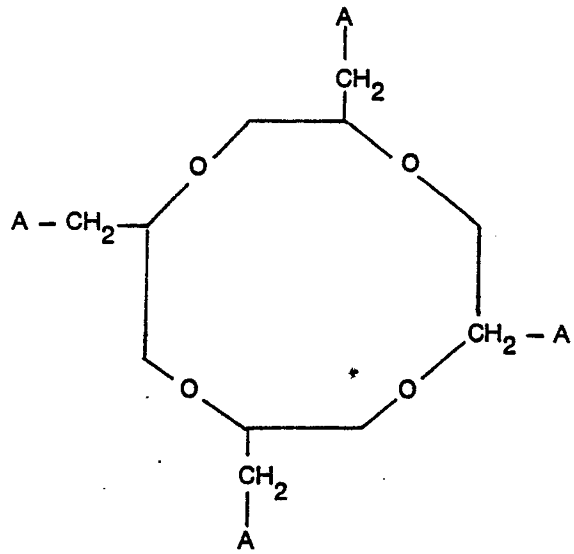

(I)

worin
A eine amphiphile Einheit darstellt, die aus einem hydrophilen und einem lipophilen Teil besteht, wobei
diese amphiphile Einheit über den hydrophilen Teil kovalent mit dem Ring verbunden ist;
der hydrophile Teil eine oder mehrere Amin-, Aminoxyd-, Ammonium-, Ammonioalkanoat-, Ammonioalkylsulfonat-, Ammonioalkylsulfinat-, Thioäther, Sulfoxyd-, Sulfonium-, Sulfoxonium-, Äther-, Hydroxyl-, Ester-, Amid- und Säuregruppen aufweist;
und
der lipophile Teil aus ein oder mehreren Gruppen besteht, ausgewählt unter (i) aliphatischen Gruppen mit 1 bis 20 Kohlenstoffatomen, (ii) substituierten aliphatischen Gruppen mit 6 bis 20 Kohlenstoffatomen, und (iii) Alkylarylgruppen mit 6 bis 20 Kohlenstoffatomen, worin der Alkylteil der Alkylarylgruppe bis zu 14 Kohlenstoffatome besitzt.

2. Mittel nach Anspruch 1, dadurch gekennzeichnet, daß die amphiphile Gruppe A ausgewählt ist unter:

a) der Gruppe:

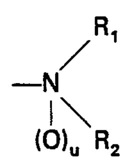

worin:
$R_1$ und $R_2$, die gleich oder verschieden sein können, aliphatische Reste mit 1 bis 20 Kohlenstoffatomen, wobei einer der Reste mindestens 8 Kohlenstoffatome aufweist, oder
substituierte aliphatische Reste mit 6 bis 20 Kohlenstoffatomen, oder
Alkylarylreste mit 6 bis 20 Kohlenstoffatomen bedeuten,
wobei die Summe der Kohlenstoffatome von $R_1$ und $R_2$ vorzugsweise kleiner oder gleich 28 ist und einer der Reste außerdem einen Dimethylamino-äthyl- oder -propyl-, Diäthylamino-äthyl- oder -propyl-, Piperidino-äthyl- oder -propyl-, Morpholino-äthyl- oder -propyl- oder Alkylpolyhydroxypropylen-Rest bedeuten kann, und
u für Null oder 1 steht;

$a_1$) der Gruppe

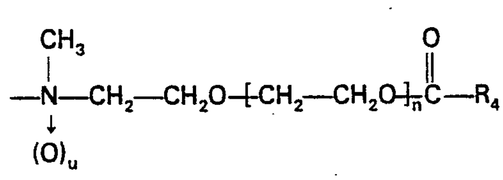

worin
$R_4$ einen nicht-substituierten oder substituierten, aliphatischen Rest oder einen Alkylarylrest mit 6 bis 20 Kohlenstoffatomen und vorzugsweise einen Alkyl-, Hydroxyalkyl-, Alkenyl-, oder Alkylphenyl-Rest

bedeutet, wobei der Alkylteil des Alkylphenylrestes bis zu 14 Kohlenstoffatome besitzt,
n irgend eine Zahl von 1 bis 20 bedeutet,
und
u für Null oder 1 steht;

$a_2$) der Gruppe:

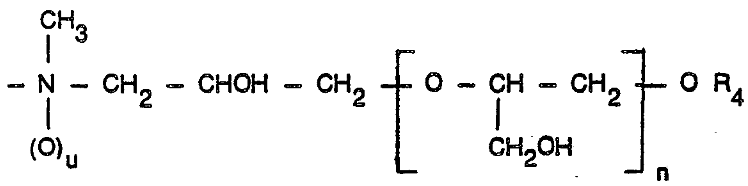

worin u, $R_4$ und n die oben genannte Bedeutung besitzen;

b) der Gruppe:

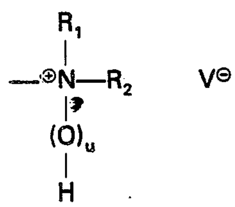

$b_1$) der Gruppe:

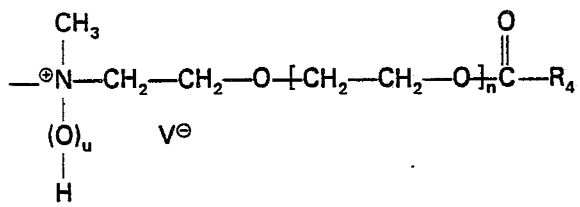

$b_2$) der Gruppe:

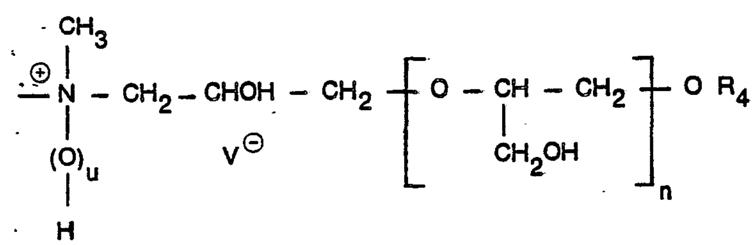

wobei $V^{\ominus}$ in den Gruppen (b), ($b_1$) und ($b_2$) für ein Anion und vorzugsweise für ein Formiat, Acetat, Citrat oder Lactatanion steht und u, n, $R_1$, $R_2$ und $R_4$ die oben bei den Gruppen a und $a_1$ angegebenen Bedeutungen besitzen;

c) der Gruppe:

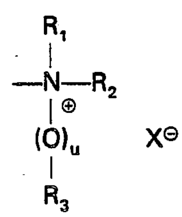

worin $R_1$, $R_2$ und u die bei der Gruppe ($a_1$) angegebene Bedeutung besitzen,
$R_3$ einen Alkyl- oder Hydroxyalkylrest mit 1 bis 3 Kohlenstoffatomen und vorzugsweise einen Methyl-, Äthyl-, Hydroxyäthyl-, Dihydroxypropyl- oder einen Benzyl-Rest bedeutet,
$X^{\ominus}$ ein Anion und vorzugsweise ein Chlorid, Bromid, Jodid, Monomethylsulfat, Methylsulfonat und p-Toluolsulfonat bedeutet;

$c_1$) der Gruppe:

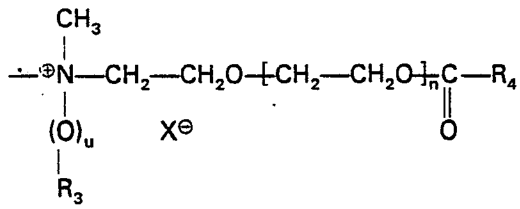

$c_2$) der Gruppe:

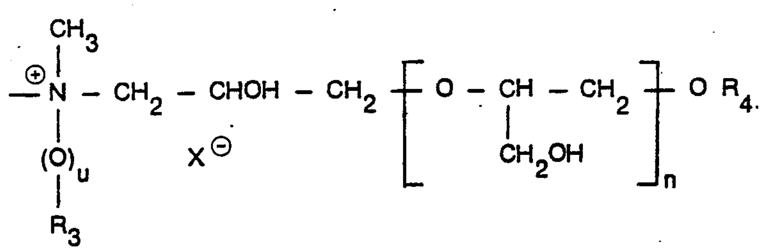

wobei in diesen Gruppen ($c_1$) und ($c_2$)
u für Null oder 1 steht,
n irgendeine Zahl von 1 bis 20 bedeutet,
$R_4$ die bei der Gruppe ($a_1$) angegebenen Bedeutungen besitzt und
$R_3$ und $Z^{\ominus}$ die bei der Gruppe (c) angegebenen Bedeutungen besitzen;

d) der Gruppe:

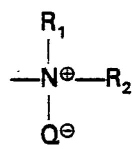

worin $Q^{\ominus}$ für eine der folgenden Gruppen steht:

$-(CH_2)_m-COO^{\ominus}$ mit m = 1, 2 oder 3
$-CH_2-CH_2-CH_2-SO_3^{\ominus}$
$-CH_2-CH_2-O-SO_2^{\ominus}$

und
$R_1$ und $R_2$ die bei der Gruppe (a) angegebene Bedeutung besitzen;

$d_1$) der Gruppe:

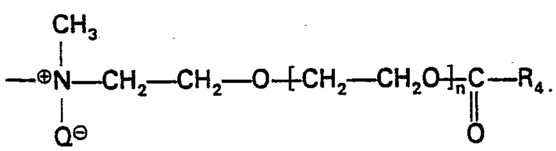

$d_2$) der Gruppe:

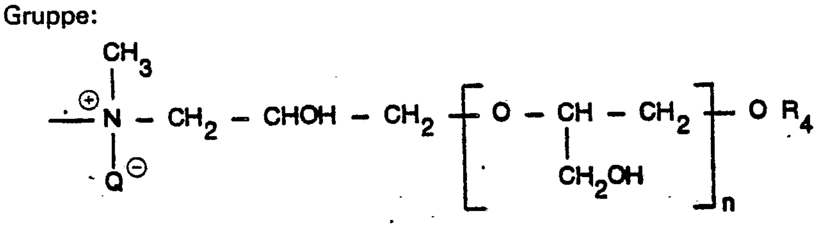

wobei n in den Gruppen ($d_1$) und ($d_2$) irgendeine Zahl zwischen 1 und 20 bedeutet,
$Q^{\ominus}$ die bei der Gruppe (d) gegebene Bedeutung besitzt und
$R_4$ die bei der Gruppe ($a_1$) gegebene Bedeutung besitzt;

e) der Gruppe:

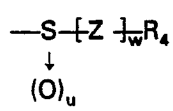

worin

$R_4$ die bei der Gruppe $a_1$ gegebene Bedeutung besitzt,
u für Null oder 1 steht,
w für Null oder 1 steht und
Z Atomgruppen mit hydrophilem Charakter bedeutet, die eine Äther-, Ester-, Amid-, Amin-, Ammonium- und/oder Hydroxyl-Gruppe sein können;

$e_1$) der Gruppe:

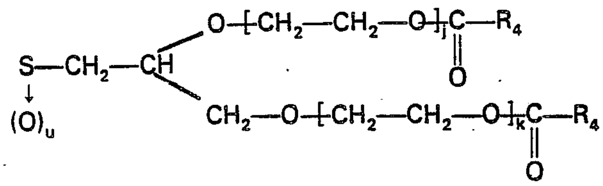

worin
j und k jeweils irgendeine Zahl kleiner 20 bedeuten, die Summe von j + k zwischen 1 und 20 beträgt,
u für Null oder 1 steht
und
$R_4$ die bei der Gruppe $a_1$) angegebene Bedeutung besitzt;

f) der Gruppe:

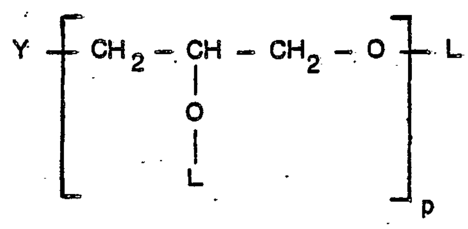

worin
L gleichzeitig ein Wasserstoffatom und eine der Gruppen bedeutet:

$—CH_2—CHOH—CH_2—(O)_q—R_5$ mit $q = 0$ oder 1

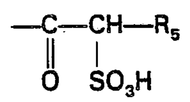 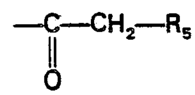

wobei das Wasserstoffatom und die andere Gruppe statistisch verteilt sind,
$R_5$ einen linearen Alkylrest mit 6 bis 18 Kohlenstoffatomen bedeutet,
p irgendeine Zahl von 1 bis 10 bedeutet und einen statistischen Mittelwert darstellt, und
Y bedeutet: —O—; —S— oder 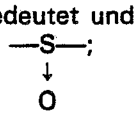;

g) der Gruppe:

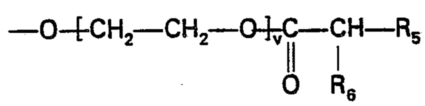

worin
v irgendeine Zahl zwischen Null und 20 bedeutet,
$R_6$ ein Wasserstoffatom oder einen $—SO_3H$-Rest bedeutet, und
$R_5$ die oben angegebene Bedeutung besitzt;

h) der Gruppe (e) oder ($e_1$), die alkyliert ist mit einem Alkylierungsmittel ausgewählt unter: Methylbromid, -jodid und -sulfat und vorzugsweise Dimethylsulfat;
und

i) der Gruppe (f), die alkyliert ist mit einem Alkylierungsmittel ausgewählt unter Methyl-bromid, -jodid, und -sulfat.

3. Mittel nach einem der Ansprüche 1 und 2, dadurch gekennzeichnet, daß der Träger ein wäßriger oder wäßrigalkoholischer Träger ist.

4. Kosmetisches Mittel zur Haarpflege, das, gelöst in einem Lösungsmittel ausgewählt unter Wasser und einer wäßrig-alkoholischen Lösung, eine kosmetisch wirksame Menge einer oder mehrerer der Verbindungen der Formel (I) nach Anspruch 1 oder 2 enthält.

5. Shampoomittel zur Haarpflege, das, gelöst in einem Lösungsmittel ausgewählt unter Wasser und einer wäßrig-alkoholischen Lösung, eine kosmetisch wirksame Menge einer oder mehrerer der Verbindungen der Formel (I) nach Anspruch 1 oder 2 enthält.

6. Kosmetisches Mittel zur Haarpflege nach Anspruch 3, das, gelöst in einem Lösungsmittel ausgewählt unter Wasser und einer wäßrig-alkoholischen Lösung, eine kosmetisch wirksame Menge einer oder mehrerer der Verbindungen der Formel (I) und zusätzlich eines oder mehrere Adjuvantien enthält, wobei die Adjuvantien ausgewählt sind unter anionischen, kationischen, amphoteren, zwitterionischen und nicht-ionischen oberflächenaktiven Mitteln; Parfums; Farbstoffen; Konservierungsmitteln; Verdickungsmitteln; schäumenden Mitteln; schaumstabilisierenden Mitteln; weichmachenden Mitteln; Haare restrukturierenden Mitteln; Anti-Schuppenmitteln, kosmetischen Harzen; Schaumsynergisten und Elektrolyten.

7. Verfahren zur Haarpflege, das darin besteht, daß man auf menschliche Haare eine wirksame Menge eines Mittels aufträgt, das, gelöst in einem Lösungsmittel, ausgewählt unter Wasser und einer wäßrig-alkoholischen Lösung, eine kosmetisch wirksame Menge eines oder mehrerer der oberflächenaktiven cyclischen Polyäther der Formel (I) nach Anspruch 1 oder 2 enthält.

8. Haarfärbemittel, das, gelöst in einem Lösungsmittel ausgewählt unter Wasser und einer wäßrig-alkoholischen Lösung, eine kosmetisch wirksame Menge einer oder mehrerer der Verbindungen der Formel (I) nach Anspruch 1 oder 2 sowie einen Haarfarbstoff und gegebenenfalls ein oder mehrere Adjuvantien enthält, wobei die Adjuvantien ausgewählt sind unter anionischen, kationischen, nicht-ionischen, zwitterionischen, amphoteren oberflächenaktiven Mitteln und ihren Mischungen; Parfums; Verdickungsmitteln; weichmachenden Mitteln; kosmetischen Harzen; und Sequestrierungsmitteln.

9. Mittel nach Anspruch 8, dadurch gekennzeichnet, daß der Farbstoff ausgewählt ist unter Anthrachinonfarbstoffen, Azofarbstoffen, nitrierten Farbstoffen der Benzolreihe, Indophenolen, Indoanilinen und Indaminen.

10. Oberflächenaktive cyclische Polyäther der allgemeinen Formel:

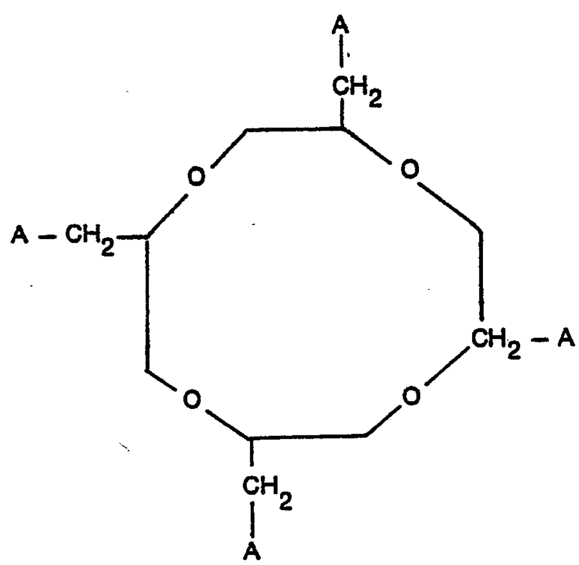

(I)

worin

A eine amphiphile Einheit bedeutet, die aus einem hydrophilen Teil und einem lipophilen Teil besteht, wobei diese amphiphile Einheit mit dem hydrophilen Teil kovalent am Ring gebunden ist und wobei der amphiphile Teil ein oder mehrere Gruppen aufweist, die ausgewählt sind unter:

a) der Gruppe:

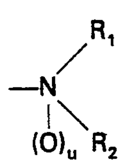

worin

$R_1$ und $R_2$, die gleich oder verschieden sein können, aliphatische Reste mit 1 bis 20 Kohlenstoffatomen, wobei einer dieser Reste mindestens 8 Kohlenstoffatome aufweist, oder substituierte aliphatische Reste mit 6 bis 20 Kohlenstoffatomen oder Alkylarylreste mit 6 bis 20 Kohlenstoffatomen bedeuten, wobei die Summe der Kohlenstoffatome von $R_1$ und $R_2$ vorzugsweise kleiner oder gleich 28 ist, und einer der Reste außerdem einen Dimethylaminoäthyl- oder -propyl-, Diäthylaminoäthyl- oder -propyl,

Piperidino -äthyl- oder -propyl-, Morpholinoäthyl- oder -propyl- oder Alkylpolyhydroxypropylen-Rest bedeuten kann,
und
u für Null oder 1 steht;

$a_1$) der Gruppe

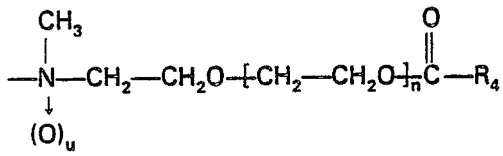

worin
$R_4$ einen nicht-substituierten oder substituierten, aliphatischen Rest oder einen Alkylarylrest mit 6 bis 20 Kohlenstoffatomen und vorzugsweise einen Alkyl-, Hydroxyalkyl-, Alkenyl-, oder Alkylphenyl-Rest bedeutet, wobei der Alkylteil des Alkylphenylrestes bis zu 14 Kohlenstoffatome besitzt,
n irgend eine Zahl von 1 bis 20 bedeutet, und
u für Null oder 1 steht;

$a_2$) der Gruppe:

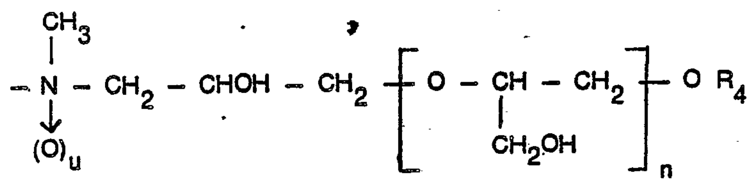

worin u, $R_4$ und n die oben genannte Bedeutung besitzen;

b) der Gruppe:

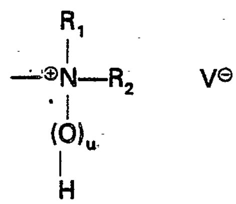

$b_1$) der Gruppe:

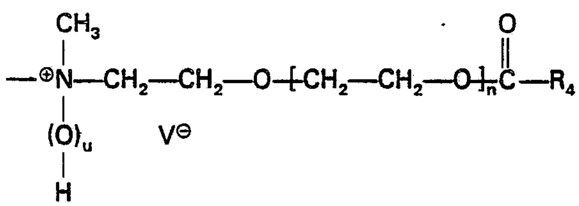

$b_2$) der Gruppe:

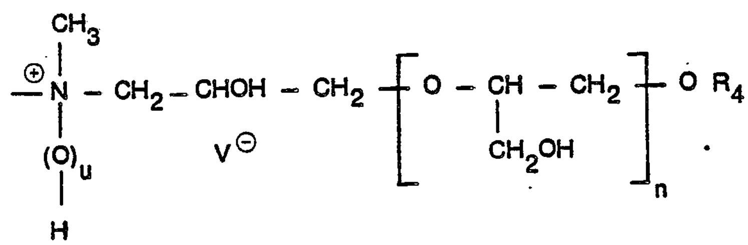

wobei $V^{\ominus}$ in den Gruppen (b), ($b_1$) und ($b_2$) für ein Anion und vorzugsweise für ein Formiat, Acetat, Citrat oder Lactatanion steht und u, n, $R_1$, $R_2$ und $R_4$ die oben bei den Gruppen a und $a_1$ angegebenen Bedeutungen besitzen;

c) der Gruppe:

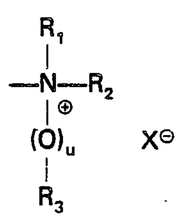

worin $R_1$, $R_2$ und u die bei der Gruppe ($a_1$) angegebene Bedeutung besitzen, $R_3$ einen Alkyl- oder Hydroxyalkylrest mit 1 bis 3 Kohlenstoffatomen und vorzugsweise einen Methyl-, Äthyl-, Hydroxyäthyl-, Dihydroxypropyl- oder einen Benzyl-Rest bedeutet, $X^{\ominus}$ ein Anion und vorzugsweise ein Chlorid, Bromid, Jodid, Monomethylsulfat, Methylsulfonat und p-Toluolsulfonat bedeutet;

$c_1$) der Gruppe:

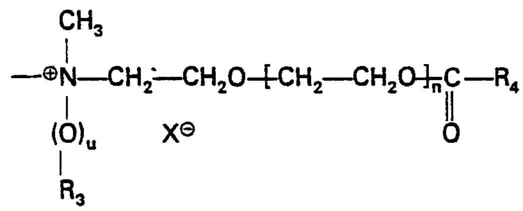

$c_2$) der Gruppe:

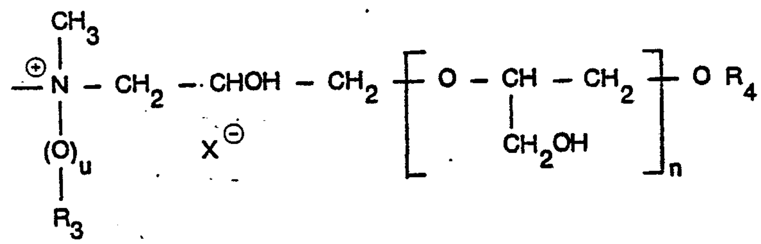

wobei in diesen Gruppen ($c_1$) und ($c_2$)
u für Null oder 1 steht,
n irgendeine Zahl von 1 bis 20 bedeutet,
$R_4$ die bei der Gruppe ($a_1$) angegebenen Bedeutungen besitzt und
$R_3$ und $X^{\ominus}$ die bei der Gruppe (c) angegebenen Bedeutungen besitzen;

d) der Gruppe:

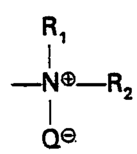

worin $Q^{\ominus}$ für eine der folgenden Gruppen steht:

$-(CH_2)_m-COO^{\ominus}$ mit m = 1, 2 oder 3

$-CH_2-CH_2-CH_2-SO_3^{\ominus}$

$-CH_2-CH_2-O-SO_2^{\ominus}$

und
$R_1$ und $R_2$ die bei der Gruppe (a) angegebene Bedeutung besitzen;

$d_1$) der Gruppe:

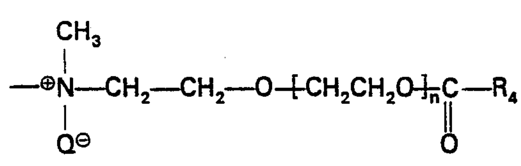

$d_2$) der Gruppe:

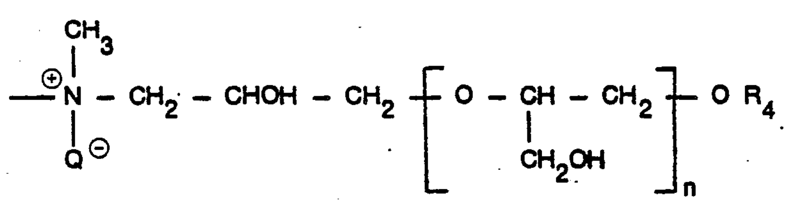

wobei n in den Gruppen ($d_1$) und ($d_2$) irgendeine Zahl zwischen 1 und 20 bedeutet,

$Q^{\ominus}$ die bei der Gruppe (d) gegebene Bedeutung besitzt und $R_4$ die bei der Gruppe ($a_1$) gegebene Bedeutung besitzt;

e) der Gruppe:

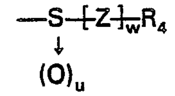

worin
$R_4$ die bei der Gruppe $a_1$ gegebene Bedeutung besitzt,
u für Null oder 1 steht,
w für Null oder 1 steht und
Z Atomgruppen mit hydrophilem Charakter bedeutet, die eine Äther-, Ester-, Amid-, Amin-, Ammonium- und/oder Hydroxyl-Gruppe sein können;

$e_1$) der Gruppe:

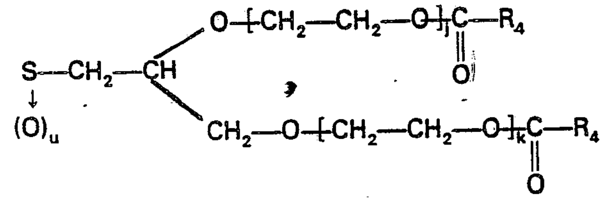

worin
j und k jeweils irgendeine Zahl kleiner 20 bedeuten, die Summe von j + k zwischen 1 und 20 beträgt,
u für Null oder 1 steht
und
$R_4$ die bei der Gruppe $a_1$) angegebene Bedeutung besitzt;

f) der Gruppe:

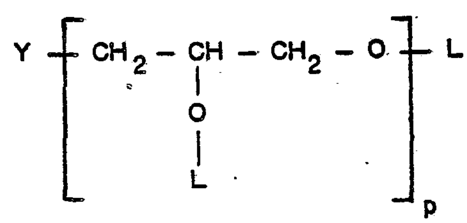

worin
L gleichzeitig ein Wasserstoffatom und eine der Gruppen bedeutet:

$$—CH_2—CHOH—CH_2—(O)_q—R_5 \text{ mit } q = 0 \text{ oder } 1$$

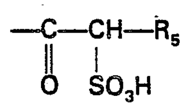 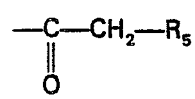

wobei das Wasserstoffatom und die andere Gruppe statistisch verteilt sind,
$R_5$ einen linearen Alkylrest mit 6 bis 18 Kohlenstoffatomen bedeutet,
p irgendeine Zahl von 1 bis 10 bedeutet und einen statistischen Mittelwert darstellt, und
Y bedeutet: —O—; —S— oder 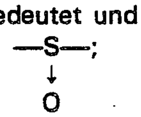;

g) der Gruppe:

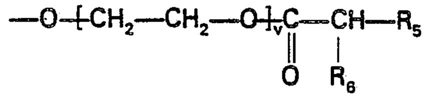

worin
v irgendeine Zahl zwischen Null und 20 bedeutet,
$R_6$ ein Wasserstoffatom oder einen $—SO_3H—$Rest bedeutet, und
$R_5$ die oben angegebene Bedeutung besitzt;

h) der Gruppe (3) oder ($e_1$), die alkyliert ist mit einem Alkylierungsmittel ausgewählt unter: Methyl-bromid, -jodid und -sulfat und vorzugsweise Dimethylsulfat; und

i) der Gruppe (f), die alkyliert ist mit einem Alkylierungsmittel ausgewählt unter Methylbromid, -jodid, und -sulfat.

11. Oberflächenaktiver cyclischer Polyäther nach Anspruch 10, dadurch gekennzeichnet, daß die amphiphile Gruppe A ausgewählt ist unter:

a) der Gruppe:

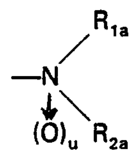

worin

$R_{1a}$ und $R_{2a}$, die gleich oder verschieden sein können, Alkyl-, Hydroxyalkyl- oder Alkenylreste mit 1 bis 20 Kohlenstoffatomen, wobei einer der Reste mindestens 8 Kohlenstoffatome aufweist, oder Alkylarylreste mit 6 bis 20 Kohlenstoffatomen bedeuten, wobei die Summe der Kohlenstoffatome von $R_{1a}$ und $R_{2a}$ vorzugsweise kleiner oder gleich 28 ist,

und

worin einer der Reste außerdem einen Dimethylamino-äthyl- oder -propyl-, Diäthylamino-äthyl- oder -propyl-, Piperidino-äthyl- oder -propyl, Morpholino-äthyl- oder -propyl- oder Alkylpolyhydroxypropylen-Rest bedeuten können

und

u für Null oder 1 steht;

$a_1$) der Gruppe:

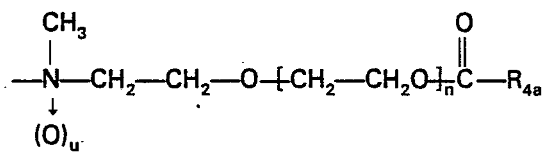

worin

$R_{4a}$ einen Alkyl-, Hydroxyalkyl-, Alkenyl- oder Alkylphenylrest mit 6 bis 20 Kohlenstoffatomen bedeutet, wobei der Alkylteil des Alkylphenylrestes bis zu 14 Kohlenstoffatome besitzt,

n für irgendeine Zahl zwischen 1 und 20 steht, und

u für Null oder 1 steht;

$a_2$) der Gruppe:

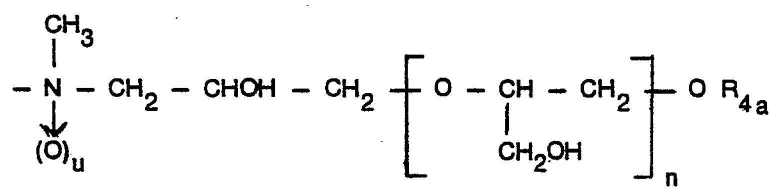

worin u, $R_{4a}$ und n die oben angegebene Bedeutung besitzen;

b) der Gruppe:

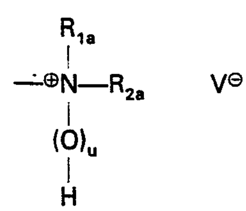

$b_1$) der Gruppe:

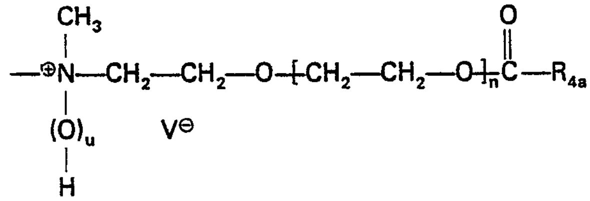

b$_2$) der Gruppe:

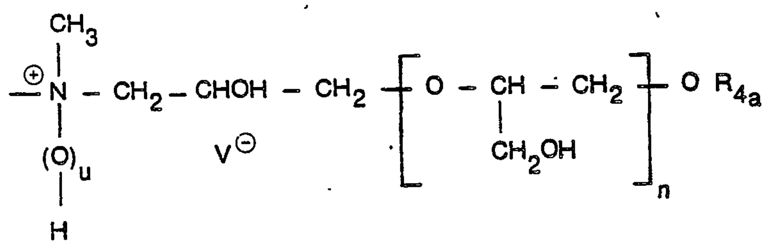

worin $V^{\ominus}$ in den Gruppen (b), (b$_1$) und (b$_2$) ein Anion und vorzugsweise ein Formiat-, Acetat-, Citrat- oder Lactat-Anion bedeutet, und
u, n, $R_{1a}$, $R_{2a}$ und $R_{4a}$ die bei den Gruppen a und a$_1$ angegebenen Bedeutungen besitzen;

c) der Gruppe:

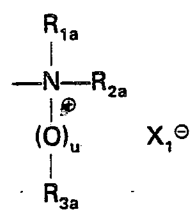

worin
$R_{1a}$, $R_{2a}$ und u die bei den Gruppe (a) angegebene Bedeutung besitzen,
$R_{3a}$ einen Methyl-, Äthyl-, Hydroxyäthyl-, Dihydroxypropyl- oder Benzyl-Rest bedeutet, und
$X_1^{\ominus}$ ein Chlorid-, Bromid-, Jodid-, Monomethylsulfat-, Methylsulfonat- oder p-Toluolsulfonat-Anion bedeutet;

c$_1$) der Gruppe:

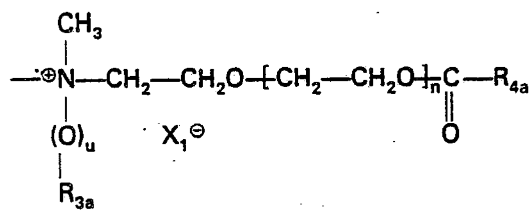

c$_2$) der Gruppe:

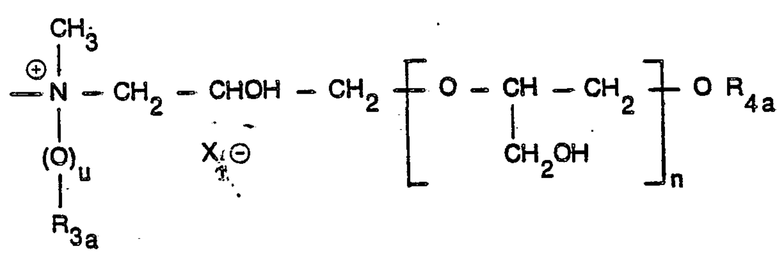

worin in diesen Gruppen (c$_1$) und (c$_2$)
u für Null oder 1 steht,
n für irgendeine Zahl zwischen 1 und 20 steht,
$R_{4a}$ die bei der Gruppe (a$_1$) angegebenen Bedeutungen besitzt, und
$R_3$ und $X_1^{\ominus}$ die bei der Gruppe (c) angegebenen Bedeutungen besitzen;

d) der Gruppe:

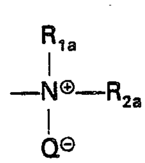

worin $Q^{\ominus}$ eine der folgenden Gruppen bedeutet:

$—(CH_2)_m—COO^{\ominus}$ mit m = 1, 2 oder 3

$—CH_2—CH_2—CH_2—SO_3^{\ominus}$

$—CH_2—CH_2—O—SO_2^{\ominus}$

$R_{1a}$ und $R_{2a}$ die bei der Gruppe (a) angegebene Bedeutung besitzen;

$d_1$) der Gruppe:

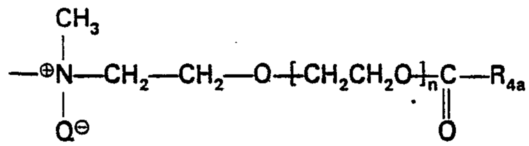

$d_2$) der Gruppe:

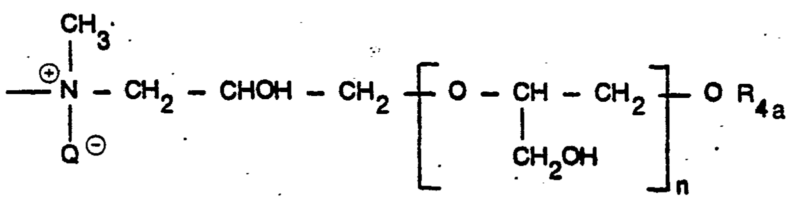

worin in den Gruppen ($d_1$) und ($d_2$)
n irgendeine Zahl zwischen 1 und 20 bedeutet,
$R_{4a}$ die bei der Gruppe ($a_1$) angegebenen Bedeutungen besitzt, und
$Q^{\ominus}$ die bei der Gruppe (d) angegebene Bedeutung besitzt;

e) der Gruppe:

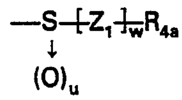

worin
$R_{4a}$ die bei der Gruppe ($a_1$) angegebenen Bedeutungen besitzt,
u für Null oder 1 steht,
w für Null oder 1 steht,
und
$Z_1$ für eine Atomgruppierung mit hydrophilem Charakter steht, ausgewählt unter:

$—CH_2—CHOH—$; $—(CH_2—CH_2O)_n—$; 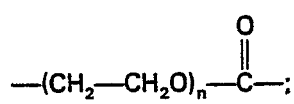

$—(CH_2)_x—COO—$; $—(CH_2)_x—COOCH_2—CHOH—$;

$—(CH_2)_x—CONH—$; 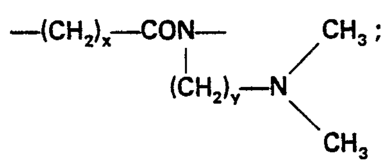

worin
x 1 oder 2 darstellt,
y 2 oder 3 darstellt,
und
n für irgendeine Zahl zwischen 1 und 20 steht;

$e_1$) der Gruppe:

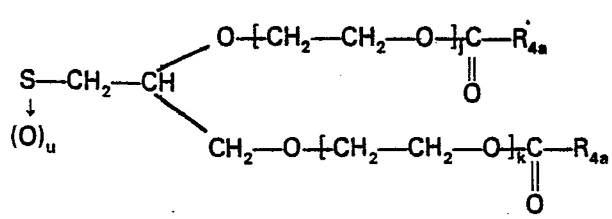

worin
j und k jeweils für irgendeine Zahl kleiner 20 stehen,
die Summe von j + k zwischen 1 und 20 beträgt,
u Null oder 1 bedeutet,
und
$R_{4a}$ die bei der Gruppe ($a_1$) angegebene Bedeutung besitzt;

f) der Gruppe:

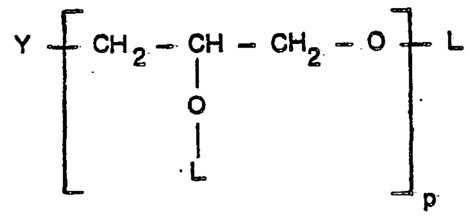

worin
L gleichzeitig ein Wasserstoffatom und eine der folgende Gruppen;

$—CH_2—CHOH—CH_2—(O)_q—R_5$ mit q = 0 oder 1

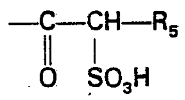

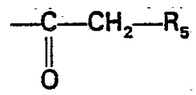

bedeutet,
wobei das Wasserstoffatom und die andere Gruppe statistisch verteilt sind,
$R_5$ einen linearen Alkylrest mit 6 bis 18 Kohlenstoffatomen darstellt,
p für irgendeine Zahl zwischen 1 und 10 steht und einen statistischen Mittelwert darstellt, und
Y für —O—, —S— oder 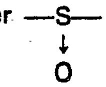 steht;

g) der Gruppe:

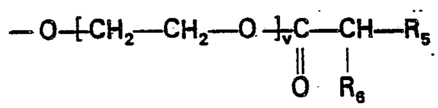

worin
v irgendeine Zahl zwischen Null und 20 darstellt,
$R_6$ ein Wasserstoffatom oder einen $—SO_3H$-Rest darstellt, und
$R_5$ die oben angegebene Bedeutung besitzt;

h) der Gruppe (e) oder ($e_1$), die alkyliert ist mit einem Alkylierungsmittel, ausgewählt unter Methyljodid, -bromid und -sulfat und vorzugsweise Dimethylsulfat,

i) der Gruppe (f) die alkyliert ist mit einem Alkylierungsmittel ausgewählt unter Methylbromid, Methyljodid und Dimethylsulfat.

12. Oberflächenaktiver cyclischer Polyäther nach Anspruch 11, dadurch gekennzeichnet, daß die amphiphile Gruppe die Gruppe (a)

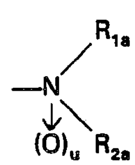

ist, worin
$R_{1a}$ und $R_{2a}$, die gleich oder verschieden sein können, Alkyl- oder Hydroxyalkylreste mit 1 bis 20 Kohlenstoffatomen bedeuten, wobei einer der Reste mindestens 8 Kohlenstoffatome aufweist,
die Summe der Kohlenstoffatome von $R_{1a}$ und $R_{2a}$ vorzugsweise kleiner oder gleich 28 ist,
einer der Reste außerdem einen Dimethylaminoäthyl- oder -propyl-, Diäthylamino-äthyl- oder -propyl-, Piperidino-äthyl- oder -propyl-, Morpholino-äthyl- oder -propyl- oder Alkylpolyhydroxypropylen-Rest bedeuten kann, und
u für Null oder 1 steht.

13. Oberflächenaktiver cyclischer Polyäther nach Anspruch 11, dadurch gekennzeichnet, daß die amphiphile Gruppe die Gruppe ($a_1$)

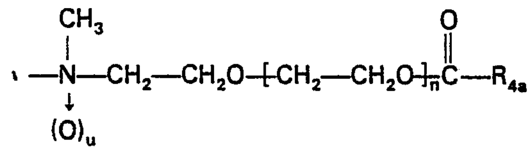

ist, worin
$R_{4a}$ einen Alkyl-, Hydroxyalkyl-, Alkenyl- oder Alkylphenylrest mit 6 bis 20 Kohlenstoffatomen bedeutet, wobei der Alkylteil des Alkylphenylrestes bis zu 14 Kohlenstoffatome besitzt,
n für irgendeine Zahl zwischen 1 und 20 steht, und
u für Null oder 1 steht.

14. Oberflächenaktiver cyclischer Polyäther nach Anspruch 11, dadurch gekennzeichnet, daß die amphiphile Gruppe A die Gruppe (b)

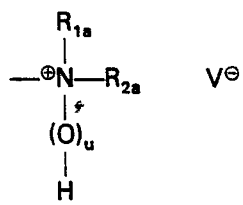

ist, worin
$R_{1a}$ und $R_{2a}$, die gleich oder verschieden sein können, Alkyl- oder Hydroxyalkylreste mit 1 bis 20 Kohlenstoff-atomen bedeuten, wobei einer dieser Reste mindestens 8 Kohlenstoffatome aufweist, die Summe der Kohlenstoffatome von $R_{1a}$ und
$R_{2a}$ kleiner oder gleich 28 ist und einer der Reste $R_{1a}$ oder $R_{2a}$ außerdem einen Rest bedeuten kann, der ausgewählt ist unter: Dimethylaminoäthyl-, Dimethylaminopropyl-, Diäthylaminoäthyl-, Diäthylaminopropyl-, Piperidinoäthyl-, Piperidinopropyl-, Morpholinoäthyl-, Morpholinopropyl- und Alkylpolyhydroxypropylen-Resten,
u für Null oder 1 steht, und
$V^\ominus$ ein Anion bedeutet, ausgewählt unter Formiat-, Acetat-, Citrat- und Lactat-Anionen.

15. Oberflächenaktiver cyclischer Polyäther nach Anspruch 11, dadurch gekennzeichnet, daß die amphiphile Gruppe die Gruppe (c)

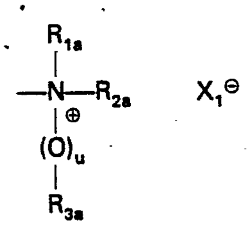

ist, worin
$R_{1a}$ und $R_{2a}$, die gleich oder verschieden sein können, Alkyl- oder Hydroxyalkyl-Reste mit 1 bis 20 Kohlenstoffatomen bedeuten, wobei einer der Reste mindestens 8 Kohlenstoffatome aufweist, die Summe der Kohlenstoffatome von $R_{1a}$ und $R_{2a}$ kleiner oder gleich 28 ist, und einer der Reste auch einen Rest darstellen kann, ausgewählt unter Dimethylaminoäthyl, Dimethylaminopropyl, Diäthylaminoäthyl, Diäthylaminopropyl, Piperidinoäthyl, Piperidinopropyl, Morpholinoäthyl, Morpholinopropyl und Alkylpolyhydroxypropylen,
$R_{3a}$ einen Methyl-, Äthyl-, Hydroxyäthyl-, Dihydroxypropyl- oder Benzylrest bedeutet,
$X_1^\ominus$ ein Anion darstellt, ausgewählt unter Chlorid, Bromid, Jodid, Monomethylsulfat, Methylsulfonat und p-Toluolsulfonat, und
u für Null oder 1 steht.

16. Oberflächenaktiver cyclischer Polyäther nach Anspruch 11, dadurch gekennzeichnet, daß die amphiphile Gruppe A die Gruppe ($c_2$)

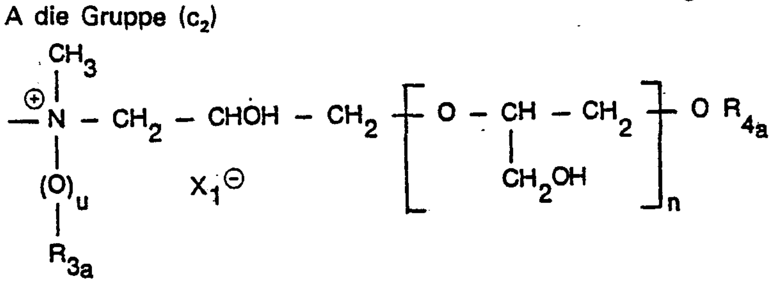

ist, worin
u für Null oder 1 steht,

n für irgendeine Zahl zwischen 1 und 20 steht, $R_{3a}$ einen Methyl-, Äthyl-, Hydroxyäthyl-, Dihydroxypropyl- oder Benzylrest bedeutet, und $R_{4a}$ einen Alkyl-, Hydroxyalkyl-, Alkenyl- oder Alkylphenylrest mit 6 bis 20 Kohlenstoffatomen bedeutet, wobei der Alkylteil des Alkylphenylrestes bis zu 14 Kohlenstoffatome besitzt.

17. Oberflächenaktiver, cyclischer Polyäther nach Anspruch 11, dadurch gekennzeichnet, daß die amphiphile Gruppe A die Gruppe (e)

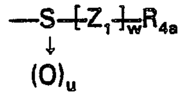

ist, worin
$R_{4a}$ einen Alkyl-, Hydroxyalkyl-, Alkenyl- oder Alkylphenylrest mit 6 bis 20 Kohlenstoffatomen darstellt, wobei der Alkylteil des Alkylphenylrestes bis zu 14 Kohlenstoffatomen aufweist,
u für Null oder 1 steht,
w für Null oder 1 steht, und
$Z_1$ eine Gruppe bedeutet, die ausgewählt ist unter:

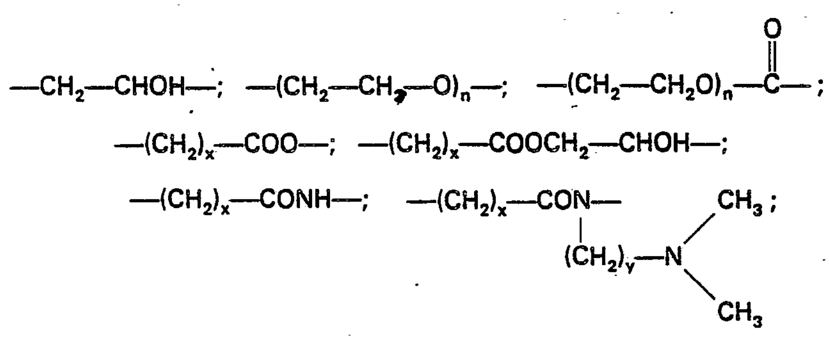

worin:
x für 1 oder 2 steht,
y für 2 oder 3 steht, und
n für irgendeine Zahl zwischen 1 und 20 steht.

18. Oberflächenaktiver cyclischer Polyäther nach Anspruch 11, dadurch gekennzeichnet, daß die amphiphile Gruppe A die Gruppe (f)

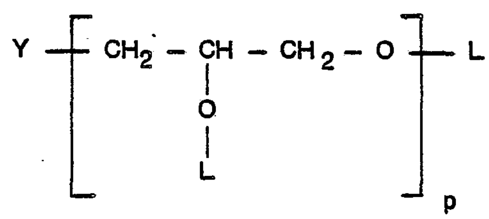

ist, worin
L gleichzeitig ein Wasserstoffatom und eine der Gruppen:

$-CH_2-CHOH-CH_2-(O)_q-R_5$, mit $q = 0$ oder 1

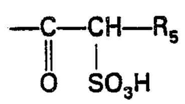

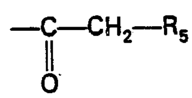

darstellt, wobei das Wasserstoffatom und die andere Gruppe statistisch verteilt sind,
$R_5$ einen linearen Alkylrest mit 6 bis 18 Kohlenstoffatomen darstellt,
p für irgendeine Zahl zwischen 1 und 10 steht und einen statistischen Mittelwert darstellt, und
Y für $-O-$, $-S-$ oder 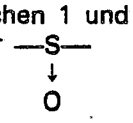
steht, wobei diese Gruppe alkyliert sein kann mit einem Alkylierungsmittel, ausgewählt unter vorzugsweise Methylbromid, Methyljodid und Dimethylsulfat.

19. Oberflächenaktiver cyclischer Polyäther nach Anspruch 11, dadurch gekennzeichnet, daß die amphiphile Gruppe A die Gruppe (g)

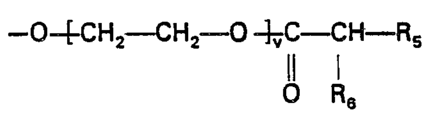

ist, worin,
v für irgendeine Zahl von 0 bis 20 steht,
$R_5$ einen linearen Alkylrest mit 6 bis 18 Kohlenstoffatomen bedeutet und
$R_6$ ein Wasserstoffatom oder einen $-SO_3H$-Rest bedeutet.

20. Verfahren zur Herstellung eines oberflächenaktiven, cyclischen Polyäthers der Formel (I), worin A:

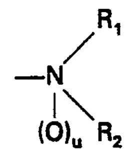

(a)

bedeutet, worin
$R_1$ und $R_2$, die gleich oder verschieden sein können, aliphatische Reste mit 1 bis 20 Kohlenstoffatomen, wobei einer der Reste mindestens 8 Kohlenstoffatome aufweist, oder substituierte aliphatische Reste mit 6 bis 20 Kohlenstoffatomen oder Alkylarylreste mit 6 bis 20 Kohlenstoffatomen bedeuten, wobei die Summe der Kohlenstoffatome von $R_1$ und $R_2$ vorzugsweise kleiner oder gleich 28 ist und einer der Reste außerdem einen Dimethylamino-äthyl-, oder -propyl- Diäthylamino-äthyl- oder -propyl-, Piperidino-äthyl- oder -propyl-, Morpholino-äthyl- oder -propyl- oder Alkylpolyhydroxypropylen-Rest bedeuten kann, und worin
u für Null oder 1 steht,
durch Umsetzung des Epichlorhydrin-Tetrameren der Formel (III)

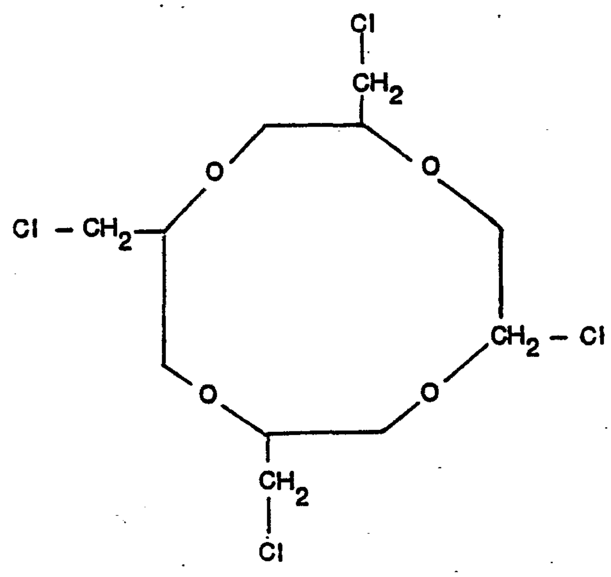

(III)

mit einem sekundären Amin $HNR_1R_2$, wobei $R_1$ und $R_2$ die oben angegebene Bedeutung besitzen, zwischen 80 und 150°C, gegebenenfalls in Gegenwart eines Lösungsmittels.

21. Verfahren zur Herstellung eines oberflächenaktiven cyclischen Polyäthers der Formel (I), worin A:

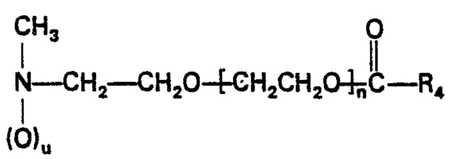

$(a_1)$

bedeutet, worin
n irgendeine Zahl zwischen 1 und 20 bedeutet,
u für Null oder 1 steht, und
$R_4$ einen substituierten oder nichtsubstituierten aliphatischen Rest oder einen Alkylarylrest mit 6 bis 20 Kohlenstoffatomen bedeutet,
durch Umsetzung des Epichlorhydrin-Tetrameren der Formel (III) mit Methyläthanolamin, anschließender Zugabe von n Molen Äthylenoxyd zu dem erhaltenen Produkt und schließlich Veresterung der OH-Funktionen mit einer Säure der Formel $R_4COOH$.

22. Verfahren zur Herstellung eines oberflächenaktiven cyclischen Polyäthers der Formel (I) worin A

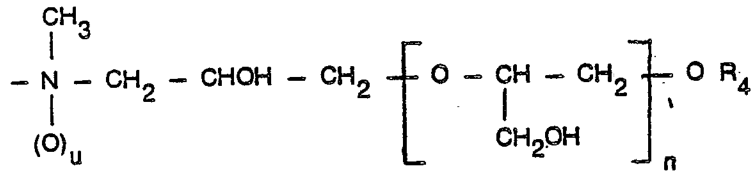

($a_2$)

bedeutet, worin,
n, u und $R_4$ die in Anspruch 10 angegebenen Bedeutungen besitzen, dadurch gekennzeichnet, daß man (1) das Epichlorhydrin-Tetramere der Formel (III) mit Methylamin umsetzt, (2) das erhaltene Produkt mit einer Verbindung der Formel:

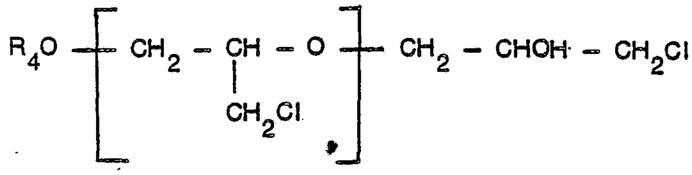

kondensiert, und (3) in der erhaltenen Verbindung die Chloratome mit Hilfe von Natrium- oder Kaliumacetat und anschließender Hydrolyse oder Alkoholyse des gebildeten Essigsäureesters durch Hydroxylgruppen ersetzt.

23. Verfahren zur Herstellung eines oberflächenaktiven, cyclischen Polyäthers der Formel (I), worin A:

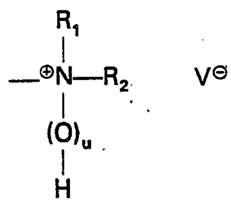

(b) oder

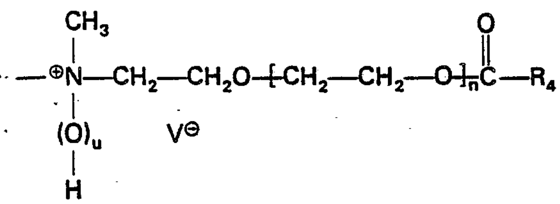

($b_1$) oder

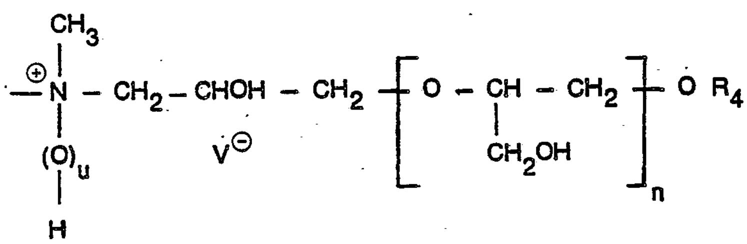

($b_2$)

bedeutet, worin,
$V^\ominus$ ein Anion und vorzugsweise ein Formiat, Acetat, Citrat oder Lactat bedeutet, und u, $R_1$, $R_2$, $R_4$ und n die in Anspruch 10 angegebenen Bedeutungen besitzen,
durch Überführen einer Verbindung der Formel (I), worin A jeweils eine Gruppe:

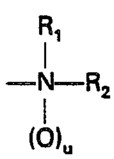

(a) oder

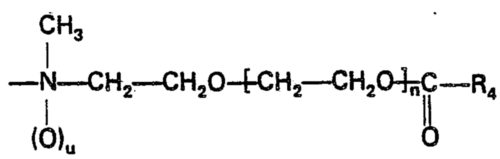

($a_1$) oder

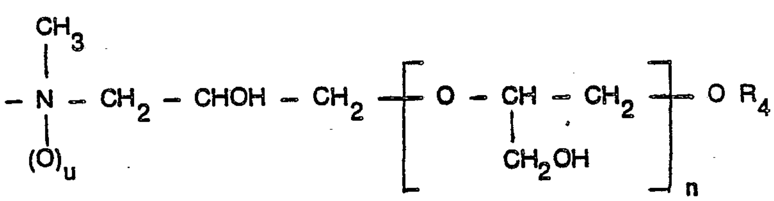

($a_2$)

bedeutet, in ein Salz mit einer organischen Säure und vorzugsweise mit Ameisensäure, Citronensäure, Milchsäure oder Weinsäure.

24. Verfahren zur Herstellung eines oberflächenaktiven, cyclischen Polyäthers der Formel (I), worin A die Gruppe

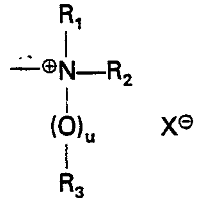 (c) oder

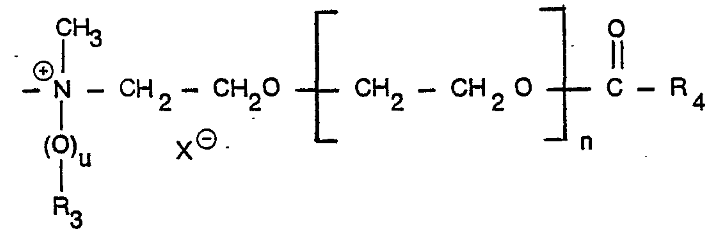 ($c_1$) oder

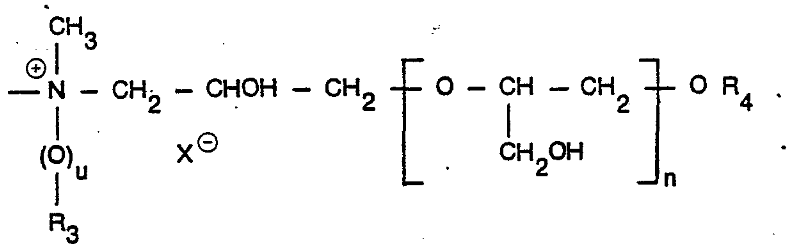 ($c_2$)

bedeutet, worin
$R_3$ einen Alkyl- oder Hydroxyalkyl-Rest mit 1 bis 3 Kohlenstoffatomen und vorzugsweise einen Methyl-, Äthyl-, Hydroxyäthyl-, Dihydroxypropyl- oder Benzylrest bedeutet,
$X^{\ominus}$ ein Anion und vorzugsweise Chlorid, Bromid, Jodid, Monomethylsulfat, Methylsulfonat und p-Toluolsulfonat bedeutet, und
u, $R_1$, $R_2$, $R_4$ und n die im Anspruch 10 angegebenen Bedeutungen besitzen,
durch Alkylierung einer Verbindung der Formel (I), worin A jeweils die Gruppe:

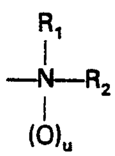 (a) oder 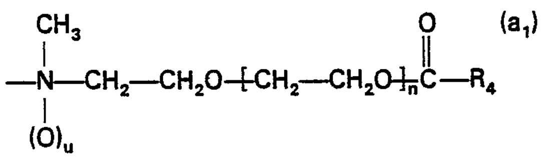 ($a_1$) oder

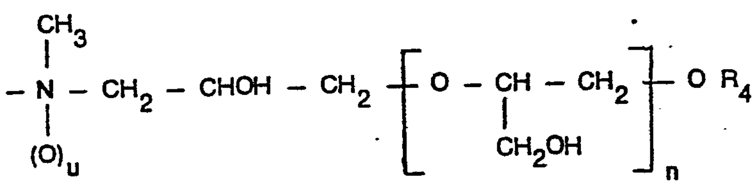 ($a_2$)

bedeutet,
mit einem Alkylierungsmittel $XR_3$, worin X und $R_3$ die oben angegebene Bedeutung besitzen.

25. Verfahren zur Herstellung eines oberflächenaktiven, cyclischen Polyäthers der Formel (I), worin A die Gruppe:

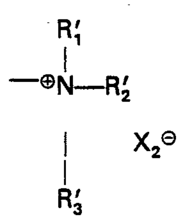

bedeutet, worin
$R_1'$ und $R_2'$ einen Alkyl- oder Hydroxyalkylrest mit 1 bis 3 Kohlenstoffatomen bedeuten,
$R_3'$ einen Alkylrest mit 8 bis 20 Kohlenstoffatomen bedeutet, und
$X_2^{\ominus}$ eine Mesylat-, oder Tosylat-Gruppe bedeutet,

durch Umsetzung des Epichlorhydrin-tetrameren der Formel (III) mit einem sekundären Amin der Formel:

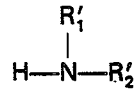

worin, $R_1'$ und $R_2'$ die oben angegebenen Bedeutungen besitzen, und anschließender Alkylierung mit einem Mesylat oder Tosylat eines Alkohols oder einer Mischung von Alkoholen mit 8 bis 20 Kohlenstoffatomen.

26. Verfahren zur Herstellung eines oberflächenaktiven, cyclischen Polyäthers der Formel (I), worin A die Gruppe:

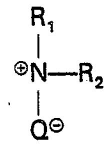 (d) oder 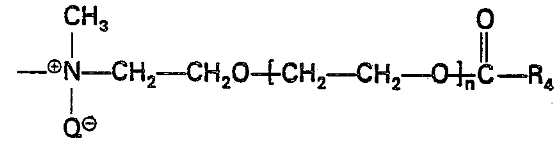 ($d_1$) oder

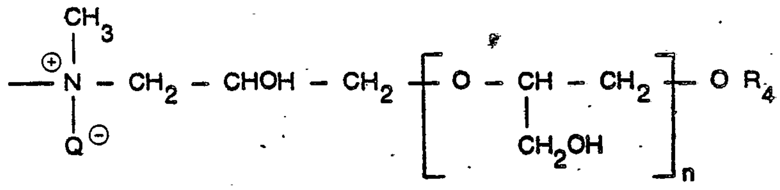 ($d_2$)

bedeutet, worin
$Q^{\ominus}$ eine der folgenden Gruppen:

$-(CH_2)_m-COO^-$ mit m = 1, 2 oder 3

$—CH_2—CH_2—CH_2—SO_3^-$

$—CH_2—CH_2OSO_2^-$

bedeutet, und
$R_1$, $R_2$, $R_4$ und n die in Anspruch 10 angegebenen Bedeutungen besitzen, durch Alkylierung einer Verbindung der Formel (I), worin A jeweils die Gruppe:

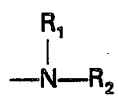 (a') oder

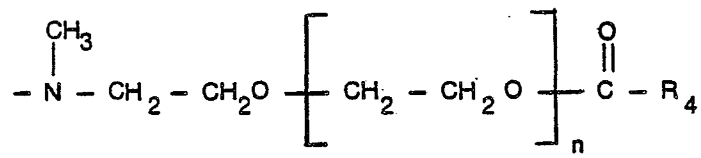 ($a_1'$) oder

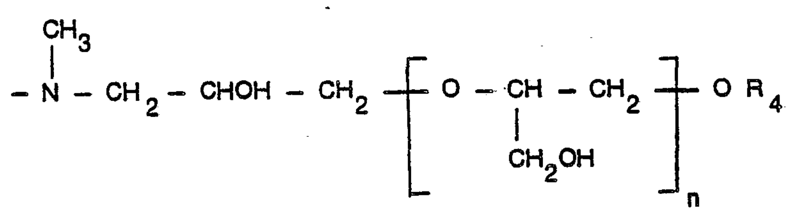 ($a_2'$)

bedeutet, und $R_1$, $R_2$, $R_4$ und n die oben angegebenen Bedeutungen besitzen, mit einer der Säuren der Formeln:

$Cl(CH_2)_mCOOH$; $Br(CH_2)_mCOOH$, mit m = 1, 2 oder 3

$ClCH_2—CH_2—CH_2—SO_3H$; $BrCH_2CH_2CH_2—SO_3H$

oder mit dem Natrium- oder Kaliumsalz einer solchen Säure oder mit Propansulton oder Glycolsulfit.

27. Verfahren zur Herstellung eines oberflächenaktiven, cyclischen Polyäthers der Formel (I), worin A:

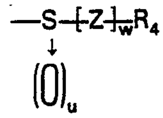

(e)

bedeutet, worin
Z Atomgruppen mit hydrophilem Charakter bedeutet, die in Form eines Restes, der eine oder mehrere Äther-, Ester-, Amid-, Amin-, Ammonium- und/oder Hydroxygruppen einschließt, vorliegen,
$R_4$ die in Anspruch 10 angegebene Bedeutung besitzt,
u für Null oder 1 steht, und
w für Null oder 1 steht,
dadurch gekennzeichnet, daß man in einer ersten Stufe das Epichlorhydrin-tetramere der Formel (III) mit einem Reagenz, ausgewählt unter Alkylmercaptanen, Hydroxyalkylmercaptanen, Alkylmono- oder -polyäthoxymercaptanen, Methyl- oder Äthylestern einer Mercaptoessigsäure oder Mercaptopropionsäure, bei einer Temperatur zwischen 80 und 150°C in Gegenwart einer alkalischen Verbindung, ausgewählt unter Natriumhydroxyd, Kaliumhydroxyd, Natrium- oder Kaliummethylat oder -äthylat, umsetzt,
und daß man gegebenenfalls in einer zweiten Stufe die in der ersten Stufe erhaltene Estergruppe durch Verseifen oder Hydrolyse in eine entsprechende Säuregruppe oder durch Umesterung in einen anderen Ester oder durch Umsetzung mit einem primären Amin oder einem sekundären-tertiären Diamin in eine Amidgruppe überführt,
und daß man gegebenenfalls die Thioäthergruppe mit Hilfe von Wasserstoffperoxyd zum Sulfoxyd oxidiert und/oder
die Thioäther- oder Sulfoxyd-Gruppe in eine Sulfonium- oder Sulfoxonium-Gruppe überführt und/oder die tert.-Amingruppe durch Alkylierung mit einem Alkylierungsmittel in eine Ammoniumgruppe überführt.

28. Verfahren nach Anspruch 27, dadurch gekennzeichnet, daß Z für einen Rest steht, der eine oder mehrere Äther- und/oder Hydroxylgruppen aufweist und
daß man in der ersten Stufe ein Reagenz, ausgewählt unter Alkyl-mercaptanen, Hydroxyalkylmercaptanen und Alkylmono- oder -poly-äthoxymercaptanen, mit dem Epichlorhydrin-tetrameren der Formel (III) reagieren läßt.

29. Verfahren nach Anspruch 27, dadurch gekennzeichnet, daß
w für 1 steht,
Z die Gruppe:

$—(CH_2)_x—COO—$ oder $—(CH_2)_x—CONH—$ oder 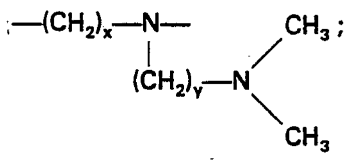

bedeutet, worin
x für 1 oder 2 steht, und
y für 2 oder 3 steht,
daß man in einer ersten Stufe eine Zwischenverbindung der Formel (IV):

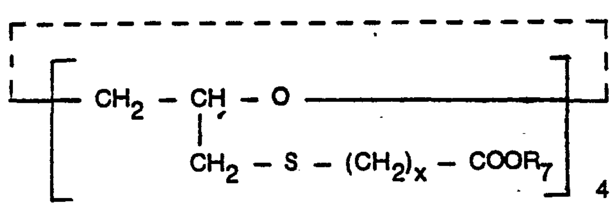

(IV)

herstellt, worin
x für 1 oder 2 steht und
$R_7$ für $CH_3$ oder $C_2H_5$ steht,
durch Umsetzung des Epichlorhydrin-tetrameren der Formel (III) mit einem Ester der Formel:

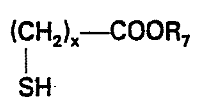

worin x und $R_7$ die oben angegebenen Bedeutungen besitzen,
und daß man in einer zweiten Stufe diese Zwischenverbindung der Formel (IV) mit einem Alkohol der

Formel $R_4OH$ oder mit einem primären Amin der Formel $R_4NH_2$ oder mit einem sekundären-tertiären Diamin der Formel:

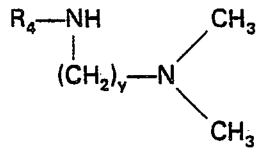

reagieren läßt, worin
$R_4$ die in Anspruch 10 angegebenen Bedeutungen besitzt,
x für 1 oder 2 steht, und
y für 2 oder 3 steht.

30. Verfahren nach den Ansprüchen 27 und 29, dadurch gekennzeichnet, daß
w für 1 steht,
Z die Gruppe:

$$-(CH_2)_x-COO-CH_2-CHOH$$

bedeutet,
worin x für 1 oder 2 steht,
daß man in einer ersten Stufe eine Zwischenverbindung der Formel

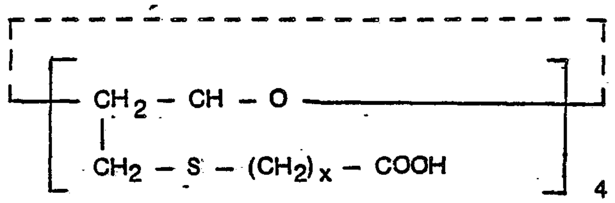

(IV A)

herstellt, durch Verseifen der Verbindung der Formel (IV), und
daß man in einer zweiten Stufe ein 1,2-Epoxy-alkan der Formel:

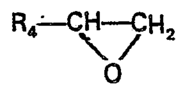

worin $R_4$ die in Anspruch 10 angegebene Bedeutung besitzt, mit der Zwischenverbindung der Formel (IV A) reagieren läßt.

31. Verfahren nach Anspruch 27, dadurch gekennzeichnet, daß
w für 1 steht,
Z die Gruppe:

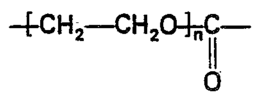

worin n irgendeine Zahl zwischen 1 und 20 darstellt, bedeutet,
daß man in einer ersten Stufe das Thioäthanol mit dem Epichlorhydrin-tetrameren der Formel (III) kondensiert,
daß man in einer zweiten Stufe 1 bis 20 Mol Äthylenoxyd zu der in der ersten Stufe erhaltenen Verbindung hinzugibt, und
daß man in einer dritten Stufe die in der zweiten Stufe erhaltene Verbindung mit einer Säure der Formel $R_4$—COOH, worin $R_4$ die in Anspruch 10 angegebenen Bedeutungen besitzt, verestert.

32. Verfahren zur Herstellung eines oberflächenaktiven, cyclischen Polyäthers der Formel (I), worin A:

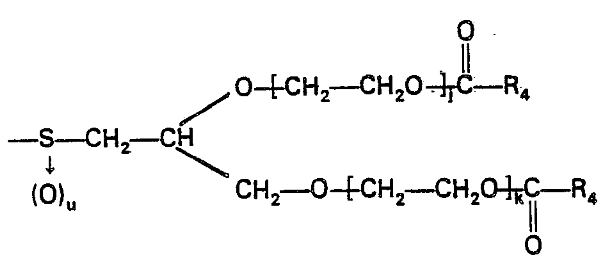

($e_1$)

bedeutet, worin
j und k jeweils irgendeine Zahl kleiner 20 bedeuten, die Summe j + k zwischen 1 und 20 beträgt,
u für Null oder 1 steht, und

$R_4$ die im Anspruch 10 angegebene Bedeutung besitzt, dadurch gekennzeichnet, daß man in einer ersten Stufe das Thioglycerin mit dem Epichlorhydrintetrameren der Formel (III) kondensiert, daß man in einer zweiten Stufe die Oxyäthylenierung der in der ersten Stufe erhaltenen Verbindung durchführt, daß man in einer dritten Stufe mit einer Säure der Formel $R_4COOH$ verestert, und daß man gegebenenfalls in einer vierten Stufen die Thioäthergruppe mit Wasserstoffperoxyd zum Sulfoxyd oxydiert und/oder die Thioäther- oder Sulfoxydgruppe mit einem Alkylierungsmittel und vorzugsweise mit Methylbromid- oder -jodid oder Dimethylsulfat in eine Sulfonium- oder Sulfoxonium-Gruppe überführt.

33. Verfahren zur Herstellung eines oberflächenaktiven cyclischen Polyäthers der Formel (I), worin A:

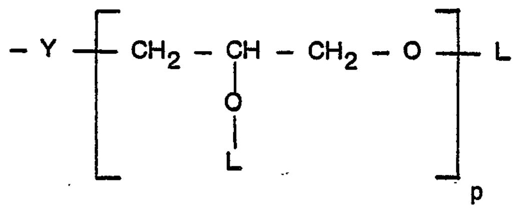

(f)

bedeutet, worin L gleichzeitig ein Wasserstoffatom und eine der Gruppen:

$—CH_2—CHOH—CH_2—(O)_q—R_5$ oder 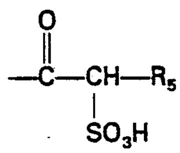 oder 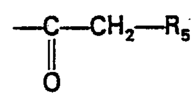

bedeutet, wobei $R_5$ einen linearen Alkylrest mit 6 bis 18 Kohlenstoffatomen darstellt, q für Null oder 1 steht, und das Wasserstoffatom und die andere Gruppe statistisch verteilt sind, und worin: Y Sauerstoff darstellt, p eine ganze oder Dezimalzahl von 1 bis 10 bedeutet, und einen statistischen Mittelwert darstellt, dadurch gekennzeichnet, daß man in einer ersten Stufe Glycidol, alkalisch katalysiert, mit dem hydroxylierten Epichlorhydrin-tetrameren der Formel (II)

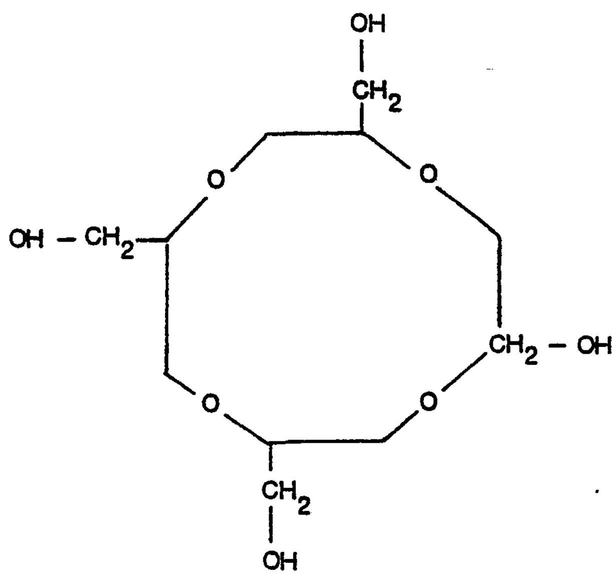

(II)

umsetzt, und daß man in einer zweiten Stufe die in der ersten Stufe erhaltene Verbindung mit einem Alkylenoxyd der Formel

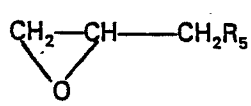

oder mit einem Alkylglycidyläther der Formel:

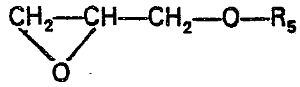

worin $R_5$ die oben angegebene Bedeutung besitzt, umsetzt.

34. Verfahren zur Herstellung eines oberflächenaktiven, cyclischen Polyäthers der Formel (I), worin A:

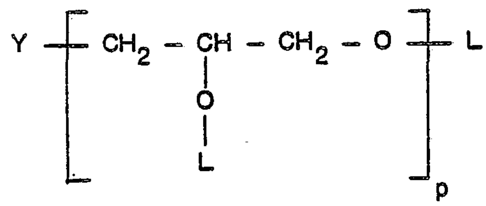

(f)

bedeutet, worin
L gleichzeitig ein Wasserstoffatom und eine der Gruppen:

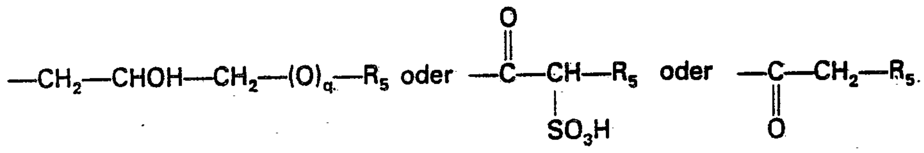

bedeutet,
wobei $R_5$ einen linearen Alkylrest mit 6 bis 18 Kohlenstoffatomen bedeutet, q für Null oder 1 steht, und das Wasserstoffatom und die andere Gruppe statistisch verteilt sind, und worin
Y für —S— oder 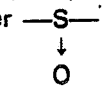 und

p eine ganze Zahl oder eine Dezimalzahl von 1 bis 10 bedeutet und einen statistischen Mittelwert darstellt,
dadurch gekennzeichnet,
daß die Reaktion in einer oder mehreren Stufen durchgeführt wird,
daß man in einer ersten Stufe eine Zwischenverbindung herstellt der Formel:

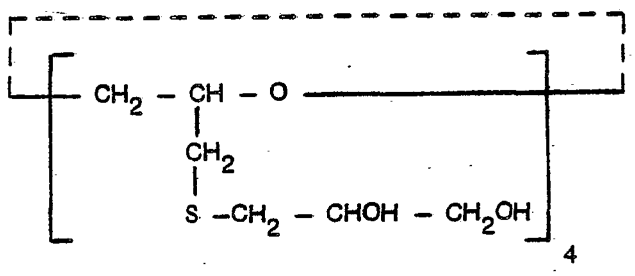

(IX)

durch Umsetzung des Epichlorhydrin-tetrameren der Formel (III) mit Thioglycerin in Gegenwart eines Lösungsmittels und einer alkalischen Verbindung, daß man dann die Zwischenverbindung der Formel (IX) in Anwesenheit einer alkalischen Verbindung mit 0 bis 9 Mol Glycidol und/oder Alkylenoxyd mit 8 bis 20 Kohlenstoffatomen und/oder Alkylglycidyläther umsetzt, und daß man gegebenenfalls dann die Hydroxylfunktionen mit einer Carbonsäure oder $\alpha$-Sulfocarbonsäure mit 8 bis 20 Kohlenstoffatomen verestert und/oder die Thioäthergruppe mit Wasserstoffperoxyd in eine Sulfoxydgruppe oxydiert und/oder die Thioäther- oder Sulfoxyd-Gruppe mit einem bekannten Alkylierungsmittel und vorzugsweise mit Methylbromid- oder -jodid oder Dimethylsulfat, zu einer Sulfonium- oder Sulfoxonium-gruppe alkyliert.

35. Verfahren zur Herstellung eines oberflächenaktiven, cyclischen Polyäthers der Formel (I), worin A die Gruppe:

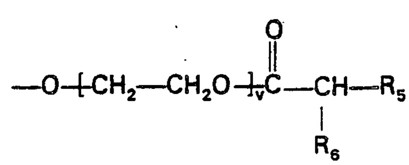

(g)

bedeutet, worin
v für irgendeine Zahl kleiner oder gleich 20, jedoch nicht für Null, steht,
$R_5$ einen linearen Alkylrest mit 6 bis 18 Kohlenstoffatomen, und
$R_6$ für H oder $SO_3H$ steht,
dadurch gekennzeichnet,
daß man in einer ersten Stufe viermal v Mol Äthylenoxyd mit dem hydroxylierten Derivat des Epichlorhydrintetrameren der Formel (II) reagieren läßt und daß man in einer zweiten Stufe die Hydroxylfunktionen mit einer Sulfocarbonsäure der Formel:

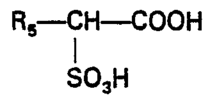

oder mit einer Carbonsäure der Formel:

$$R_5—CH_2—COOH$$

verestert.

36. Verfahren zur Herstellung eines Oberflächenaktiven, cyclischen Polyäthers der Formel (I), worin A die Gruppe:

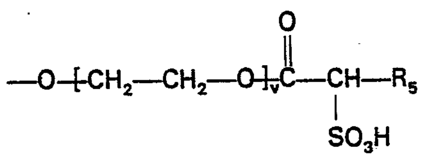 (g)

bedeutet, worin
v für Null steht und
$R_5$ die in Anspruch 19 angegebene Bedeutung besitzt, dadurch gekennzeichnet,
daß man die Hydroxylfunktionen des hydroxylierten Derivats des Epichlorhydrin-tetrameren der Formel (II) mit einer Sulfocarbonsäure der Formel:

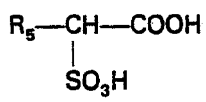

verestert.